(19)

Europäisches
Patentamt
European
Patent Office
Office européen
des brevets

(11)    **EP 1 578 952 B1**

(12)                    **EUROPEAN PATENT SPECIFICATION**

(45) Date of publication and mention
of the grant of the patent:
**23.11.2011  Bulletin 2011/47**

(21) Application number: **03810070.7**

(22) Date of filing: **12.12.2003**

(51) Int Cl.:
*C12Q 1/68* (2006.01)

(86) International application number:
**PCT/US2003/039836**

(87) International publication number:
**WO 2004/053105 (24.06.2004 Gazette 2004/26)**

(54) **DIRECT SNP DETECTION WITH UNAMPLIFIED DNA**

DIREKTER SNP-NACHWEIS MIT NICHTAMPLIFIZIERTER DNA

DETECTION DE SNP DIRECTE AVEC ADN NON AMPLIFIE

(84) Designated Contracting States:
**AT BE BG CH CY CZ DE DK EE ES FI FR GB GR
HU IE IT LI LU MC NL PT RO SE SI SK TR**

(30) Priority:  **12.12.2002  US 432772 P
12.12.2002  US 433442 P**

(43) Date of publication of application:
**28.09.2005  Bulletin 2005/39**

(73) Proprietor: **Nanosphere, Inc.
Northbrook, Illinois 60062 (US)**

(72) Inventors:
• **BAO, Yijia, P.
Des Plaines, IL 60016 (US)**
• **MARLA, Sudhakar, S.
Evanston, IL 60202 (US)**
• **MULLER, Uwe
Waukegan, IL 60087 (US)**
• **STORHOFF, James, J.
Evanston, IL 60202 (US)**
• **HETZEL, Susan, R.
Salem, WI 53168 (US)**

(74) Representative: **Grund, Martin et al
Grund
Intellectual Property Group
Postfach 44 05 16
80754 München (DE)**

(56) References cited:
**WO-A-00/50869          WO-A-01/23604
US-A- 5 474 796         US-A- 5 849 486
US-A1- 2002 090 649**

• **HAMELS S ET AL: "CONSENSUS PCR AND
MICROARRAY FOR DIAGNOSIS OF THE GENUS
STAPHYLOCOCCUS, SPECIES, AND
METHICILLIN RESISTANCE" BIOTECHNIQUES,
INFORMA LIFE SCIENCES PUBLISHING,
WESTBOROUGH, MA, US, vol. 31, no. 6,
December 2001 (2001-12), pages 1364-1366,1368,
XP001153876 ISSN: 0736-6205**
• **MARTINEAU F ET AL: "Development of a PCR
assay for identification of staphylococci at genus
and species levels" JOURNAL OF CLINICAL
MICROBIOLOGY, WASHINGTON, DC, US, vol. 39,
no. 7, July 2001 (2001-07), pages 2541-2547,
XP003007591 ISSN: 0095-1137**
• **ALEXANDRE I ET AL: "COLORIMETRIC SILVER
DETECTION OF DNA MICROARRAYS"
ANALYTICAL BIOCHEMISTRY, ACADEMIC
PRESS, NEW YORK, NY, US, vol. 295, no. 1, 1
August 2001 (2001-08-01), pages 1-8,
XP001204826 ISSN: 0003-2697**
• **CAO Y C; JIN R; MIRKIN C A: "Nanoparticles with
Raman spectroscopic fingerprints for DNA and
RNA detection", SCIENCE, vol. 297, no. 5586, 30
August 2002 (2002-08-30), pages 1536-1540,
XP002977805,**
• **TATON T A, MIRKIN C A, LETSINGER R L:
"Scanometric DNA array detection with
nanoparticle probes", SCIENCE, vol. 289, 8
September 2000 (2000-09-08), pages 1757-1760,
XP002158394,**

EP 1 578 952 B1

Description

## FIELD OF THE INVENTION

The invention relates to a method for detection of a target nucleic acid molecule in a sample that comprises nucleic acid molecules of higher biological complexity than that of amplified nucleic acid molecules, for example in genomic DNA. In particular, the invention relates to methods and probes for SNP detection using nanoparticle-labeled probes. The invention also relates to methods for detecting biological organisms, and in particular, bacterial pathogens such as Staphylococcal DNA in a sample and for detecting antibiotic resistance genes such as the mecA gene, which confers resistance to the antibiotic methicillin.

## BACKGROUND OF THE INVENTION

Single nucleotide polymorphisms (SNPs) or single base variations between genomic DNA observed in different individuals not only form the basis of genetic diversity, they are expected to be markers for disease propensity, to allow better disease management, to enhance understanding of disease states and to ultimately facilitate the discovery of more effective drugs. As a consequence, numerous efforts are ongoing with the common goal of developing methods that reliably and rapidly identify SNPs. The majority of these efforts require target amplification by methods such as PCR because of the inherent complexity of human genomic DNA (haploid genome = $3 \times 10^9$ bp) and the associated sensitivity requirements. The ability to detect SNPs directly in human genomic DNA would simplify the assay and eliminate target amplification-related errors in SNP identification.

Single nucleotide polymorphisms can be identified by a number of methods, including DNA sequencing, restriction enzyme analysis, or site-specific hybridization. However, high-throughput genome-wide screening for SNP and mutations requires the ability to simultaneously analyze multiple loci with high accuracy and sensitivity. To increase sensitivity as well as specificity, current high-throughput methods for single nucleotide detection rely on a step that involves amplification of the target nucleic acid sample, usually by the polymerase chain reaction (PCR) (see, e.g., Nikiforov et al., U.S. Pat. No. 5,679,524 issued Oct. 21, 1997; McIntosh et al., PCT publication WO 98/59066 dated Dec. 30, 1998; Goelet et al., PCT publication WO 95/12607 dated May 11, 1995; Wang et al., 1998, Science 280: 1077-1082; Tyagi et al., 1998, Nature Biotechnol. 16:49-53; Chen et al., 1998, Genome Res. 8:549-556; Pastinen et al., 1996, Clin. Chem. 42:1391-1397; Chen et al, 1997, Proc. Natl. Acad. Sci. 94:10756-10761; Shuber et al., 1997, Hum. Mol. Gen. 6:337-347; Liu et al., 1997, Genome Res. 7:389-398; Livak et al., Nature Genet. 9:341-342; Day and Humphries, 1994, Anal. Biochem. 222:389-395). Typically there are two reasons why PCR amplification is necessary for conventional hybridization based SNP detection. First, when obtaining total human DNA, which has a size of 3,000,000,000 base pairs per haploid genome, the target sequence containing the SNP site represents only a very small fraction of the total DNA. For instance, a 20 base target sequence represents only 0.00000033% of the total DNA (for a normal genome there are two copies of that target sequence, but they may have different SNP sites and are therefore considered different sites). Thus, a typical DNA sample of a few micrograms may be insufficient for many of the current techniques for lack of sensitivity. A more important reason, however, is that hybridization to that 20 base target sequence with oligonucleotides that are sufficiently short to allow single base discrimination do not hybridize exclusively to the target region, but bind to a small extent to other regions in the genome. Given the overwhelming amount of non-target DNA, the non-specific hybridization creates such a large background that it buries the specific signal. Thus, a PCR amplification of one target region is a necessary step to dramatically reduce the amount of non-specific sequences. This amplification step is referred to as "complexity reduction." However, the fidelity of the PCR technique is limited. Combinations of pairs of PCR primers tend to generate spurious reaction products or fail in some particular regions. Moreover, the number of errors in the final reaction product increases exponentially with each round of PCR amplification after a non-target sequence has been copied, or if an error has been introduced into the target sequence because of mis-incorporation. Thus, PCR errors can be a substantial drawback when searching for rare variations in nucleic acid populations.

Finally, a drawback of using target amplification is that each SNP site has to be separately amplified. Since there are potentially millions of SNPs in the human genome, this becomes an insurmountable task. Even the "state of the art" amplification methods and strategies that partially circumvent the problem of SNP-site-specific amplification can identify only a small percentage of the total number of SNPs simultaneously (less than 0.1%) (see *e.g*. Kennedy et al., 2003, Nature Biotechnol. 21:1233-1237). Eric Lander of the Whitehead Institute for Biomedical Research and one of the leaders of the human genome project cited elimination of target amplification as one of the most significant challenges in genome wide screening of SNPs (see Lander E. 1999, Nature Genetics Suppl. 21: 3-4). Thus, there remains a need in the art for more sensitive, effective, and cost efficient methods for detecting SNPs in a sample that do not require target amplification or complexity reduction.

The identification of DNA mutations is also critical for identifying biological microorganisms (see Edwards et.

al, J. Clin. Micro. 39: 3047-3051). For example, the genus Staphylococcus contains at least 38 different species, and a large number of these species have been identified in hospital-based infections (Edwards et. al, J. Clin. Micro. 39: 3047-3051). Therefore, the rapid identification and speciation of organisms is critical for identifying the source of infection which helps determine patient treatment, and epidemiologically for recognizing outbreaks of infection and cross transmission of nosocomial pathogens (Olive and Bean, 1999, J. Clin. Micro. 37: 1661-1669). Conventional methods for identifying bacteria based on biochemical tests are often lengthy (> 1 day) and often do not enable accurate identification of specific species (Hamels et. al, 2001, Biotechniques 31: 1364-1372). Therefore, significant effort has been devoted to developing more rapid, accurate, and less expensive methods for identifying specific bacterial species based on identification of nucleic acid sequences, especially in the case of nosocomial pathogens such as Staphylococcus. Microorganisms of the same family or genus contain phylogenetically conserved genes that encode for the same protein (Hamels et. al, 2001, Biotechniques 31: 1364-1372). Although the gene sequences from the same family are typically highly conserved, species-specific sequence mutations within a variety of genes (e.g. 16S rRNA) have been identified. Oligonucleotide probes which target a variable region of the 16S rRNA gene have been developed to identify a variety of coagulase negative and positive Staphylococcus species in real time PCR assays (Edwards et. al, J. Clin. Micro. 39: 3047-3051).

Furthermore, microarrays have been developed to identify the genus Staphylococcus, species, and antibiotic resistance using PCR-amplified femA gene sequences (Hamels et. al, 2001, Biotechniques 31: 1364-1372). The microarrays contained oligonucleotide probes which recognized species specific sequence variations in the femA gene (sequence variation of three bases or greater) associated with the five most clinically relevant *Staphylococcus* species (*S. aureus*, *S. epidermidis*, *S. haemolyticus, S. hominis*, *S. saprophyticus*), while an oligonucleotide probe that targeted a conserved region of the same gene was used to identify the genus *Staphylococcus.* However, one of the major drawbacks of both the Microarray and real time PCR based assays is the requirement of PCR which can be less than ideal both clinically and from a cost perspective (see above PCR discussion for SNP identification). Thus, there remains a need in the art for more sensitive, effective, and cost efficient methods for detecting and speciating biological organisms in a sample that do not require target amplification or complexity reduction. MARTINEAU F et al. describe the development of a PCR assay for identification of staphylococci at genus and species levels (JOURNAL OF CLINICAL MICROBIOLOGY, WASHINGTON, DC, US, vol. 39, no. 7, July 2001 (2001-07), pages 2541-2547). CAO Y C. et al. disclose nanoparticles with Raman spectroscopic fingerprints for DNA and RNA detection (SCIENCE, vol. 297, no. 5586, 30 August 2002 (2002-08-30), pages 1536-1540).

## SUMMARY OF THE INVENTION

The invention relates to subject matter as defined in the appended claims. Disclosed are methods for detecting a target nucleic acid sequence in a sample, wherein the sample comprises nucleic acid molecules of higher biological complexity than that of amplified nucleic acid molecules and the target nucleic acid sequence differs from a known nucleic acid sequence by at least a single nucleotide. A single nucleotide difference, for example, can be a single nucleotide polymorphism.

Disclosed are methods for detecting a target nucleic acid sequence in a sample without prior target amplification or complexity reduction comprise the steps of: a) providing an addressable substrate having a capture oligonucleotide bound thereto, wherein the capture oligonucleotide has a sequence that is complementary to at least part of a first portion of the target nucleic acid sequence; b) providing a detection probe comprising detector oligonucleotides, wherein the detector oligonucleotides have sequences that are complementary to at least part of a second portion of the target nucleic acid sequence of step (a); c) contacting the sample with the substrate and the detection probe under conditions that are effective for the hybridization of the capture oligonucleotide to the first portion of the target nucleic acid sequence and the hybridization of the detection probe to the second portion of the target nucleic acid sequence; and d) detecting whether the capture oligonucleotide and detection probe hybridized with the first and second portions of the target nucleic acid sequence.

Disclosed are methods for detecting a target nucleic acid sequence in a sample without prior target amplification or complexity reduction comprise the steps of: a) providing an addressable substrate having a plurality of capture oligonucleotides bound thereto, wherein the capture oligonucleotides have sequences that are complementary to one or more portions of the target nucleic acid sequence; b) providing a detector probe comprising detector oligonucleotides, wherein the detector oligonucleotides have sequences that are complementary to one or more portions of the target nucleic acid sequence of step (a) that are not recognized by a capture oligonucleotide on the substrate; c) contacting the sample with the substrate and the detector probe under conditions that are effective for the hybridization of the capture oligonucleotides to one or more portions of the target nucleic acid sequence and the hybridization of the detector probe to one or more portions of the target nucleic acid sequence that is not recognized by a capture oligonucleotide; and d) detecting whether the capture oligonucleotide and detector probe hybridized with the target nucleic acid sequence.

Disclosed are methods for identifying a single nucleotide polymorphism in a sample, wherein the sample comprises nucleic acid molecules of higher biological complexity relative to amplified nucleic acid molecules.

Disclosed are methods for identifying a single nucleotide polymorphism in a sample without prior target amplification or complexity reduction comprise the steps of: a) providing an addressable substrate having at least one capture oligonucleotide bound thereto, wherein the at least one capture oligonucleotide have sequences that are complementary to at least a part of a nucleic acid target that comprises a specific polymorphism; b) providing a detector probe having detector oligonucleotides bound thereto, wherein the detector oligonucleotides have sequences that are complementary to at least a portion of the nucleic acid target of step (a); c) contacting the sample with the substrate and the detector probe under conditions that are effective for the hybridization of the capture oligonucleotide to the nucleic acid target and the hybridization of the detector probe to the nucleic acid target; and d) detecting whether the capture oligonucleotide and detector probe hybridized with the nucleic acid target.

Disclosed are methods for identifying a single nucleotide polymorphism in a sample without prior target amplification or complexity reduction comprise the steps of: a) providing an addressable substrate having a plurality of capture oligonucleotides bound thereto, wherein the capture oligonucleotides have sequences that are complementary to multiple portions of a nucleic acid target, each portion comprising a specific polymorphism; b) providing a detector probe comprising detector oligonucleotides, wherein the detector oligonucleotides have sequences that are complementary to at least a portion of the nucleic acid target of step (a) that is not recognized by a capture oligonucleotide on the substrate; c) contacting the sample with the substrate and the detector probe under conditions that are effective for the hybridization of the capture oligonucleotides to multiple portions of the nucleic acid target and the hybridization of the detector probe to the nucleic acid target; and d) detecting whether the capture oligonucleotide and detector probe hybridized with the nucleic acid target.

The nucleotide difference or Single Nucleotide Polymorphism of the target nucleic acid can be recognized by either the capture oligonucleotide bound to the substrate or by the detector oligonucleotides.

The target nucleic acid molecules in a sample can comprise genomic DNA, genomic RNA, expressed RNA, plasmid DNA, mitochondrial or other cell organelle DNA, free cellular DNA, viral DNA or viral RNA, or a mixture of two or more of the above.

A substrate can comprise a plurality of capture oligonucleotides, each of which can recognize one or more different single nucleotide polymorphisms or nucleotide differences, and the sample can comprise more than one nucleic acid target, each of which comprises a different single nucleotide polymorphism or nucleotide difference that can hybridize with one of the plurality of capture oligonculeotides. In addition, one or more types of detector probes can be provided in a method of the invention, each of which has detector oligonucleotides bound thereto that are capable of hybridizing with a different nucleic acid target.

A sample can be contacted with the detector probe so that a nucleic acid target present in the sample hybridizes with the detector oligonucleotides on the detector probe, and the nucleic acid target bound to the detector probe can then be contacted with the substrate so that the nucleic acid target hybridizes with the capture oligonucleotide on the substrate. Alternatively, a sample can be contacted with the substrate so that a nucleic acid target present in the sample hybridizes with a capture oligonucleotide, and the nucleic acid target bound to the capture oligonucleotide can then be contacted with the detector probe so that the nucleic acid target hybridizes with the detector oligonucleotides on the detector probe. A sample can be contacted simultaneously with the detector probe and the substrate.

A detector oligonucleotide can comprise a detectable label. The label can be, for example, fluorescent, luminescent, phosphorescent, radioactive, or a nanoparticle, and the detector oligonucleotide can be linked to a dendrimer, a molecular aggregate, a quantum dot, or a bead. The label can allow for detection, for example, by photonic, electronic, acoustic, opto-acoustic, gravity, electro-chemical, electro-optic, mass-spectrometric, enzymatic, chemical, biochemical, or physical means.

The detector probe can be a nanoparticle probe having detector oligonucleotides bound thereto. The nanoparticles can be made of, for example, a noble metal, such as gold or silver. A nanoparticle can be detected, for example, using an optical or flatbed scanner. The scanner can be linked to a computer loaded with software capable of calculating grayscale measurements, and the grayscale measurements are calculated to provide a quantitative measure of the amount of nucleic acid detected. Where the nanoparticle is made of gold, silver, or another metal that can promote autometallography, the substrate that is bound to the nanoparticle by means of a target nucleic acid molecule can be detected with higher sensitivity using silver stain. Alternatively, the substrate bound to a nanoparticle can be detected by detecting light scattered by the nanoparticle.

Disclosed is that oligonucleotides attached to a substrate can be located between two electrodes, the nanoparticles can be made of a material that is a conductor of electricity, and step (d) in the methods of the invention can comprise detecting a change in conductivity. In yet another embodiment, a plurality of oligonucleotides, each of which can recognize a different target nucleic acid sequence, are attached to a substrate in an array of spots and each spot of oligonucleotides is located between two electrodes, the nanoparticles are made of a material that is a conductor of electricity, and step (d) in the methods of the invention comprises detecting a change in conductivity. The electrodes can be made, for example, of gold and the nanoparticles are made of gold. Alternatively, a substrate can be contacted with silver stain to produce a change in conductivity.

The methods can be used to distinguish between two or more species of a common genus. In one aspect, the species can differ by two or more non-consecutive nucleotides. In another aspect, the species can differ by two or more consecutive nucleotides.

A target nucleic acid sequence can be a portion of a gene of a Staphylococcus bacterium. In one aspect, the Staphylococcus bacterium can be, for example, *S. aureus*, *S. haemolyticus*, *S. epidermidis*, *S. lugdunensis*, *S. hominis*, or *S. saprophyticus*. Thus, the methods can be used for Staphylococcus speciation (*i.e.* differentiating between different species of Staphylococcus bacteria).

A target nucleic acid sequence can be a portion of the mecA gene. Thus, the methods can be used to identify methicillin resistant strains of bacteria.

A target nucleic acid sequence, a capture oligonucleotide, and/or a detection oligonucleotide can comprise the sequence set forth in SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 24, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO:77, or SEQ ID NO:78.

## BRIEF DESCRIPTION OF THE DRAWINGS

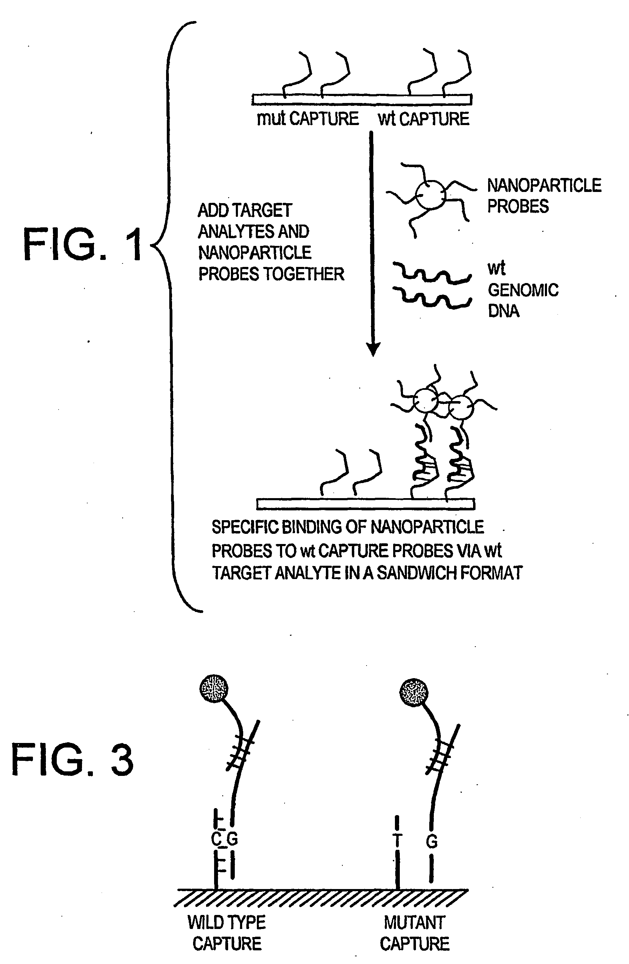

Figure 1 shows a schematic representation of the single-step hybridization process of the invention.

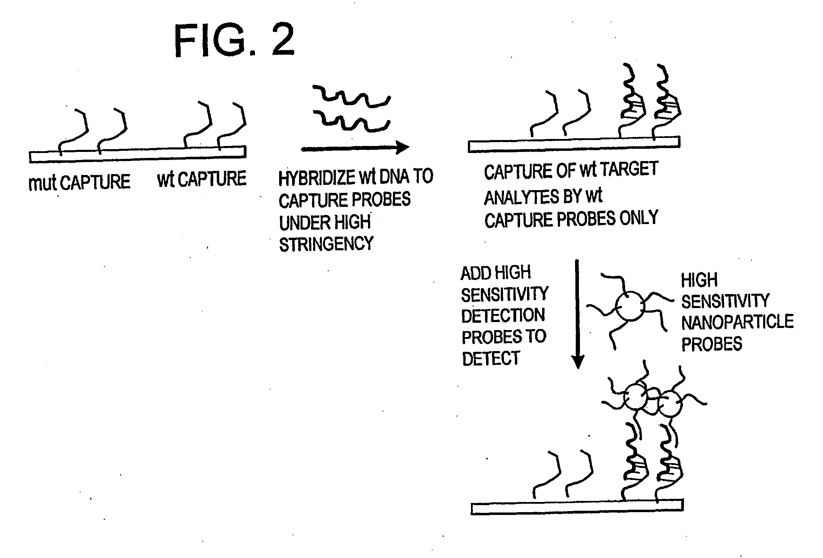

Figure 2 shows a schematic representation of the two-step hybridization process of the invention.

Figure 3 illustrates schematically a hybridized complex of a nanoparticle-tabeled detection probe, a wild-type or mutant capture probe bound to a substrate, and a wild-type target. For SNP detection, the assay is performed under appropriate experimental conditions that retain the perfectly matched complexes (left) while preventing the complex containing the mismatch from forming (right).

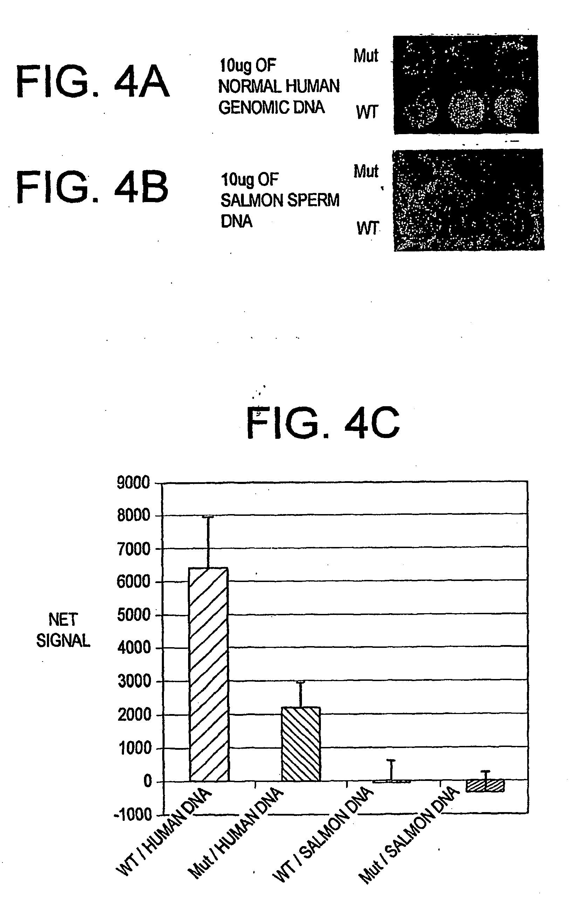

Figure 4 illustrates SNP detection of the factor V gene (1691 G ->A) with unamplified human genomic DNA [part (a)] or salmon sperm DNA [part (b)] on Superaldehyde® slides that have wild type or mutant factor V gene capture probes. Part (c) is a graph that summarizes a detection signal intensity analysis for human genomic DNA and nonspecific salmon sperm DNA in the presence of either the wild type or mutant capture probes.

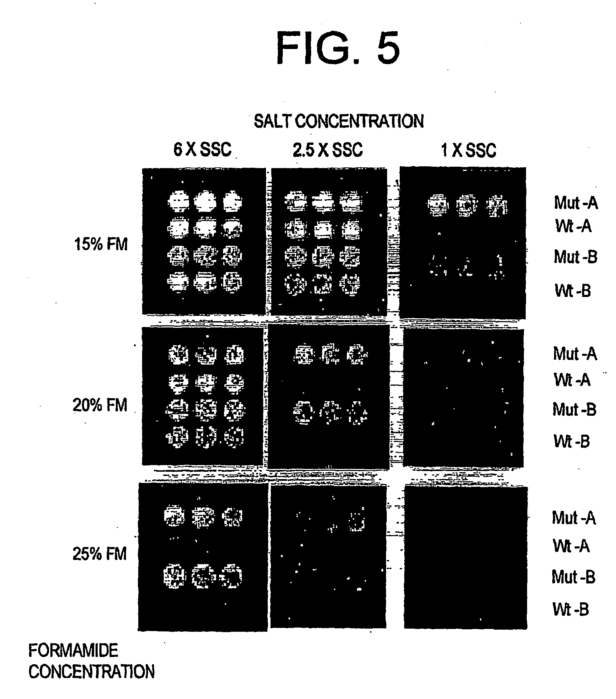

Figure 5 demonstrates the importance of adjusting the hybridization conditions in order to make the methods of the invention capable of discriminating between two target nucleic acids that differ by 1 nucleotide (the SNP site).

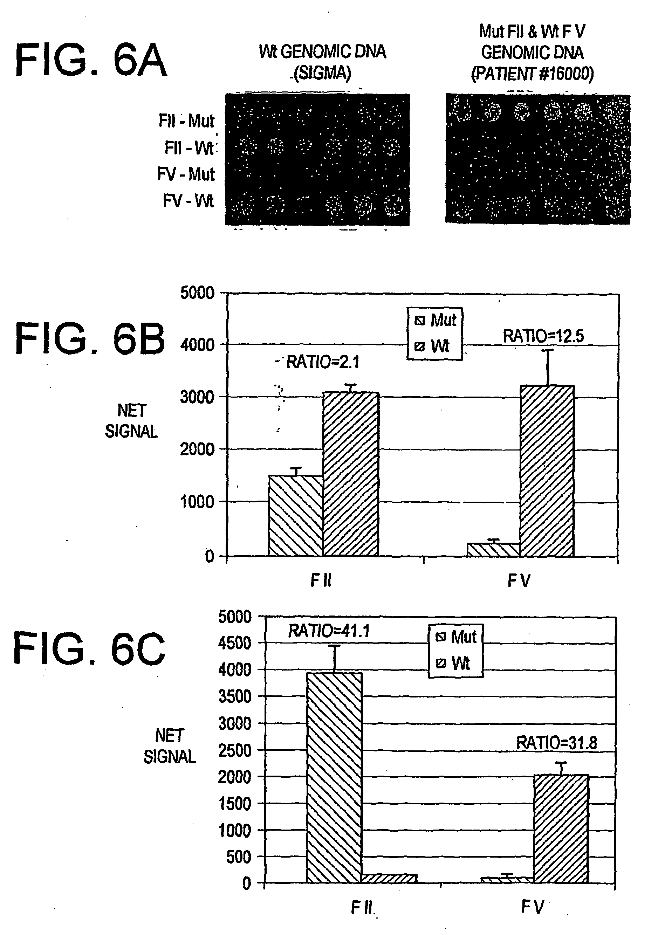

Figures 6 (a), (b), and (c) show that the array (capture probe sequences) and hybridization conditions can be designed such that more than one SNP type can be tested within the same array and under the same hybridization conditions, and that SNP discrimination is possible between wt and mutant DNA, independent of the input DNA.

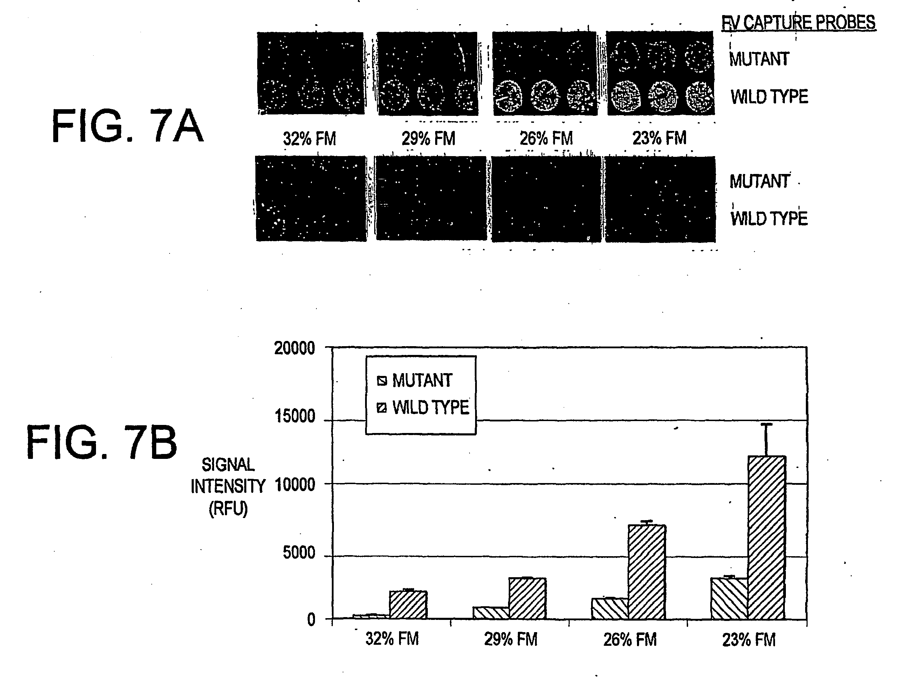

Figure 7 demonstrates SNP detection of the factor V mutant gene (1691 G ->A) with unamplified human genomic DNA (part (a)) using hybridizations with various formamide concentrations on CodeLink® slides that have arrayed wild-type and mutant factor V gene capture probes. Part (b) is a graph that summarizes the detection signal intensity analysis for human genomic DNA following hybridizations with various formamide concentrations in the presence of either the wild-type or mutant capture probes.

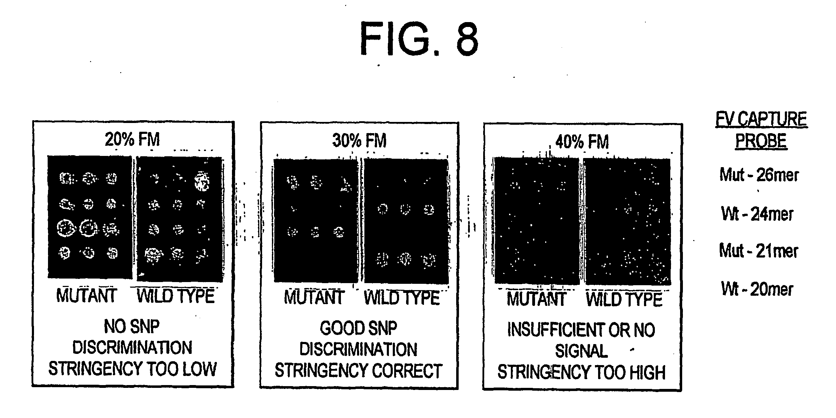

Figure 8 shows that under optimally tuned conditions, the human wt DNA generates a signal on the wt probes only, while the human mutant DNA generates a signal only at the mutant capture probes only.

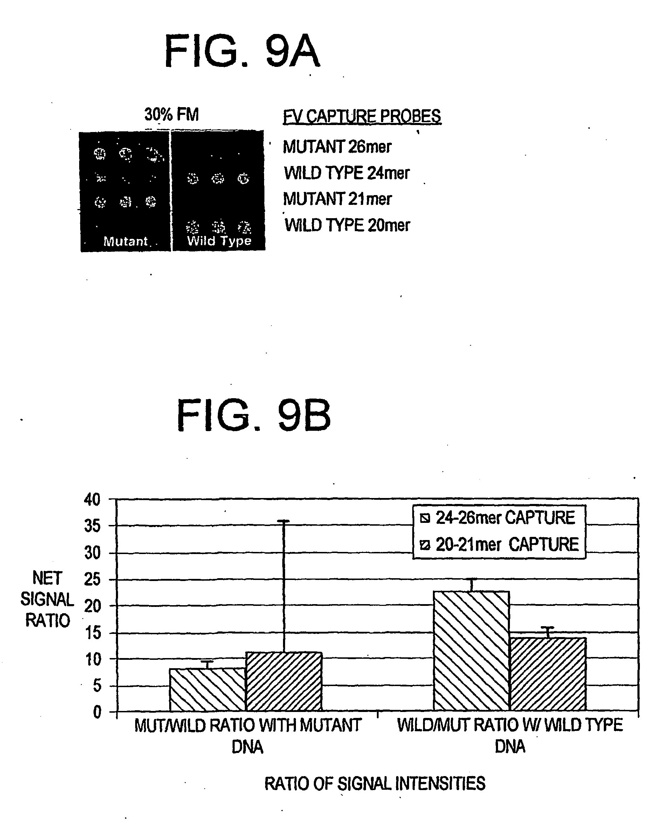

Figures 9 (a) - (d) show the quantitative data for the perfect (center) hybridization condition in Figure 8.

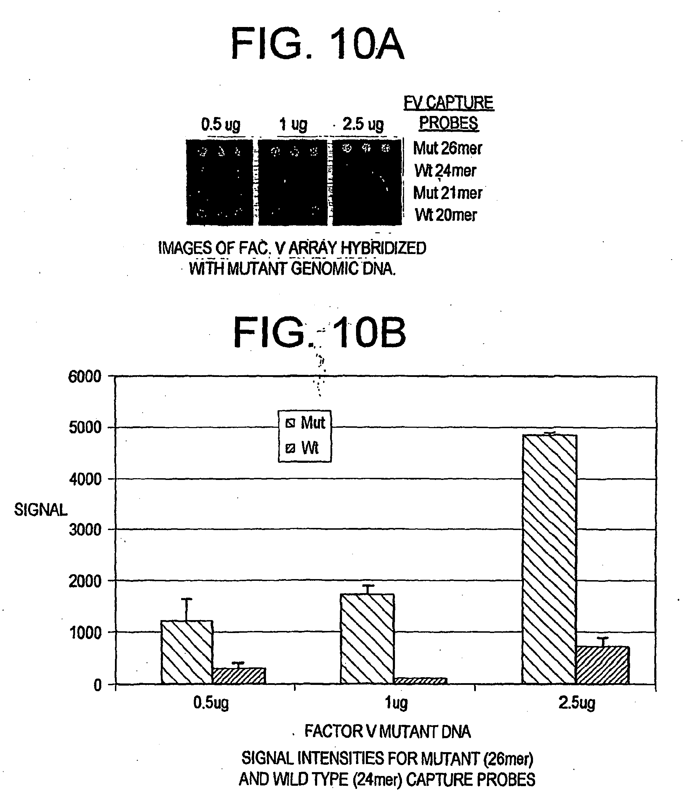

Figures 10 (a) and (b) show that SNP discrimination can be performed with very little (less than 1 microgram) total human DNA. It also demonstrates the importance of capture oligonucleotide design, and appropriate match of the stringency conditions to the length and nucleotide composition of the capture (and detection) probes.

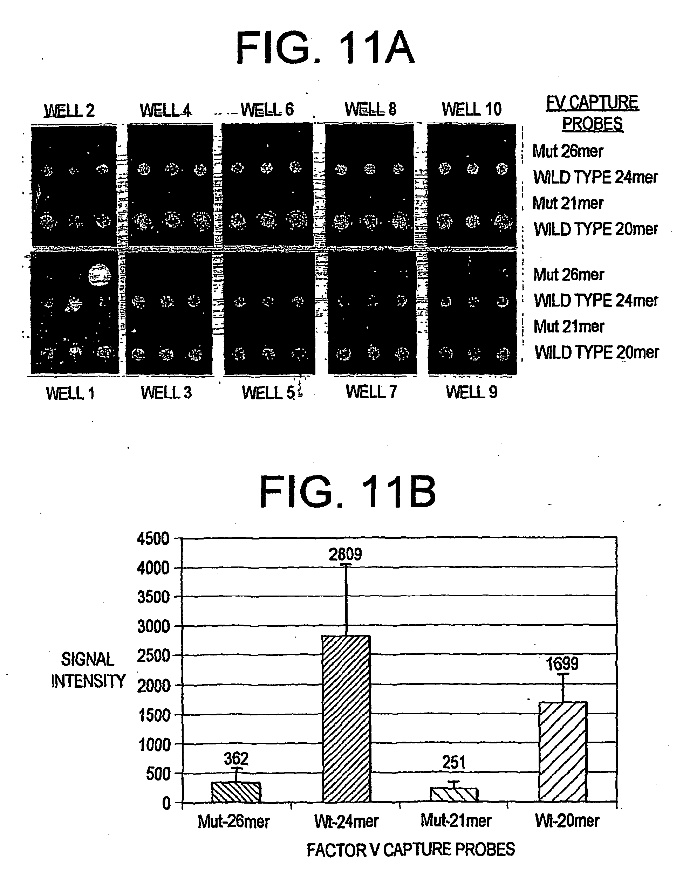

Figures 11 (a) and (b) show the results of SNP detection using methods of the invention in genomic DNA in 10 separate hybridizations on a single slide. The standard deviation of the net signal intensities for the match and mismatch in 10 separate hybridization wells did not overlap, meaning that for each hybridization reaction the SNP genotype of the input DNA could be reliably determined.

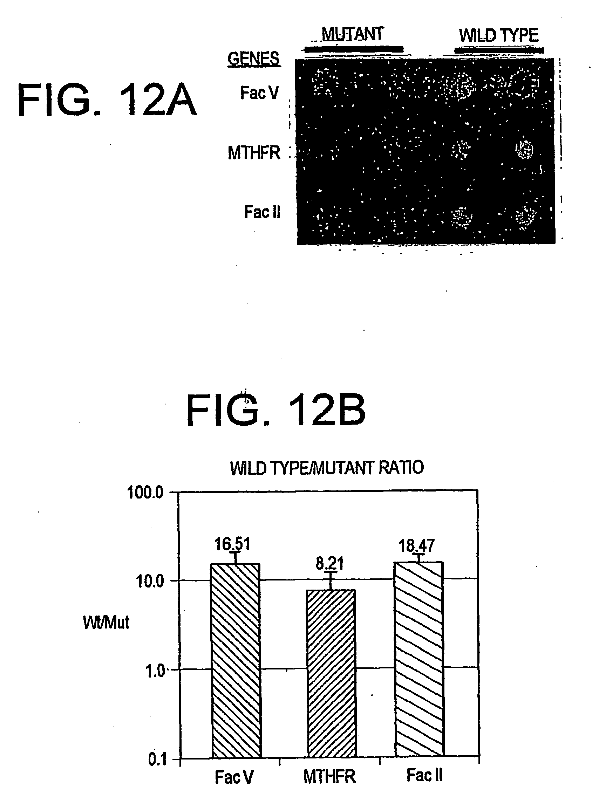

Figures 12 (a) and (b) show the results of multiplex SNP identification in whole genomic DNA using methods of the invention, which detected genotypes of factor V, factor II, and MTHFR genes.

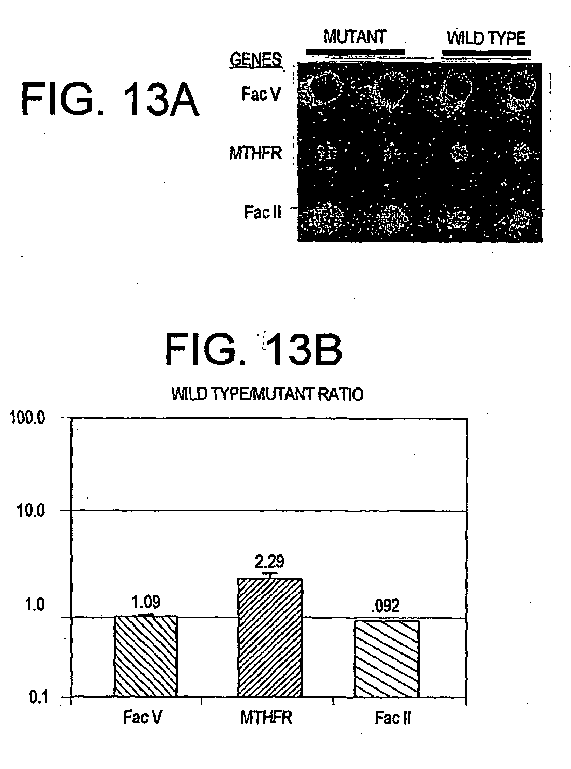

Figures 13 (a) and (b) show the results of multiplex SNP detection in whole genomic DNA from patient sample GM16028, which demonstrates the ability of the methods of the invention to identify heterozygous SNP genotypes for factor V, factor II, and MTHFR genes in a single individual.

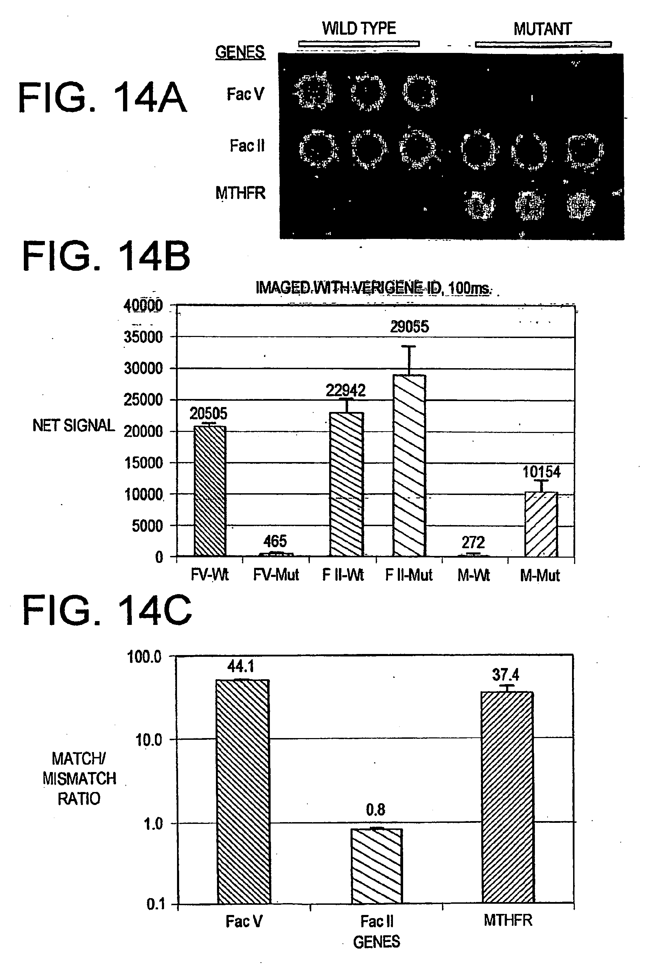

Figures 14 (a) and (c) show the results of multiplex SNP detection in whole genomic DNA from patient sample GM00037, which demonstrates the ability of the methods of the invention to identify that a single individual is wild type for one gene (in this case factor V), heterozygous for another gene (in this case factor II), and mutant for a third gene (in this case MTHFR).

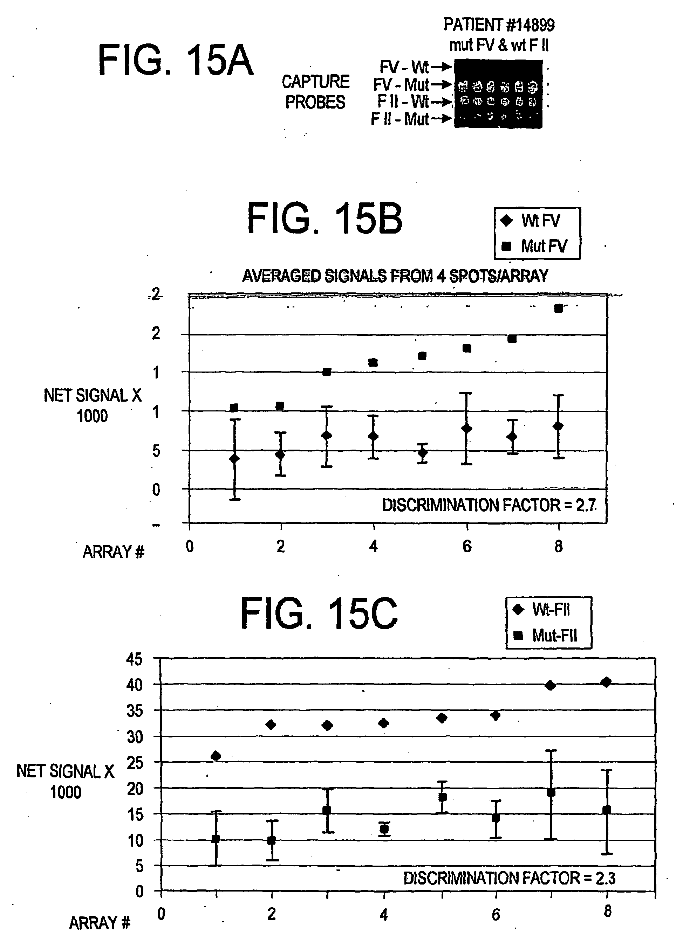

Figures 15 (a) - (f) show results from three different investigators performing the methods of the invention on two separate patient samples.

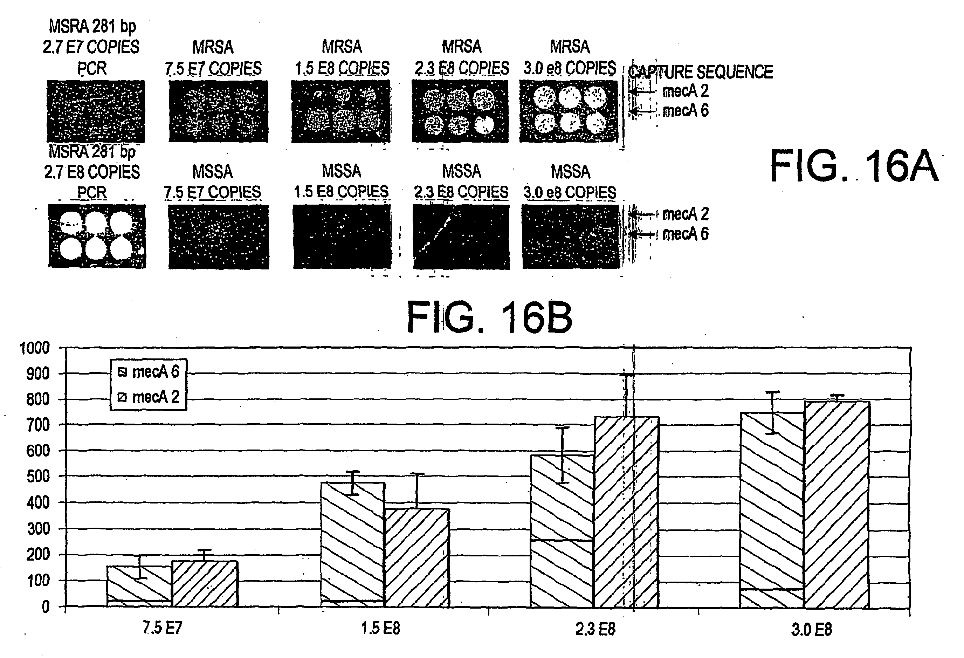

Figures 16 (a) - (b) illustrate the specific detection of a mecA gene from Staphylococcus genomic DNA isolated from methicillin resistant (mecA +) *S. aureus* bacterial cells using mecA 2 and mecA 6 capture oligonucleotides immobilized on a glass slide, and gold nanoparticles labeled with mecA 4 as a detection probe. Staphylococcus genomic DNA isolated from methicillin sensitive (mecA -) *S. aureus* bacterial cells was used as a negative control. A known amount of PCR amplified mecA gene (281 base-pair fragment labeled MRSA 281 bp) was used as a positive control. Part (a) illustrates a series of scanned images from wells of a microarray containing differing amounts of methicillin resistant genomic DNA target (75 - 300 million copies), as well as positive and negative control samples. Part (b) is a graph representing data analysis of the samples. The net signal from methicillin resistant *S. aureus* genomic DNA is plotted by subtracting the signal from the corresponding negative control spots. In all plots, the horizontal black line represents three standard deviations over the negative control spots containing methicillin sensitive *S. aureus* genomic DNA. The Figure demonstrates specific detection of the mecA from total bacterial genomic DNA.

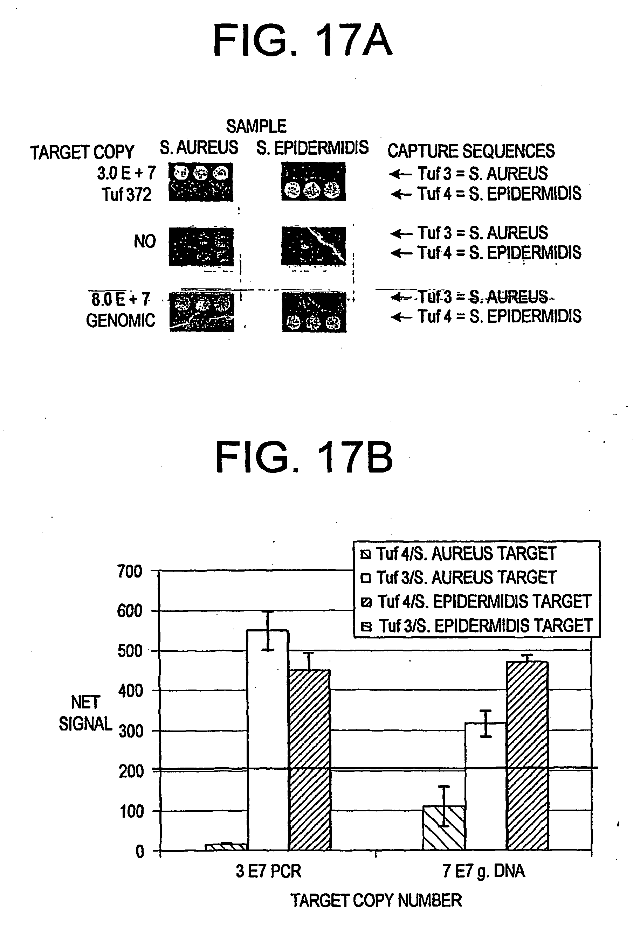

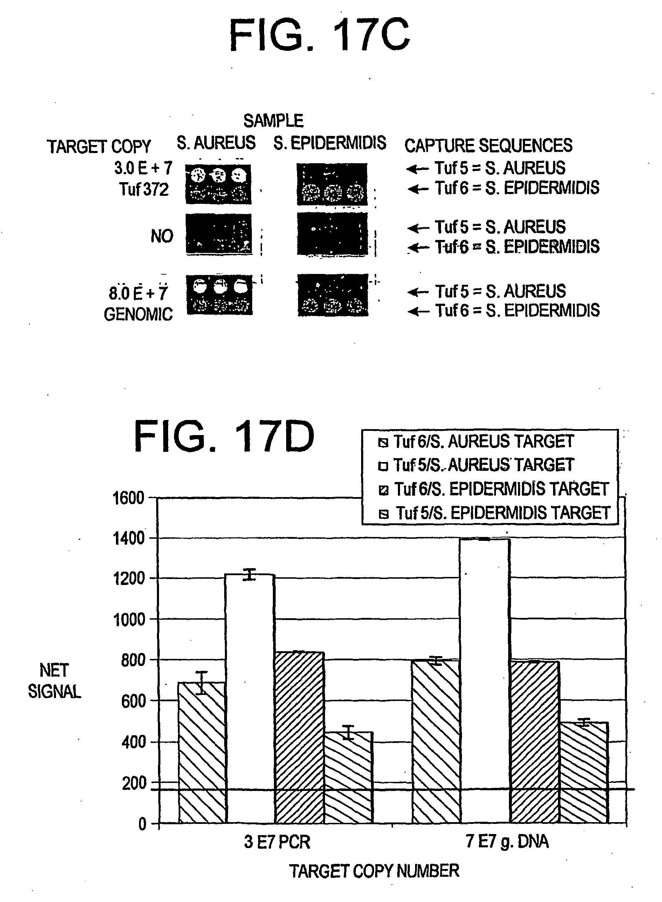

Figure 17 illustrates staphylococcal speciation using PCR amplicons or genomic DNA from *S. aureus* and *S. epidermidis* (ATCC no. 700699 and 35984, respectively). For testing of total genomic DNA, a sonication step was performed to fragment the DNA sample prior to array hybridization. Part (a) is a series of scanned images from wells from a microarray containing either Tuf 372 bp amplicons or genomic DNA (300 ng, ~ 8.0 E7 copies). Water (no target) was used as a control. The array plate included Tuf 3 and Tuf 4 capture probes bound thereto. Gold nanoparticle-labeled Tuf 2 probes were used as detection probes. Part (b) provides a graph representing data analysis of the samples shown in Part (a). The horizontal black line represents three standard deviations over the background. Part (c) Tuf 372 bp amplicons or genomic DNA (8.0 E7 copies). The array plated included Tuf 5 and Tuf 6 capture probes bound thereto. Part (d) provides a graph representing data analysis of the samples shown in Part (c). The horizontal black line represents three standard deviations over background.

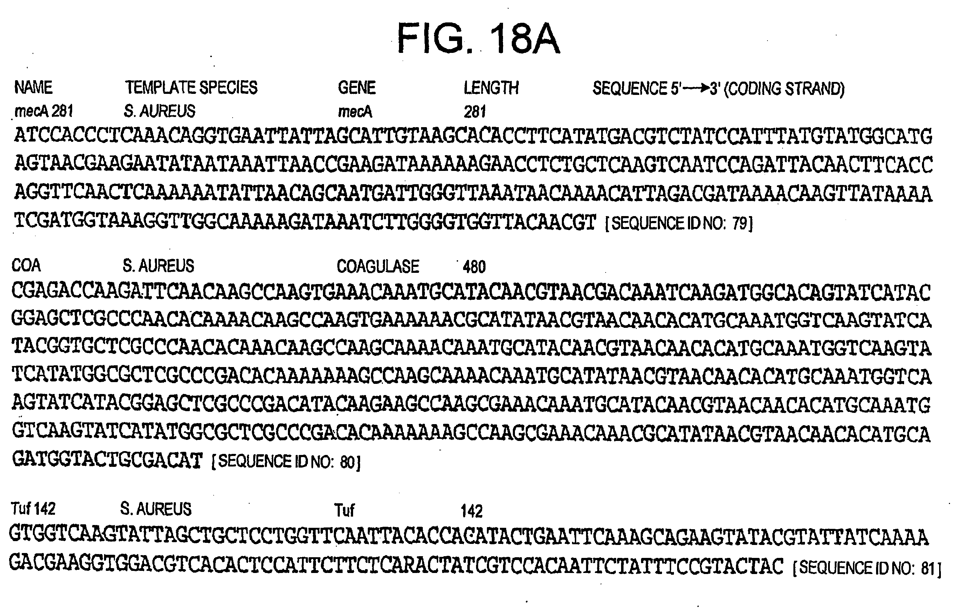

Figures 18 (a) - (c) provide the sequences of *S. aureus* mecA 281 base pair, *S. aureus* coa 450 base pairs, *S. aureus* Tuf 142 base pairs, *S. aureus* Tuf 372 base pair and *S. epidermidis* Tuf 372 base pair PCR amplicons used in examples 4-6..

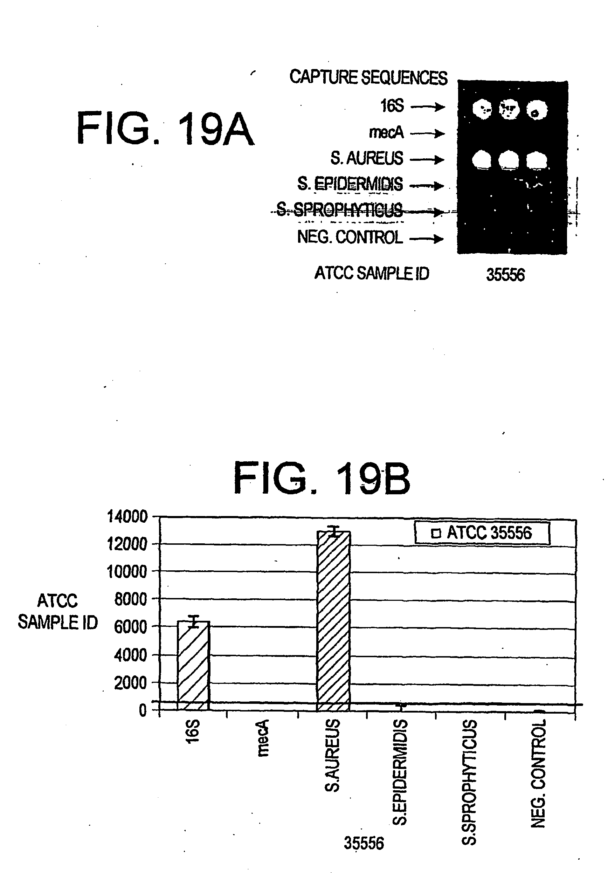

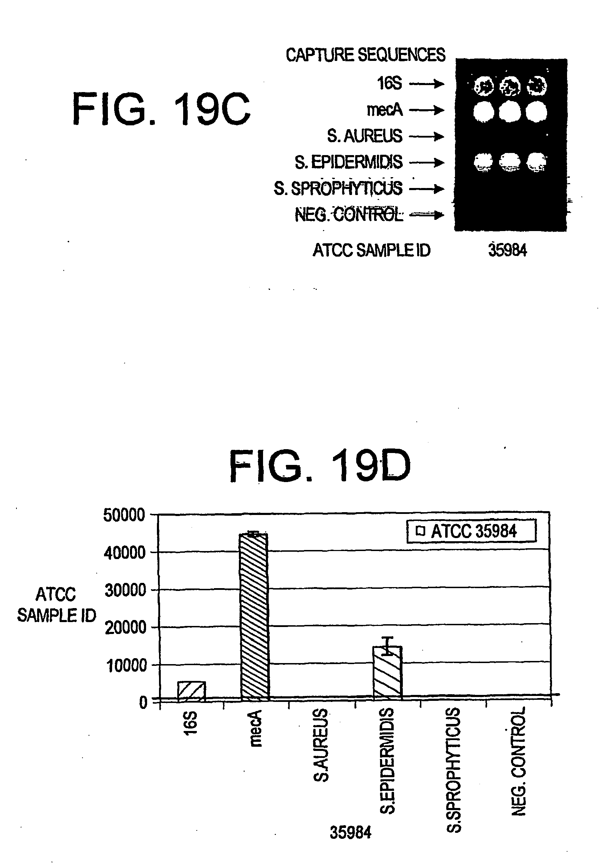

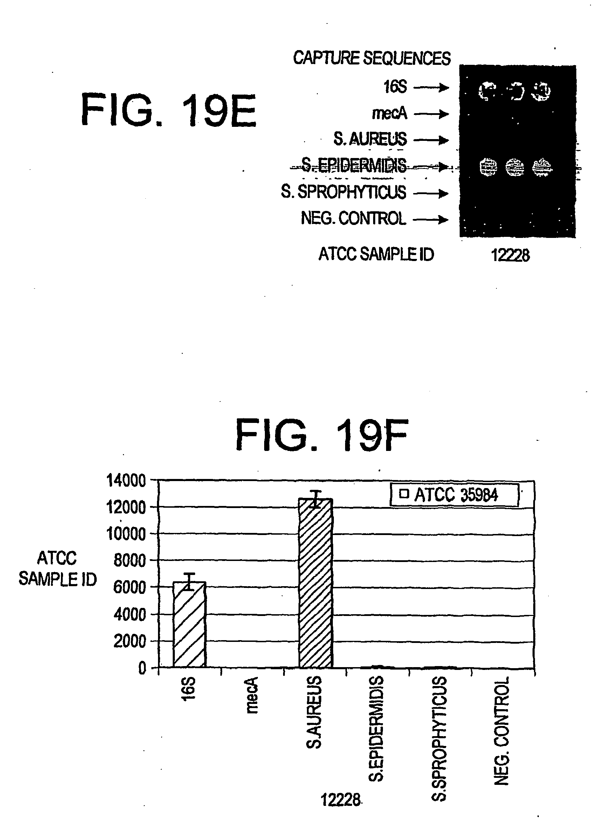

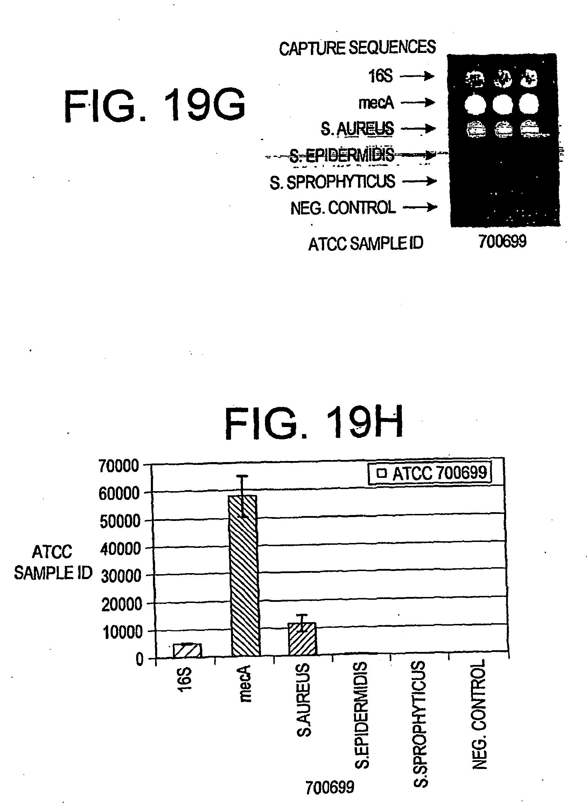

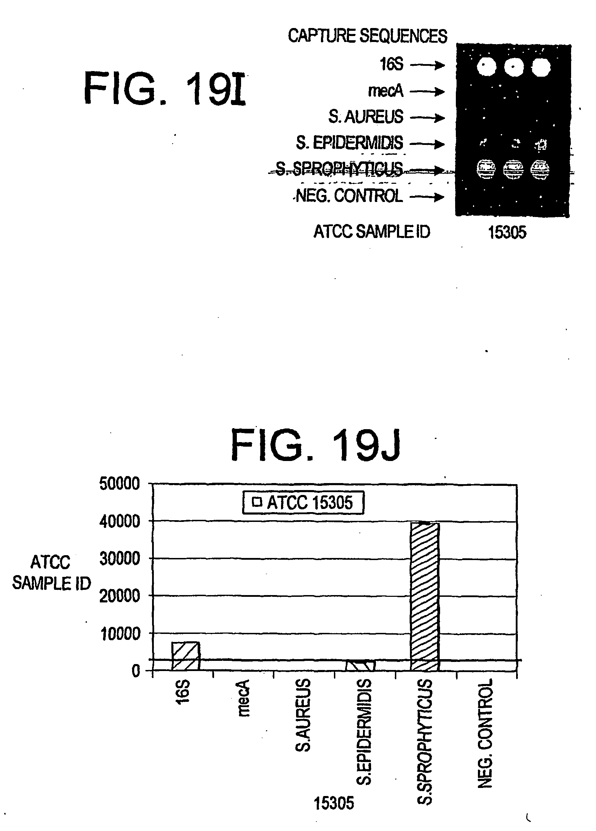

Figures 19 (a) - (j) illustrate staphylococcus speciation and mecA gene detection using PCR amplified targets taken from commercially available staphylococcus strains ATCC 35556, ATCC 35984, ATCC 12228, ATCC 700699, and ATCC 15305. Parts (a), (c), (e), (g), and (i) are a series of scanned images from wells from a microarray containing either the PCR products of the 16S, Tuf, or mecA genes representing the five genomic samples. Parts (b), (d), (f), and (h) are a series of graphs representing data analysis of the five samples. In all plots, a horizontal black line represents three standard deviations over the background.

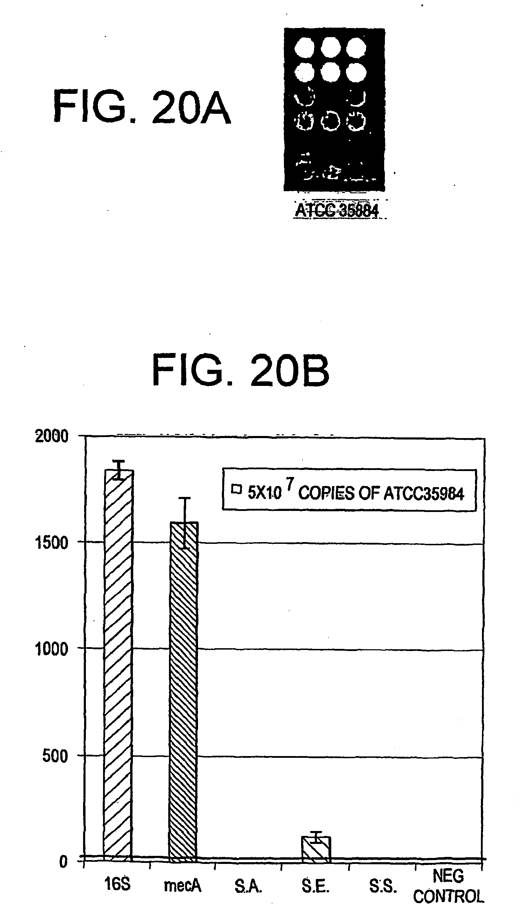

Figures 20 (a) - (f) illustrate staphylococcus speciation and mecA detection using sonicated genomic DNA targets taken from commercially available Staphylococcus strains ATCC 35984, ATCC 700699, and ATCC 12228. Parts (a), (c) and (e) are a series of scanned images from wells from a microarray containing either genomic DNA from ATCC 35984, ATCC 700699, or ATCC 12228. The array plate included 16S 12, mecA 6, Tuf 3, Tuf 4, Tuf 10 capture probes with a negative hybridization control bound thereto. Gold nanoparticle-labeled 16S 13, mecA 4, and Tuf 2 probes were used as detection probes. Parts (b), (d) and (f) are a series of graphs representing data analysis of the three samples. In all plots, a horizontal black line represents three standard deviations over the background.

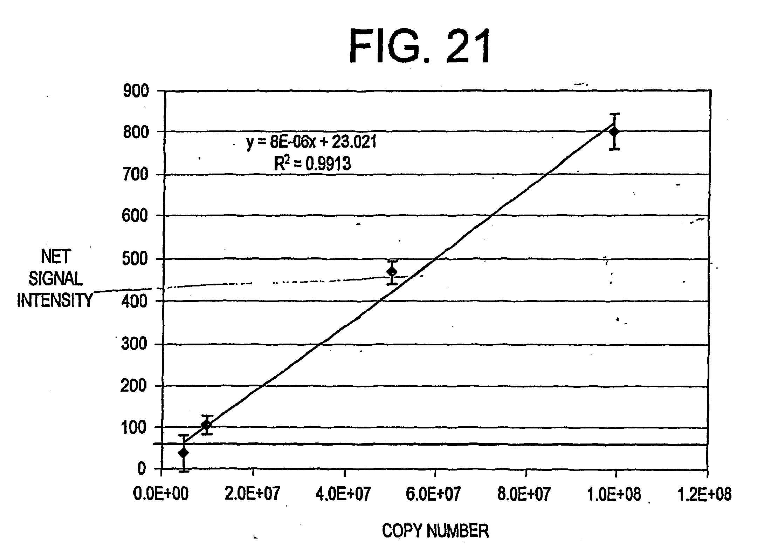

Figure 21 is a graph that illustrates the sensitivity limit for mecA gene detection using a genomic DNA target. Data analysis of mecA gene detection in a genomic sample of ATCC 700699 using the sequences from Table 3 in 5x SCC, 0.05% Tween 20, 0.01% BSA, 15% v/v formamide and 200 pM nanoparticle probe at 45C for 1.5 hours. The graph shows a limit of detection at 330 fM in a 50 $\mu$l reaction (34 ng total genomic DNA). Three standard deviations over the background is represented by the horizontal at 80 in the plot.

## DETAILED DESCRIPTION OF THE PREFERRED EMBODIMENTS

Unless otherwise required by context, singular terms shall include pluralities and plural terms shall include the singular.

As utilized in accordance with the present disclosure, the following terms, unless otherwise indicated, shall be understood to have the following meanings:

As used herein, a "nucleic acid sequence," a "nucleic acid molecule," or "nucleic acids" refers to one or more oligonucleotides or polynucleotides as defined herein. As used herein, a "target nucleic acid molecule" or "target nucleic acid sequence" refers to an oligonucleotide or polynucleotide comprising a sequence that a user of a method of the invention desires to detect in a sample.

The term "polynucleotide" as referred to herein means single-stranded or doublestranded nucleic acid polymers of at least 10 bases in length. In certain embodiments, the nucleotides comprising the polynucleotide can be ribonucleotides or deoxyribonucleotides or a modified form of either type of nucleotide. Said modifications include base modifications such as bromouridine, ribose modifications such as arabinoside and 2',3'-dideoxyribose and internucleotide linkage modifications such as phosphorothioate, phosphorodithioate, phosphoroselenoate, phosphorodiselenoate, phosphoroanilothioate, phoshoraniladate and phosphoroamidate. The term "polynucleotide" specifically includes single and double stranded forms of DNA.

The term "oligonucleotide" referred to herein includes naturally occurring, and modified nucleotides linked together by naturally occurring, and/or non-naturally occurring oligonucleotide linkages. Oligonucleotides are a polynucleotide subset comprising members that are generally single-stranded and have a length of 200 bases or fewer. In certain embodiments, oligonucleotides are 10 to 60 bases in length. In certain embodiments, oligonucleotides are 12, 13, 14, 15, 16, 17, 18, 19, or 20 to 40 bases in length. Oligonucleotides may be single stranded or double stranded, e.g. for use in the construction of a gene mutant. Oligonucleotides of the invention may be sense or antisense oligonucleotides with reference to a protein-coding sequence.

The term "naturally occurring nucleotides" includes deoxyribonucleotides and ribonucleotides. The term "modified nucleotides" includes nucleotides with modified or substituted sugar groups and the like. The term "oligonucleotide linkages" includes oligonucleotide linkages such as phosphorothioate, phosphorodithioate, phosphoroselenoate, phosphorodiselenoate, phosphoroanilothioate, phoshoraniladate, phosphoroamidate, and the like. *See*, *e.g.*, LaPlanche et al., 1986, Nucl. Acids Res., 14:9081; Stec et al., 1984, J. Am. Chem. Soc., 106:6077; Stein et al., 1988, Nucl. Acids Res., 16:3209; Zon et al., 1991, Anti-Cancer Drug Design, 6:539; Zon et al., 1991, OLIGONUCLEOTIDES AND ANALOGUES: A PRACTICAL APPROACH, pp. 87-108 (F. Eckstein, Ed.), Oxford University Press, Oxford England; Stec et al., U.S. Pat. No. 5,151,510; Uhlmann and Peyman, 1990, Chemical Reviews, 90:543, the disclosures of which are hereby incorporated by reference for any purpose. An oligonucleotide can include a detectable label to enable detection of the oligonucleotide or hybridization thereof.

An "addressable substrate" used in a method of the invention can be any surface capable of having oligonucleotides bound thereto. Such surfaces include, but are not limited to, glass, metal, plastic, or materials coated with a functional group designed for binding of oligonucleotides. The coating may be thicker than a monomolecular layer; in fact, the coating could involve porous materials of sufficient thickness to generate a porous 3-dimensional structure into which the oligonucleotides can diffuse and bind to the internal surfaces.

The term "capture oligonucleotide" as used herein refers to an oligonucleotide that is bound to a substrate and comprises a nucleic acid sequence that can locate (*i.e.* hybridize in a sample) a complementary nucleotide sequence or gene on a target nucleic acid molecule, thereby causing the target nucleic acid molecule to be attached to the substrate via the capture oligonucleotide upon hybridization. Suitable, but non-limiting examples of a capture oligonucleotide include DNA, RNA, PNA, LNA, or a combination thereof. The capture oligonucleotide may include natural sequences or synthetic sequences, with or without modified nucleotides.

A "detection probe" of the invention can be any carrier to which one or more detection oligonucleotides can be attached, wherein the one or more detection oligonucleotides comprise nucleotide sequences complementary to a particular nucleic acid sequence. The carrier itself may serve as a label, or may contain or be modified with a detectable label, or the detection oligonucleotides may carry such labels. Carriers that are suitable for the methods of the invention include, but are not limited to, nanoparticles, quantum dots, dendrimers, semi-conductors, beads, up- or down-converting phosphors, large proteins, lipids, carbohydrates, or any suitable inorganic or organic molecule of sufficient size, or a combination thereof

As used herein, a "detector oligonucleotide" or "detection oligonucleotide" is an oligonucleotide as defined herein that comprises a nucleic acid sequence that can be used to locate (*i.e.* hybridize in a sample) a complementary nucleotide sequence or gene on a target nucleic acid molecule. Suitable, but non-limiting examples of a detection oligonucleotide include DNA, RNA, PNA, LNA, or a combination thereof. The detection oligonucleotide may include natural sequences or synthetic sequences, with or without modified nucleotides.

As used herein, the terms "label" refers to a detectable marker that may be detected by photonic, electronic, opto-electronic, magnetic, gravity, acoustic, enzymatic, or other physical or chemical means. The term "labeled" refers to incorporation of such a detectable marker, *e.g.*, by incorporation of a radiolabeled nucleotide or attachment to an oligonucleotide of a detectable marker.

A "sample" as used herein refers to any quantity of a substance that comprises nucleic acids and that can be used in a method of the invention. For example, the sample can be a biological sample or can be extracted from a biological sample derived from humans, animals, plants, fungi, yeast, bacteria, viruses, tissue cultures or viral cultures, or a combination of the above. They may contain or be extracted from solid tissues (e.g. bone marrow, lymph nodes, brain, skin), body fluids (e.g. serum, blood, urine, sputum, seminal or lymph fluids), skeletal tissues, or individual cells. Alternatively, the sample can comprise purified or partially purified nucleic acid molecules and, for example, buffers and/or reagents that are used to generate appropriate conditions for successfully performing a method of the invention.

In one embodiment of the invention, the target nucleic acid molecules in a sample can comprise genomic DNA, genomic RNA, expressed RNA, plasmid DNA, cellular nucleic acids or nucleic acids derived from cellular organelles (e.g. mitochondria) or parasites, or a combination thereof.

As used herein, the "biological complexity" of a nucleic acid molecule refers to the number of non-repeat nucleotide sequences present in the nucleic acid molecule, as described, for example, in Lewin, GENE EXPRESSION 2, Second Edition: Eukaryotic Chromosomes, 1980, John Wiley & Sons, New York. For example, a simple oligonucleotide of 30 bases that contains a non-repeat sequence has a complexity of 30. The *E. coli* genome, which contains 4,200,000 base pairs, has a complexity of 4,200,000, because it has essentially no repeat sequences. The human genome, however, has on the order of 3,000,000,000 base pairs, much of which is repeat sequences (*e.g.* about 2,000,000,000 base pairs). The overall complexity (i.e. number of non-repeat nucleotides) of the human genome is on the order of 1,000,000,000.

The complexity of a nucleic acid molecule, such as a DNA molecule, does not depend on a number of different repeat sequences (*i.e.* copies of each different sequence present in the nucleic acid molecule). For example, if a DNA has 1 sequence that is *a* nucleotides long, 5 copies of a sequence that is *b* nucleotides long, and 50 copies of a sequence that is *c* nucleotides long, the complexity will be $a + b + c$, while the repetition frequencies of sequence *a* will be 1, *b* will be 5, and *c* will be 10.

The total length of different sequences within a given DNA can be determined experimentally by calculating the $C_0t_{1/2}$ for the DNA, which is represented by the following formula,

$$Cot_{1/2} = \frac{1}{k}$$

where $C$ is the concentration of DNA that is single stranded at time $t_{1/2}$ (when the reaction is 1/2 complete) and $k$ is the rate constant. A $C_0t_{1/2}$ represents the value required for half reassociation of two complementary strands of a DNA. Reassociation of DNA is typically represented in the form of Cot curves that plot the fraction of DNA remaining single stranded ($C/C_0$) or the reassociated fraction ($1-C/C_0$) against the log of the $C_0t$. Cot curves were introduced by Britten and Kohne in 1968 (1968, Science 161:529-540). Cot curves demonstrate that the concentration of each reassociating sequence determines the rate of renaturation for a given DNA. The $C_0t_{1/2}$, in contrast, represents the total length of different sequences present in a reaction.

The $C_0t_{1/2}$ of a DNA is proportional to its complexity. Thus, determining the complexity of a DNA can be accomplished by comparing its $C_0t_{1/2}$ with the $C_0t_{1/2}$ of a standard DNA of known complexity. Usually, the standard DNA used to determine biological complexity of a DNA is an *E. coli* DNA, which has a complexity identical to the length of its genome ($4.2 \times 10^6$ base pairs) since every sequence in the *E. coli* genome is assumed to be unique. Therefore, the following formula can be used to determine biological complexity for a DNA.

$$\frac{Cot_{1/2}\,(\text{any DNA})}{Cot_{1/2}\,(E.\,coli\,\text{DNA})} = \frac{\text{complexity}\,(\text{any DNA})}{4.2 \times 10^6}$$

Disclosed are methods for reliable detection and discrimination (*i.e.* identification) of a target nucleic molecule having a nucleotide mutation (for example, a single nucleotide polymorphism) in total human DNA without the need for enzymatic complexity reduction by PCR or any other method that preferentially amplifies a specific DNA sequence. Specifically, the methods comprise a combination of hybridization conditions (including reaction volume, salts, formamide, temperature, and assay format), capture oligonucleotide sequences bound to a substrate, a detection probe, and a sufficiently sensitive means for detecting a target nucleic acid molecule that has been recognized by both the capture oligonucleotide and the detection probe.

As demonstrated in the Examples, a successful method for detecting a single nucleotide polymorphism in total human DNA without prior amplification or complexity reduction to selectively enrich for the target sequence, and without the aid of any enzymatic reaction, by single-step hybridization, which encompasses two hybridization events: hybridization of a first portion of the target sequence to the capture probe, and hybridization of a second portion of said target sequence to the detection probe is disclosed. Figure 1 shows a schematic representation of the single-step hybridization. As discussed above, both hybridization events happen in the same reaction. The target can bind to a capture oligonucleotide first and then hybridize also to a detection probe, such as the nanoparticle shown in the schematic, or the target can bind the detection probe first and then the capture oligonucleotide.

Disclosed are methods for reliable detection and discrimination (*i.e.* identification) of a target nucleic acid molecule having one or more non-consecutive nucleotide mutations in total DNA without the need for enzymatic complexity reduction by PCR or any other method that preferentially amplifies a specific DNA sequence. For example, the methods can be used to distinguish between two or more target nucleic acid molecules from two or more different species of a common genus; wherein the species differ by two or more non-consecutive nucleotides using capture oligonucleotides that differ by one or more nucleotides and/or detection oligonucleotides that differ by one or more nucleotides. The methods can also be used to distinguish between two or more species of a common genus that differ by two or more consecutive nucleotides.

The methods can be accomplished using a two-step hybridization. Figure 2 shows a schematic representation of the two-step hybridization. In this process, the hybridization events happen in two separate reactions. The target binds to the capture oligonucleotides first, and after removal of all non-bound nucleic acids, a second hybridization is performed that provides detection probes that can specifically bind to a second portion of the captured target nucleic acid.

Methods that involve the two-step hybridization will work without accommodating certain unique properties of the detection probes (such as high Tm and sharp melting behavior of nanoparticle probes) during the first hybridization event (*i.e.* capture of the target nucleic acid molecule) since the reaction occurs in two steps. The first step is not sufficiently stringent to capture only the desired target sequences. Thus, the second step (binding of detection probes) is then provided to achieve the desired specificity for the target nucleic acid molecule. The combination of these two discriminating hybridization events allows the overall specificity for the target nucleic acid molecule. However, in order to achieve this exquisite specificity the hybridization conditions are chosen to be very stringent. Under such stringent conditions, only a small amount of target and detection probe gets captured by the capture probes. This amount of target is typically so small that it escapes detection by standard fluorescent methods because it is buried in the background. It is therefore critical for this invention to detect this small amount of target using an appropriately designed detection probe. The detection probes described consist in a carrier portion that is typically modified to contain many detection oligonucleotides, which enhances the hybridization kinetics of this detection probe. Second, the detection probe is also labeled with one or more high sensitivity label moieties, which together with the appropriate detection instrument, allows for the detection of the small number of captured target-detection probe complexes. Thus, it is the appropriate tuning of all factors in combination with a high sensitivity detection system that allows this process to work.

The two-step hybridization methods can comprise using any detection probes as described herein for the detection step. In a preferred embodiment, nanoparticle probes are used in the second step of the method. Where nanoparticles are used and the stringency conditions in the second hybridization step are equal to those in the first step, the detection oligonucleotides on the nanoparticle probes can be longer than the capture oligonucleotides. Thus, conditions necessary for the unique features of the nanoparticle probes (high Tm and sharp melting behavior) are not needed.

The single- and two-step hybridization methods in combination with the appropriately designed capture oligos and detection probes of the invention provide new and unexpected advantages over previous methods of detecting target nucleic acid sequences in a sample. Specifically, the methods do not require an amplification step to maximize the number of targets and simultaneously reduce the relative concentration of non-target sequences in a sample to enhance the possibility of binding to the target, as required, for example, in polymerase chain reaction (PCR) based detection methods. Specific detection without prior target sequence amplification provides tremendous advantages. For example, amplification often leads to contamination of research or diagnostic labs, resulting in false positive test outcomes. PCR or other target amplifications require specifically trained personnel, costly enzymes and specialized equipment. Most importantly, the efficiency of amplification can vary with each target sequence and primer pair, leading to errors or failures in determining the target sequences and/or the relative amount of the target sequences present in a genome. In addition, the methods of the invention involve fewer steps and are thus easier and more efficient to perform than gel-based methods of detecting nucleic acid targets, such as Southern and Northern blot assays.

Disclosed is a method for detecting a target nucleic acid sequence in a sample, wherein the sample comprises nucleic acid molecules of higher biological complexity than that of amplified nucleic acid molecules and the target nucleic acid sequence differs from a known nucleic acid sequence by at least a single nucleotide, the method comprising the steps of: a) providing an addressable substrate having a capture oligonucleotide bound thereto, wherein the capture oligonucleotide can recognize at least part of a first portion of the target nucleic acid sequence; b) providing a detection probe comprising detector oligonucleotides, wherein the detector oligonucleotides can hybridize with at least part of a second portion of the target nucleic acid sequence of step (a); c) contacting the sample with the substrate and the detection probe under conditions that are effective for the specific and selective hybridization of the capture oligonucleotide to the first portion of the target nucleic acid sequence and the specific and selective hybridization of the detection probe to the second portion of the target nucleic acid sequence; and d) detecting whether the capture oligonucleotide and detection probe hybridized with the first and second portions of the target nucleic acid sequence. The addressable substrate can have a plurality of capture oligonucleotides bound thereto that can recognize multiple portions of the target nucleic acid sequence and one or more detector probes comprising detector oligonucleotides that can hybridize with one or more portions of the target nucleic acid sequence that are not recognized by the capture oligonucleotides.

Disclosed is a method for identifying a single nucleotide polymorphism in a sample, wherein the sample comprises nucleic acid molecules of higher biological complexity than that of amplified nucleic acid molecules, the method comprising the steps of: a) providing an addressable substrate having at least one capture oligonucleotide bound thereto, wherein the at least one capture oligonucleotide can recognize a nucleic acid target that comprises a specific polymorphism; b) providing a detector probe having detector oligonucleotides bound thereto, wherein the detector oligonucleotides can hybridize with at least a portion of the nucleic acid target of step (a); c) contacting the sample with the substrate and the detector probe under conditions that are effective for the specific and selective hybridization of the capture oligonucleotide to the nucleic acid target and the hybridization of the detector probe to the nucleic acid target; and d) detecting whether the capture oligonucleotide and detector probe hybridized with the nucleic acid target. The addressable substrate can have a plurality of capture oligonucleotides bound thereto that can recognize multiple portions of the target nucleic acid sequence and the detector probe comprises detector oligonucleotides that can hybridize with a portion of the target nucleic acid sequence that is not recognized by the capture oligonucleotides.

The methods can discriminate between two sequences that differ by as little as one nucleotide. Thus, the methods can be used to detect a specific target nucleic acid molecule that has a mutation of at least one nucleotide. The mutation can be a single nucleotide polymorphism (SNP).

A detector oligonucleotide can be detectably labeled. Various methods of labeling polynucleotides are known in the art and may be used advantageously in the methods disclosed herein. In a particular embodiment, a detectable label of the invention can be fluorescent, luminescent, Raman active, phosphorescent, radioactive, or efficient in scattering light, have a unique mass, or other has some other easily and specifically detectable physical or chemical property, and in order to enhance said detectable property the label can be aggregated or can be attached in one or more copies to a carrier, such as a dendrimer, a molecular aggregate, a quantum dot, or a bead. The label can allow for detection, for example, by photonic, electronic, acoustic, opto-acoustic, gravity, electro-chemical, enzymatic, chemical, Raman, or mass-spectrometric means.

A detector probe can be a nanoparticle probe having detector oligonucleotides bound thereto. Nanoparticles have been a subject of intense interest owing to their unique physical and chemical properties that stem from their size. Due to these properties, nanoparticles offer a promising pathway for the development of new types of biological sensors that are more sensitive, more specific, and more cost effective than conventional detection methods. Methods for synthesizing nanoparticles and methodologies for studying their resulting properties have been widely developed over the past 10 years (Klabunde, editor, Nanoscale Materials in Chemistry, Wiley Interscience, 2001). However, their use in biological sensing has been limited by the lack of robust methods for functionalizing nanoparticles with biological molecules of interest due to the inherent incompatibilities of these two disparate materials. A highly effective method for functionalizing nanoparticles with modified oligonucleotides has been developed. See U.S. Patent Nos. 6,361,944 and 6,417,340 (assignee: Nanosphere, Inc.). The process leads to nanoparticles that are heavily functionalized with oligonucleotides, which have surprising particle stability and hybridization properties. The resulting DNA-modified particles have also prove to be very robust as evidenced by their stability in solutions containing elevated electrolyte concentrations, stability towards centrifugation or freezing, and thermal stability when repeatedly heated and cooled. This loading process also is controllable and adaptable. Nanoparticles of differing size and composition have been functionalized, and the loading of oligonucleotide recognition sequences onto the nanoparticle can be controlled via the loading process. Suitable, but non-limiting examples of nanoparticles include those described U.S. Patent No. 6,506,564; International Patent Application No. PCT/US02116382; U.S. Patent Application Serial No. 10/431,341 filed May 7, 2003; and International Patent Application No. PCT/U503/14100.

The aforementioned loading method for preparing DNA-modified nanoparticles, particularly DNA-modified gold nanoparticle probes, has led to the development of a new colorimetric sensing scheme for oligonucleotides. This method is based on the hybridization of two gold nanoparticle probes to two distinct regions of a DNA target of interest. Since each of the probes are functionalized with multiple oligonucleotides bearing the same sequence, the binding of the target results in the formation of target DNA/gold nanoparticle probe aggregate when sufficient target is present. The DNA target recognition results in a colorimetric transition due to the decrease in interparticle distance of the particles. This colorimetric change can be monitored optically, with a UV-vis spectrophotometer, or visually with the naked eye. In addition, the color is intensified when the solutions are concentrated onto a membrane. Therefore, a simple colorimetric transition provides evidence for the presence or absence of a specific DNA sequence. Using this assay, femtomole quantities and nanomolar concentrations of model DNA targets and polymerase chain reaction (PCR) amplified nucleic acid sequences have been detected. Importantly, it has been demonstrated that gold probe/DNA target complexes exhibit extremely sharp melting transitions which makes them highly specific labels for DNA targets. In a model system, one base insertions, deletions, or mismatches were easily detectable via the spot test based on color and temperature, or by monitoring the melting transitions of the aggregates spectrophotometrically (Storhoff et. al, J. Am. Chem. Soc., 120, 1959 (1998). See also, for instance, U.S. Patent No. 5,506,564.

Due to the sharp melting transitions, the perfectly matched target could be detected even in the presence of the mismatched targets when the hybridization and detection was performed under extremely high stringency (*e.g.*, a single degree below the melting temperature of the perfect probe/target match). It is important to note that with broader melting transitions such as those observed with molecular fluorophore labels, hybridization and detection at a temperature close to the melting temperature would result in significant loss of signal due to partial melting of the probe/target complex leading to lower sensitivity, and also partial hybridization of the mismatched probe/target complexes leading to lower specificity due to mismatched probe signal. Therefore, nanoparticle probes offer higher specificity detection for nucleic acid detection method.

As described herein, nanoparticle probes, particularly gold nanoparticle probes, are surprising and unexpectedly suited for direct SNP detection with genomic DNA and without amplification. First, the extremely sharp melting transitions observed in nanoparticle oligonucleotide detection probe translate to a surprising and unprecedented assay specificity that could allow single base discrimination even in a human genomic DNA background. Second, a silver-based signal amplification procedure in a DNA microarray-based assay can further provide ultra-high sensitivity enhancement.

A nanoparticle can be detected in a method of the invention, for example, using an optical or flatbed scanner. The scanner can be linked to a computer loaded with software capable of calculating grayscale measurements, and the grayscale measurements are calculated to provide a quantitative measure of the amount of nucleic acid detected.

Suitable scanners include those used to scan documents into a computer which are capable of operating in the reflective mode (*e.g.*, a flatbed scanner), other devices capable of performing this function or which utilize the same type of optics, any type of greyscale-sensitive measurement device, and standard scanners which have been modified to scan substrates according to the invention (*e.g.*, a flatbed scanner modified to include a holder for the substrate) (to date, it has not been found possible to use scanners operating in the transmissive mode): The resolution of the scanner must be sufficient so that the reaction area on the substrate is larger than a single pixel of the scanner. The scanner can be used with any substrate, provided that the detectable change produced by the assay can be observed against the substrate (*e.g.*, a gray spot, such as that produced by silver staining, can be observed against a white background, but cannot be observed against a gray background). The scanner can be a black-and-white scanner or, preferably, a color scanner.

Most preferably, the scanner is a standard color scanner of the type used to scan documents into computers. Such scanners are inexpensive and readily available commercially. For instance, an Epson Expression 636 (600 x 600 dpi), a UMAX Astra 1200 (300 x 300 dpi), or a Microtec 1600 (1600 x 1600 dpi) can be used. The scanner is linked to a computer loaded with software for processing the images obtained by scanning the substrate. The software can be standard software which is readily available commercially, such as Adobe Photoshop 5.2 and Corel Photopaint 8.0. Using the software to calculate greyscale measurements provides a means of quantitating the results of the assays.

The software can also provide a color number for colored spots and can generate images (*e.g.*, printouts) of the scans, which can be reviewed to provide a qualitative determination of the presence of a nucleic acid, the quantity of a nucleic acid, or both. In addition, it has been found that the sensitivity of assays can be increased by subtracting the color that represents a negative result from the color that represents a positive result.

The computer can be a standard personal computer, which is readily available commercially. Thus, the use of a standard scanner linked to a standard computer loaded with standard software can provide a convenient, easy, inexpensive means of detecting and quantitating nucleic acids when the assays are performed on substrates. The scans can also be stored in the computer to maintain a record of the results for further reference or use. Of course, more sophisticated instruments and software can be used, if desired.

Silver staining can be employed with any type of nanoparticles that catalyze the reduction of silver. Preferred are nanoparticles made of noble metals (*e.g.*, gold and silver). See Bassell, et al., J. Cell Biol., 126, 863-876 (1994); Braun-Howland et al., Biotechniques, 13, 928-931 (1992). If the nanoparticles being employed for the detection of a nucleic acid do not catalyze the reduction of silver, then silver ions can be complexed to the nucleic acid to catalyze the reduction. See Braun et al., Nature, 391, 775 (1998). Also, silver stains are known which can react with the phosphate groups on nucleic acids.

Silver staining can be used to produce or enhance a detectable change in any assay performed on a substrate, including those described above. In particular, silver staining has been found to provide a huge increase in sensitivity for assays employing a single type of nanoparticle so that the use of layers of nanoparticles, aggregate probes and core probes can often be eliminated.

Disclosed is that oligonucleotides attached to a substrate can be located between two electrodes, the nanoparticles can be made of a material that is a conductor of electricity, and step (d) in the methods of the invention can comprise detecting a changed in conductivity. A plurality of oligonucleotides, each of which can recognize a different target nucleic acid sequence, are attached to a substrate in an array of spots and each spot of oligonucleotides is located between two electrodes, the nanoparticles are made of a material that is a conductor of electricity, and step (d) in the methods of the invention comprises detecting a change in conductivity. The electrodes can be made, for example, of gold and the nanoparticles are made of gold. Alternatively, a substrate can be contacted with silver stain to produce a change in conductivity.

Disclosed is that nucleic acid molecules in a sample are of higher biological complexity than amplified nucleic acid molecules. One of skill in the art can readily determine the biological complexity of a target nucleic acid sequence using methods as described, for example, in Lewin, GENE EXPRESSION 2, Second Edition: Eukaryotic Chromosomes, 1980, John Wiley & Sons, New York.

Hybridization kinetics are absolutely dependent on the concentration of the reaction partners, *i.e.* the strands that have to hybridize. In a given quantity of DNA that has been extracted from a cell sample, the amount of total genomic, mitochondrial (if present), and extra-chromosomal elements (if present) DNA is only a few micrograms. Thus, the actual concentrations of the reaction partners that are to hybridize will depend on the size of these reaction partners and the complexity of the extracted DNA. For example, a target sequence of 30 bases that is present in one copy per single genome is present in different concentrations when comparing samples of DNA from different sources and with different complexities. For example, the concentration of the same target sequence in 1 microgram of total human DNA is about 1000 fold lower than in a 1 microgram bacterial DNA sample, and it would be about 1,000,000. fold lower than in a sample consisting in 1 microgram of a small plasmid DNA..

The high complexity ($1X10^9$ nucleotides) of the human genome demands an extraordinary high degree of specificity because of redundancies and similar sequences in genomic DNA. For example, to differentiate a capture strand with 25meric oligonuclotides from the whole human genome requires a degree of specificity with discrimination ability of 40,000,000:1. In addition, since the wild type and mutant targets differ only by one base in 25mer capture sequence, it requires distinguishing two targets with 96% homology for successful genotyping. The methods surprisingly and unexpectedly provide efficient, specific and sensitive detection of a target nucleic acid molecule having high complexity compared with amplified nucleic acid molecules.

The biological complexity of target nucleic acid molecules in a sample derived from human tissues is on the order of 1,000,000,000, but may be up to 10 fold higher or lower for genomes from plants or animals. Preferably, the biological complexity is about 50,000 to 5,000,000,000. Most preferably, the biological complexity is about 1,000,000,000.

In one embodiment, the hybridization conditions are effective for the specific and selective hybridization, whereby single base mismatches are detectable, of the capture oligonueleotide and/or the detector oligonucleotides to the target nucleic acid sequence, even when said target nucleic acid is part of a nucleic acid sample with a biological complexity of 50,000 or larger, as shown, for example, in the Examples below.

The methods can further be used for identifying specific species of a biological microorganism (e.g. Staphylococcus) and/or for detecting genes that confer antibiotic resistance (e.g. mecA gene which confers resistance to the antibiotic methicillin).

Methicillin resistant strains of Staphylococcus aureus (MRSA) have become first ranking nosocomial pathogens worldwide. These bacteria are responsible for over 40% or all hospital-bom staphylococcal infections in large teaching hospitals in the United States. Most recently they have become prevalent in smaller hospitals (20% incidence in hospitals with 200 to 500 beds), as well as in nursing homes (Wenzel et al., 1992, Am. J. Med. 91(Supp 3B):221-7). An unusual and most unfortunate property of MRSA strains is their ability to pick up additional resistance factors which suppress the susceptibility of these strains to other, chemotherapeutically useful antibiotics. Such multi-resistant strains of bacteria are now prevalent all over the world and the most "advanced" forms of these pathogens carry resistance mechanisms to most of the usable antibacterial agents (Blumberg et al., 1991, J. Inf. Disease, Vol.63, pp. 1279-85).

The central genetic element of methicillin resistance is the so called mecA gene. This gene is found on a piece of DNA of unknown, non-staphylococcal origin that the ancestral MRSA cell(s) must have acquired from a foreign source. The mecA gene encodes for a penicillin binding protein (PBP) called PBP2A (Murakami and Tomasz, 1989, J. Bacteriol. Vol. 171, pp. 874-79), which has very low affinity for the entire family of beta lactam antibiotics. In the current view, PBP2A is a kind of "surrogate" cell wall synthesizing enzyme that can take over the vital task of cell wall synthesis in staphylococci when the normal complement of PBPs (the normal catalysts of wall synthesis) can no longer function because they have become fully inactivated by beta lactam antibiotic in the environment. The critical nature of the mecA gene and its gene product PBP2A for the antibiotic resistant phenotype was demonstrated by early transposon inactivation experiments in which the transposon Tn551 was maneuvered into the mecA gene. The result was a dramatic drop in resistance level from the minimum inhibitory concentration (MIC) value of 1600 ug/ml in the parental bacterium to the low value of about 4 ug/ml in the transposon mutant (Matthews and Tomasz, 1990, Antimicrobial Agents and Chemotherapy, Vol. 34, pp.1777-9).

Staphylococcal infections acquired in hospital have become increasingly difficult to treat with the rise of antibiotic resistant strains, and the increasing number of infections caused by coagulase negative Staphylococcal species. Effective treatment of these infections is diminished by the lengthy time many tests require for the determination of species identification (speciation) and antibiotic resistance. With the rapid identification of both species and antibiotic resistance status, the course of patient treatment can be implemented earlier and with less use of broad-spectrum antibiotics. Accordingly, there is an apparent need for a rapid, highly sensitive and selective method for identifying and distinguishing Staphylococci species/or and for mecA gene detection.

Disclosed are oligonucleotide sequences and their reverse complements for Staphylococcal speciation and/or methicillin resistance gene (mecA) detection, nanoparticle-labeled probes, methods, and kits that employ these sequences. These sequences have been designed to be highly sensitive as well as selective for *Staphylococcal* species or the *mecA* gene, which gives rise to some forms of antibiotic resistance. These sequences can be used for the intended purpose of *mecA* gene detection or *Staphylococcal* speciation as well as negative controls for other systems. Currently, *S. aureus* can be differentiated from *S. epidermidis* using either sets probes Tuf 3 and 4, or Tuf 5 and 6 in combination with probe Tuf 2 as shown in the Examples below. Sequences labeled 16S are used to detect the presence of 16S rRNA or DNA contained within the genus *Staphylococcus.* Conventional methods such as standard phosphoramidite chemistry can be used to create these sequences both as capture probes and/or as nanoparticle labeled probes.

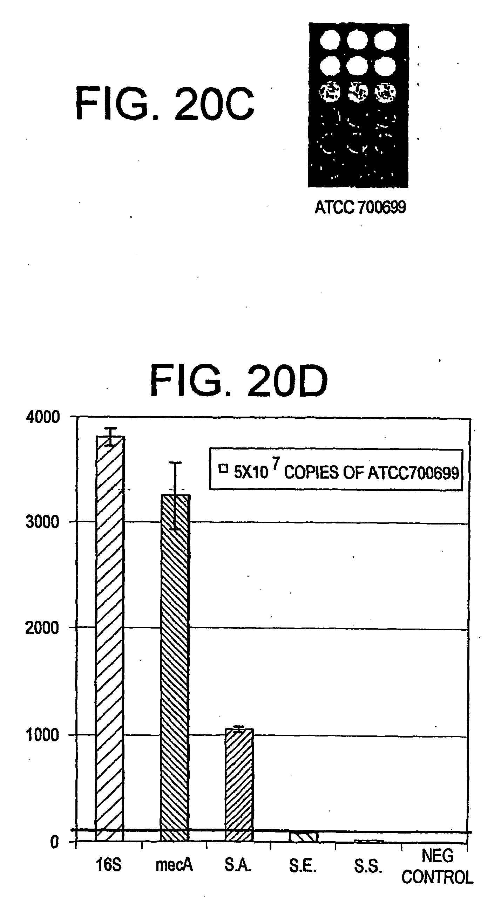

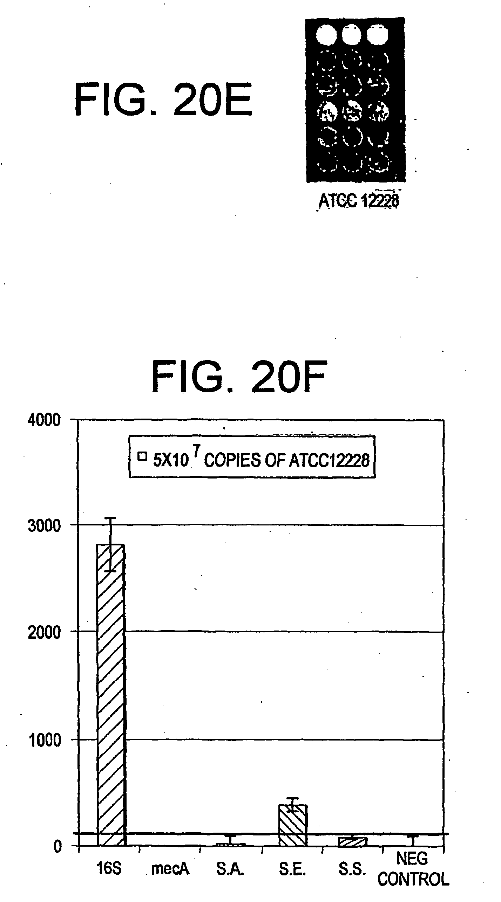

The sequences can be used in a method for staphylococcal speciation and/or mecA detection with unamplified genomic DNA. The mecA gene sequences of the invention have been used to detect as little as $1 \times 10^{-13}$ M (100 fM, 3 x $10^6$ copies) double stranded PCR products and 33 ng ($1 \times 10^7$ copies) of sonicated total genomic DNA in a 50 $\mu$l reaction for the mecA gene under the current assay conditions and format (see Figure21). The sensitivity and specificity of the *Tuf* gene sequences used for speciation of *S. aureus* and *S. epidermidis* also were tested in the assay using PCR amplified gene products or total bacterial genomic DNA. The current lower limit of detection was determined to be $1 \times 10^{-12}$ M (1 pM, or $3 \times 10^7$ copies) of double stranded PCR products and 150 ng ($5 \times 10^7$ copies) of sonicated genomic DNA in a 50 $\mu$l reaction (see Figure20). The conditions under which these assays were performed are described below. The methods surprisingly provide efficient, sensitive, and specific detection of different species of Staphylococcus by distinguishing DNA sequences that differ by a single base-pair or greater, using bacterial genomic DNA without prior complexity reduction or target amplification.

When using PCR amplicons, a second nanoparticle probe can be used in place of the capture sequence attached to the array substrate as described in PCT/US01/46418 (Nanosphere, Inc., Assignee). This system can be detected optically (*e.g.* color or light scattering) when target DNA hybridizes to both nanoparticle probes, which leads to a color change. This type of assay can be used for the purpose of *Staphylococcus* species identification or *mecA* gene detection as described for the above assays.

## EXAMPLES

The invention is demonstrated further by the following illustrative examples. In these examples all percentages are by weight if for solids and by volume if for liquids, and all temperatures are in degrees Celsius unless otherwise noted.

**Example 1**

**Single-step and Two-step hybridization methods for identifying SNPs in <u>unamplified genomic DNA using Nanoparticle probes</u>**

Gold nanoparticle-oligonucleotide probes to detect the target factor II, MTHFR and factor V sequences were prepared using procedures described in PCT/US97/12783, filed July 21, 1997; PCTIUS00/17507, filed June 26, 2000; PCT/US01/01190, filed January 12, 2001, which are incorporated by reference in their entirety. Figure 3 illustrates conceptually the use of gold nanoparticle probes having oligonucleotides bound thereto for detection of target DNA using a DNA microarray having wild type or mutant capture probe oligonucleotides. The sequence of the oligonucleotides bound to the nanoparticles are complementary to one portion of the sequence of target while the sequence of the capture oligonucleotides bound to the glass chip are complementary to another portion of the target sequence. Under hybridization conditions, the nanoparticle probes, the capture probes, and the target sequence bind to form a complex. Signal detection of the resulting complex can be enhanced with conventional silver staining.

*(a) Preparation Of Gold Nanoparticles*

Gold colloids (13 nm diameter) were prepared by reduction of $HAuCl_4$ with citrate as described in Frens, 1973, Nature Phys. Sci., 241:20 and Grabar, 1995, Anal. Chem.67:735. Briefly, all glassware was cleaned in aqua regia (3 parts HCl, 1 part $HNO_3$), rinsed with Nanopure $H_2O$ then oven dried prior to use. $HAuCl_4$ and sodium citrate were purchased from Aldrich Chemical Company. Aqueous $HAuCl_4$ (1 mM, 500 mL) was brought to reflux while stirring. Then, 38.8 mM sodium citrate (50 mL) was added quickly. The solution color changed from pale yellow to burgundy, and refluxing was continued for 15 min. After cooling to room temperature, the red solution was filtered through a Micron Separations Inc. 1 micron filter. Au colloids were characterized by UV-vis spectroscopy using a Hewlett Packard 8452A diode array spectrophotometer and by Transmission Electron Microscopy (TEM) using a Hitachi 8100 transmission electron microscope. Gold particles with diameters of 15 nm will produce a visible color change when aggregated with target and probe oligonucleotide sequences in the 10-35 nucleotide range.

### (b) Synthesis Of oligonucleotides

The capture probe oligonucleotides complementary to segments of the MTHFR, factor II or factor V DNA sequence were synthesized on a 1 micromole scale using a ABI 8909 DNA synthesizer in single column mode using phosphoramidite chemistry [Eckstein, F. (ed.) Oligonucleotides and Analogues: A Practical Approach (IRL Press, Oxford, 1991)]. The capture sequences contained either a 3'-amino modifier that serves as the active group for covalent attachment during the arraying process. The oligonucleotides were synthesized by following standard protocols for DNA synthesis. Columns with the 3'-amino modifier attached to the solid support, the standard nucleotide phosphoramidites and reagents were obtained from Glen Research. The final dimethoxytrityl (DMT) protecting group was not cleaved from the oligonucleotides to aid in purification. After synthesis, DNA was cleaved from the solid support using aqueous ammonia, resulting in the generation of a DNA molecule containing a free amine at the 3'-end. Reverse phase HPLC was performed with an Agilent 1100 series instrument equipped with a reverse phase column (Vydac) by using 0.03 M $Et_3NH^+$ $OAc^-$ buffer (TEAA), pH 7, with a 1%/min. gradient of 95% $CH_3CN$/5% TEAA. The flow rate was 1 mL/ min. with UV detection at 260 nm. After collection and evaporation of the buffer, the DMT was cleaved from the oligonucleotides by treatment with 80% acetic acid for 30 min at room temperature. The solution was then evaporated to near dryness; water was added, and the cleaved DMT was extracted from the aqueous oligonucleotide solution using ethyl acetate. The amount of oligonucleotide was determined by absorbance at 260 nm, and final purity assessed by analytical reverse phase HPLC.

The capture sequences employed in the assay for the MTHFR gene are as follows: MTHFR wild-type, 5' GATGAAATCGGCTCCCGCAGAC -$NH_2$ 3' (MTHFR-SNP/Cap6-WT22; SEQ ID NO: 1), and MTHFR mutant, 5' AT-GAAATCG_A_CTCCCGCAGACA-$NH_2$ 3' (MTHFR-SNP/Cap7-mut22; SEQ ID NO: 2). The corresponding capture oligonucleotides for the Factor V gene are as follows: Factor V wild type, 5' TGG ACA GGC GAG GAA TAC AGG TAT-$NH_2$ 3' (FV-Cap-WT24; SEQ ID NO: 3) and Factor V mutant, 5' CTG GAC AGG CAA GGA ATA CAG GTA TT-$NH_2$ 3' (FV-Cap-mut26; SEQ ID NO: 4). Factor II wild-type: 5' CTCAGCGAGCCTCAATGCTCCC-$NH_2$ 3' (FII-SNP/Cap1-WT22; SEQ ID NO: 5) and Factor II mutant, 5' CTCTCAGCAAGCCTCAATGCTCC - $NH_2$ 3' (FII-SNP/Cap1-mut23; SEQ ID NO: 6).

The detection probe oligonucleotides designed to detect Factor II, MTHFR and Factor V genes comprise a steroid disulfide linker at the 5'-end followed by the recognition sequence. The sequences for the probes are described: FII probe, 5' Epi-TCC TGG AAC CAA TCC CGT GAA AGA ATT ATT TTT GTG TTT CTA AAA CT 3' (FII-Pro I-47; SEQ ID NO: 7), MTHFR probe, 5'Epi-AAA GAT CCC GGG GAC GAT GGG GCA AGT GAT GCC CAT GTC GGT GCA TGC CTT CAC AAA G 3'(MTHFR-Pro II-58; SEQ ID NO: 8), Factor V probe, 5' Epi-CCA CAG AAA ATG ATG CCC AGT GCT TAA CAA GAC CAT ACT ACA GTG A 3' (FV-Pro 46; SEQ ID NO: 9).

The synthesis of the probe oligonucleotides followed the methods described for the capture probes with the following modifications. First, instead of the amino-modifier columns, supports with the appropriate nucleotides reflecting the 3'-end of the recognition sequence were employed. Second, the 5'-terminal steroid-cyclic disulfide was introduced in a coupling step by employing a modified phosphoramidite containing the steroid disulfide (see Letsinger et al., 2000, Bioconjugate Chem. 11:289-291 and PCT/US01/01190 (Nanosphere, Inc.), the disclosure of which is incorporated by reference in its entirety). The phosphoramidite reagent may be prepared as follows: a solution of epiandrosterone (0.5g), 1,2-dithiane-4,5-diol (0.28 g), and p-toluenesulfonic acid (15 mg) in toluene (30 mL) was refluxed for 7 h under conditions for removal of water (Dean Stark apparatus); then the toluene was removed under reduced pressure and the residue taken up in ethyl acetate. This solution was washed with water, dried over sodium sulfate, and concentrated to a syrupy residue, which on standing overnight in pentane/ether afforded a steroid-dithioketal compound as a white solid (400 mg); Rf (TLC, silica plate, ether as eluent) 0.5; for comparison, Rf values for epiandrosterone and 1,2-dithiane-4,5-diol obtained under the same conditions are 0.4, and 0.3, respectively. Recrystallization from pentane/ether afforded a white powder, mp 110-112 ˚C; $^1$H NMR, δ 3.6 (1H, $C^3OH$), 3.54-3.39 (2H, m 2OCH of the dithiane ring), 3.2-3.0 (4H, m $2CH_2S$), 2.1-0.7 (29H, m steroid H); mass spectrum (ES+) calcd for $C_{23}H_{36}O_3S_2$ (M+H) 425.2179, found 425.2151. Anal. ($C_{23}H_{37}O_3S_{2)}$ S: calcd, 15.12; found, 15.26. To prepare the steroid-disulfide ketal phosphoramidite derivative, the steroid-dithioketal (100 mg) was dissolved in THF (3 mL) and cooled in a dry ice alcohol bath. N,N-diisopropylethylamine (80 μL) and β- cyanoethyl chlorodiisopropylphosphoramidite (80 μL) were added successively; then the mixture was warmed to room temperature, stirred for 2 h, mixed with ethyl acetate (100 mL), washed with 5% aq. $NaHCO_3$ and with water, dried over sodium sulfate, and concentrated to dryness. The residue was taken up in the minimum amount of dichloromethane, precipitated at -70 ˚C by addition of hexane, and dried under vacuum; yield 100 mg; $^{31}$P NMR 146.02. After completion of the DNA synthesis, the epiandrosterone-disulfide linked oligonucleotides were deprotected from the support under aqueous ammonia conditions and purified on HPLC using reverse phase column as described above.

*(c) Attachment Of Oligonucleotides To Gold Nanoparticles*

The probe was prepared by incubating initially a 4 $\mu$M solution of the oligonucleotide with a ~14 nM solution of a 15 nm citrate-stabilized gold nanoparticle colloid solution in a final volume of 2 mL for 24 h. The salt concentration in this preparation was raised gradually to 0.8 M over a period of 40 h at room temperature. The resulting solution was passed through a 0.2 $\mu$m cellulose acetate filter and the nanoparticle probe was pelleted by spinning at 13,000 G for 20 min. After removing the supernatant, the pellet was re-suspended in water. In a final step, the probe solution was pelleted again and resuspended in a probe storage buffer (10 mM phos, 100 mM NaCl, 0.01% w/v $NaN_3$). The concentration was adjusted to 10 nM after estimating the concentration based on the absorbance at 520 nm ($\varepsilon=2.4\times10^8\,M^{-1}cm^{-1}$)

The following nanoparticle-oligonucleotide conjugates specific for factor II, MTHFR and factor V DNA were prepared in this manner:

Factor II Probe:  gold-S'-5'-[ TCC TGG AAC CAA TCC CGT GAA AGA ATT ATT TTT GTG TTT CTA AAA CT -3']$_n$ (FII-ProI-47; SEQ ID NO: 10)

MTHFR Probe:  gold-S'-5'-[ AAA GAT CCC GGG GAC GAT GGG GCA AGT GAT GCC CAT GTC GGT GCA TGC CTT CAC AAA G -3']$_n$ (MTHFR-II58; SEQ ID NO: 11)

Factor V Probe:  gold-S'-5'-[ CCA CAG AAA ATG ATG CCC AGT GCT TAA CAA GAC CAT ACT ACA GTG A -3']$_n$ (FV-46; SEQ ID NO: 12)

S' indicates a connecting unit prepared via an epiandrosterone disulfide group; n reflect the number of the recognition oligonucleotides.

*(d) Preparation of DNA microarrays*

Capture strands were arrayed on Superaldehyde slides (Telechem) or CodeLinke slides (Amersham, Inc.) by using a GMS417 arrayer (Affymetrix). The positioning of the arrayed spots was designed to allow multiple hybridization experiments on each slide, achieved by partitioning the slide into separate test wells by silicone gaskets (Grace Biolabs). The wild type and mutant spots were spotted in triplicate in manufacturer-provided spotting buffers. Protocols recommended by the manufacturer were followed for post-array processing of the slides.

*(e) Hybridization*

*Factor V SNP detection assay procedure*

The Factor V SNP detection was performed by employing the following protocol. Sonicated human placental DNA, genotyped as homozygous wild-type, or salmon sperm DNA (Sigma) was precipitated with ethanol and dissolved in a 10 nM solution of FV probe solution. Additional components were added to this mixture such that the final hybridization mixture (5 $\mu$L) contained 3xSSC, 0.03% Tween 20, 23% formamide, 5 nM FV probe, and 10 $\mu$g human DNA, or as indicated. The hybridization mixture was added to the test well after a 4 min, 99 °C heat denaturation step. The arrays were incubated at 50°C for 90 min. Post-hybridization washes were initiated by immersing arrays for 1 min in 0.5 M $NaNO_3$, 0.05% Tween 20 at room temperature. The gasket was removed and the test slide was washed again in 0.5M $NaNO_3$/0.05% Tween 20 solution and incubated at room temperature for 3 min (2x) with gentle agitation. The slides were stained with the silver enhancing solution as described above and dried on a spin dryer and imaged on an Array-Worx® biochip reader (Model no. AWE, Applied Precision Inc., Issaquah, WA, U.S.A.).

*(f) Results*

*Factor V SNP detection*

Figure 4 shows SNP discrimination of Factor V gene in human genomic DNA on Superaldehyde slides. The test array contains wild type and mutant capture spots. The array shown on the top was hybridized with wild-type human genomic DNA while the array on the bottom was hybridized with sonicated salmon sperm DNA. The signal at the wild-type spots is significantly higher than mutant spots with wild-type human genomic DNA hybridization to indicate a Factor V homozygous wild-type genotype. Under the hybridization conditions, no signal is observed for the salmon sperm DNA hybridization and serves as a control in the assay. SNP discrimination was also examined with arrays on CodeLink® slides.

The experiment was designed to show that the hybridization on the wt capture spots was not due to some other sequences, but was specific to a genome that contains the human factor V genre. Using total human wt DNA, the expected high hybridization signal was observed at the wt capture spots, and about 3 fold less signal was observed at the mutant spot. However, when the genomic DNA extracted from salmon sperm was used as target, no signals are observed, since this DNA does not contain the human factor V gene.

The importance of adjusting the hybridization conditions in order to make this process capable of discriminating between two target nucleic acids that differ by 1 nucleotide (the SNP site) is shown in Figure 5. An appropriate balance between formamide and SSC buffer salt concentration has to be determined such that the target sequence (in this case from a homozygous patient with a mutation in the Factor V gene) binds preferentially to its cognate capture probe (*i.e.* the Mut-A or Mut-B sequence). In addition, Figure 5 shows the effect of various sizes of capture oligonucleotide sequences in hybridization. The Mut-A sequence was 26 nucleotides long, while the Mut-B sequence was 21 nucleotides long. The results demonstrated a significant difference in the specific signal at the condition of 15% FM/1XSSC, but at 25% FM/6XSSC there was no difference and both probes generated a strong signal with good discrimination.

To determine if more than one SNP type could be detected in the same sample under the same conditions, genomic DNA was tested for the presence of wild type and mutant Factor II and Factor V genes. Normal human (wt) genomic DNA, capture oligonucleotides attached to a substrate, and nanoparticle probes were mixed together in 35% FM and 4X SSC at 40°C for one hour. A signal was generated preferentially at the wt capture spots for both, the Factor II and the Factor V gene (Figure 6). When using the total genomic DNA from an individual that was homozygous for a mutation in Factor II, but homozygous wt for Factor V, the same array under the same hybridization conditions gave a signal preferentially at the mutant capture spots for Factor II and on the wt capture spots for Factor V, clearly and correctly identifying the genetic make-up of this person with respect to his SNP configuration of these two genes (Figure 6). The results demonstrate that the capture oligonucleotide sequences and hybridization conditions can be designed so that more than one SNP type can be tested within the same array and under the same hybridization conditions. Also, SNP discrimination is possible between wt and mutant DNA, independent of whether the input DNA is from a normal or a mutant source.

*Two-Step Hybridization*

More experiments were conducted to determine the effect of various stringency conditions on SNP discrimination. Test arrays were hybridized at different stringencies by employing different percentages of formamide in the assay (Figure 7). With increasing stringency there is loss of signal, which translates to improved specificity of the signal. Almost no signal was observed in the no-target controls. Quantitation of the signals from the spots revealed a 3-6--fold higher signal for the wild-type spots over that for the mutant spots (Figure 7B). Together the results provide support for SNP discrimination in genomic DNA without the need for any target amplification strategies.

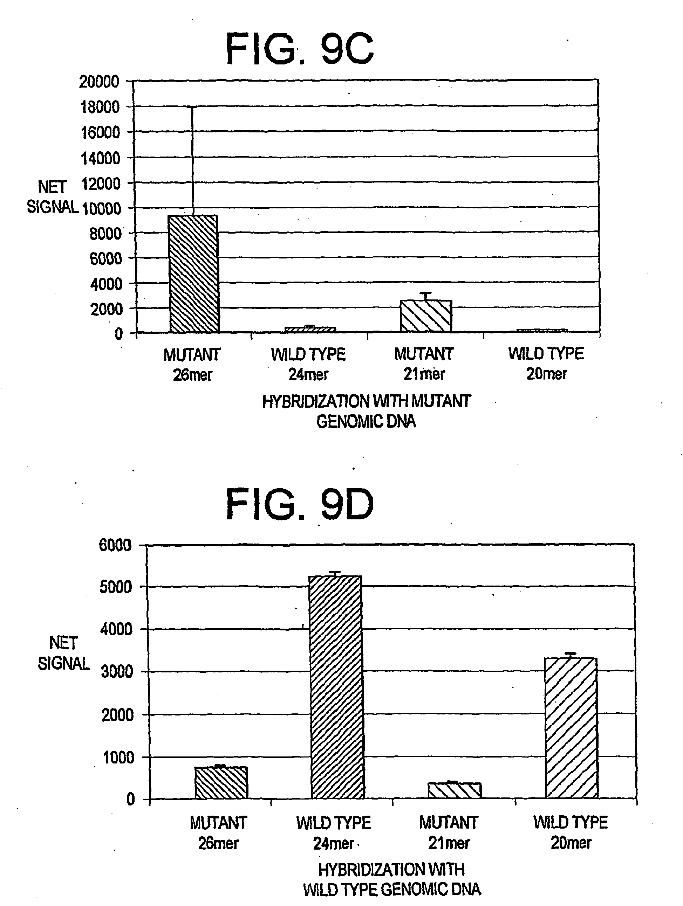

Capture oligonucleotides of various lengths, including 20, 21, 24, or 26 nucleotides (FV-WT20 (SEQ ID NO: 13): 5'(GGACAGGCGAGGAATACAGG)-(PEG)x3-NH$_2$, 3' FV-mut21 (SEQ ID NO: 14): 5'(TGGACAGGCAAGGAATA-CAGG)-(PEG)x3-NH$_2$ 3', FV-wt24 (SEQ ID NO: 15): 5' TGG ACA GGC GAG GAA TAC AGG TAT-NH$_2$ 3', FV-mut26 (SEQ ID NO: 16): 5' CTG GAC AGG CAA GGA ATA CAG GTA TT-NH$_2$ 3') were printed on CodeLink slides as described above and were added to 5 μg of normal human placenta genomic DNA (Sigma, St. Louis, MO) or factor V mutant human genomic DNA (isolated from repository culture GM14899, factor V deficiency, Coriell Institute). The slides and DNA were incubated in 20% FM, 30% FM, or 40% FM, and 4X SSC/0/04% Tween at 40°C for 2 hours in the first step. The slides were then washed in 2XSSC at room temperature for 3 minutes. After washing, nanoparticle probes with detection oligonucleotides that recognized Factor V were added and the mixture was then incubated for 1 hour at 40°C. The signal was detected by silver staining as described above. The results showed that under optimally tuned conditions (30% FM in this case), the human wt DNA generated a signal on the wt probes only, while the human mutant DNA generated a signal only at the mutant capture probes (Figure 8). Changing the stringency conditions resulted in either loss of discrimination (stringency too low) or loss of signal (stringency too high). Figure 9 shows the quantitative data for the perfect (center) hybridization condition in Figure 8.

The experiment was then repeated under the optimal conditions with various concentrations of DNA. As seen in Figure 10, SNP discrimination was successful when the concentration of DNA was 0.5 $\mu$g, 1.0 $\mu$g, and 2.5 $\mu$g. Thus, the method could detect the SNP with very little (less than 1 microgram) total human DNA. These results also demonstrated the importance of capture oligonucleotide design and the appropriate match of the stringency conditions to the length and nucleotide composition of the capture (and detection) probes.

The reproducibility of the two-step hybridization method was examined by performing 10 identical hybridization with 5 $\mu$g of wild type whole genomic DNA in separate wells on a single slide. The standard deviation of the net signal intensities for the match and mismatch in the 10 separate hybridization wells as shown in Figure 11 did not overlap, indicating that for each hybridization reaction, the SNP configuration of the input DNA could be reliably determined.

Next, the method was used to detect Factor V, Factor II, and MTHFR SNPs and wild type genes in the same sample preparation. Capture oligonucleotides for factor V, factor II, or MTHFR were incubated with 5 $\mu$g of whole genomic DNA under hybridization conditions described above. Nanoparticle probes specific for detecting factor V, factor II, or MTHFR were added in the second step. The results of this experiment, shown in Figures 12-14, showed that the SNP configuration of at least three different genes could be analyzed simultaneously in a single array, under the same conditions. Figure 13 shows the results of this multiplex SNP detection in a patient DNA sample (GM16028) that was heterozygous for each gene. Figure 14 shows the results of the multiplex SNP detection in a patient who was heterozygous for factor II, wild type for factor V, and mutant for MTHFR. The method accurately identified the genotype of the patient (patient sample GM00037). These results showed that the discrimination power was sufficiently strong to discriminate between a homozygous and heterozygous mutant gene. For instance, a person can be homozygous wt, mutant or heterozygous (meaning one wt and one mutant gene) for any given SNP. These three different conditions could be correctly identified for three separate SNP sites independently, in a single assay. The results demonstrated that the methods of the invention could simultaneously identify multiple SNPs in a single sample. While only three SNPs were examined in the experiment, one of skill in the art will recognize that this is only a representative number. Many more SNP sites could be tested within the same array.

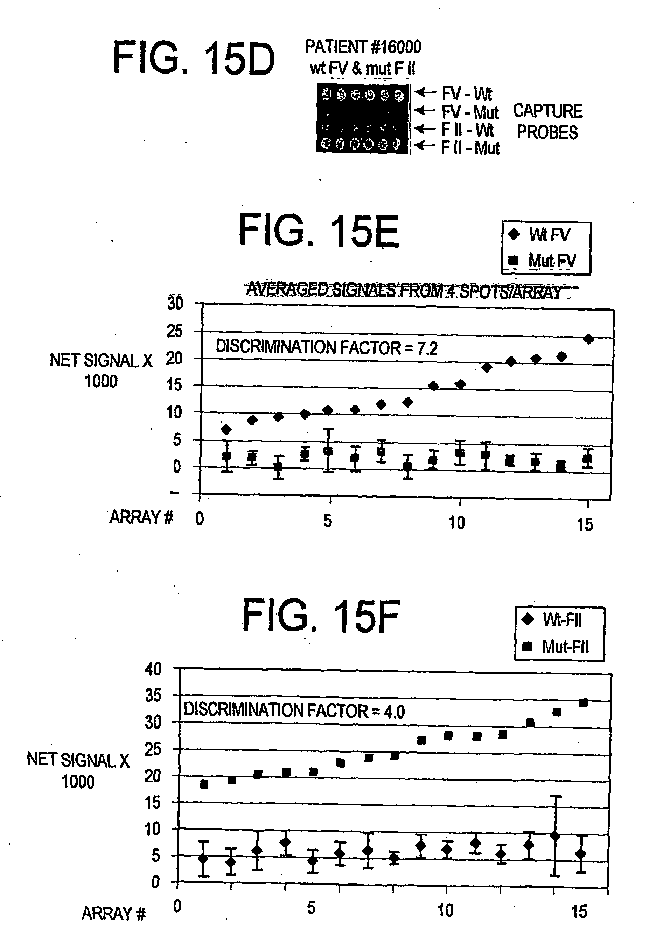

In addition to these experiments, two different investigators separately hybridized eight different slides with the DNA from 2 different patients (1 array per slide for patient GM14899 DNA and 2 arrays/slide for patient GM1600 DNA) using the methods described in these Examples. Each array had 4 repeat spots for each of 2 genes (factor II and factor V) and for each type of capture probe (mutant or wt). The net signal intensities were averaged, sorted, and then plotted starting with the lowest signal intensity. For the mismatch signals (the lower ones on each plot) three times the standard deviation was added to the average net signal. The mutant and corresponding wt signal were always plotted above each other. As shown in Figure 15, even for the smallest signal intensities, the net signal of the match was always larger than the net signal plus three-time standard deviation of the mismatch signal. Thus, in each case the correct SNP configuration could be determined with better than 99% reliability. The results further demonstrate the reproducibility and robustness of the methods described herein.

### Example 2

### Hybridization conditions for methods of the invention

Standard recommendations [T. Maniatis, E.F. Fritsch, and J. Sambrook in "Molecular Cloning, A Laboratory Manual", Cold Spring Harbor Laboratory, 1982, p324) for efficient hybridization reactions described in the art typically stipulate a hybridization temperature that is ~10-20 degrees centigrade below the Tm that is calculated for the hybridization conditions one has chosen, including salt and formamide concentration. There are different methods to calculate Tm's, each based on the exact oligonucleotide sequence and buffer conditions. For example, such calculations can be made using computer programs, which are commercially available or available online, such as the HYTHER™ server that was developed and is maintained at the Wayne State. University web site. Using all available programs on the HYTHER™ server for these calculations, the inventors computed the Tm's for both capture and detection probes (*i.e.* oligonucleotides). As shown in Table 1, the Tm's for the capture probes are either below or very near the temperature that was chosen for the hybridization (i.e. 40˚C). Thus, very low hybridization efficiency would be expected under these conditions. Moreover, capture oligonucleotides are attached to a substrate surface directly, *i.e.* without a linker sequence, meaning that the oligonucleotides closest to the surface may not be able to participate in the hybridization to the target sequence, thereby reducing the effective Tm even further. Based on the teachings in the art, the conditions used in the methods of the invention unexpectedly achieved an efficient hybridization, especially in the case where the target sequence represents only a minute fraction (*i.e.* 1/100,000,000 or a 1 million's %) of the complex DNA mixture that the human genome represents.

**Table 1**

| Capture | Sequence | No corrections (35%FM) | Santalucia et al. (35%FM) | Fotin et al. (35%FM) |
|---|---|---|---|---|
| | | | TM calculated with HyTher™ (Wayne State University) | |
| | | | TM correction for hybridization to surface bound probes according to: | |
| FII-SNP/Cap 1-wt22 (SEQ ID NO: 5) | CTCAGCGAGCCTCAATGCTCCC | 46.7 | **37.0** | 45.0 |
| FII-SNP/Cap1-mut23 | **CTCTCAGCAAGCCTCAATGCTCC** | 47.2 | **35.7** | 46.3 |
| MTHFR-SNP/Cap6-wt22 (SEQ ID NO: 6) | **GATGAAATCGGCTCCCGCAGAC** | 40.3 | **35.5** | **43.0** |
| MTHFR-SNP/Cap7-mut22 (SEQ ID NO: 2) | **ATGAAATCGACTCCCGCAGACA** | 40.7 | **36.2** | **44.0** |
| FV-Cap-WT-24 (SEQ ID NO: 15) | TGGACAGGCGAGGAATACAGGTAT | 44.8 | **35.5** | 42.9 |
| FV-Cap-mut26 (SEQ ID NO: 16) | CTGGACAGGCAAGGAATACAGGTATT | 44.5 | **35.8** | 42.9 |
| **Probe** | | | | |
| FV-46 (SEQ ID NO: 12) | 5' Epi- CCA CAG AAA ATG ATG CCC AGT GCT TAA CAA GAC CAT ACT ACA GTG A 3' | 54.8 | 49.2 | 57.6 |
| FII-Prol-47 (SEQ ID NO: 10) | 5' Epi-TCC TGG AAC CAA TCC CGT GAA AGA ATT ATT TTT GTG TTT CTA AAA CT 3' | 52.2 | 46.9 | 54.8 |
| MTHFR-Pro II-58 (SEQ ID NO: 8) | 5' Epi-AAA GAT CCC GGG GAC GAT GGG GCA AGT GAT GCC CAT GTC GGT GCA TGC CTT CAC AAA G 3' | 68.4 | 58.6 | 68.5 |

**Example 3**

**Preparation of nanoparticle-oligonucleotide conjugate probes**

In this Example, a representative nanoparticle-oligonucleotide conjugate detection probe was prepared for the use in the PCR amplification of mecA and Tuf gene targets. Gold nanoparticle-oligonucleotide probes to detect for the target mecA or Tuf gene sequences was prepared using procedures described in PCT/US97/12783, filed July 21, 1997; PCT/US00/17507, filed June 26, 2000; PCT/US01/01190, filed January 12, 2001, which are incorporated by reference in their entirety.

### (a) *Preparation Of Gold Nanoparticles*

Gold colloids (13 nm diameter) were prepared by reduction of $HAuCl_4$ with citrate as described in Frens, 1973, Nature Phys. Sci., 241:20 and Grabar, 1995, Anal. Chem.67:735. Briefly, all glassware was cleaned in aqua regia (3 parts HCl, 1 part $HNO_3$), rinsed with Nanopure $H_2O$, then oven dried prior to use. $HAuCl_4$ and sodium citrate were purchased from Aldrich Chemical Company. Aqueous $HAuCl_4$ (1 mM, 500 mL) was brought to reflux while stirring. Then, 38.8 mM sodium citrate (50 mL) was added quickly. The solution color changed from pale yellow to burgundy, and refluxing was continued for 15 min. After cooling to room temperature, the red solution was filtered through a Micron Separations Inc. 1 micron filter. Au colloids were characterized by UV-vis spectroscopy using a Hewlett Packard 8452A diode array spectrophotometer and by Transmission Electron Microscopy (TEM) using a Hitachi 8100 transmission electron microscope. Gold particles with diameters of 13 nm will produce a visible color change when aggregated with target and probe oligonucleotide sequences in the 10-35 nucleotide range.

### (b) *Synthesis Of Steroid Disulfide*

An oligonucleotide complementary to a segment of the mecA and Tuf DNA sequences were synthesized on a 1 micromole scale using a Milligene Expedite DNA synthesizer in single column mode using phosphoramidite chemistry. Eckstein, F. (ed.) Oligonulcleotides and Analogues: A Practical Approach (IRL Press, Oxford, 1991). All solutions were purchased from Milligene (DNA synthesis grade). Average coupling efficiency varied from 98 to 99.8%, and the final dimethoxytrityl (DMT) protecting group was not cleaved from the oligonucleotides to aid in purification.

To facilitate hybridization of the probe sequence with the target, a deoxyadenosine oligonucleotide ($da_{15}$ peg for all probes except probe Tuf 2 which has a $da_{10}$ peg) was included on the 5' end in the probe sequence as a spacer.

To generate 5'-terminal steroid-cyclic disulfide oligonucleotide derivatives (see Letsinger et al., 2000, Bioconjugate Chem. 11:289-291 and PCT/US01/01190 (Nanosphere, Inc.), the disclosure of which is incorporated by reference in its entirety), the final coupling reaction was carried out with a cyclic dithiane linked epiandrosterone phosphoramidite on Applied Biosystems automated synthesizer, a reagent that prepared using 1,2 -dithiane-4,5-diol, epiandrosterone and p-toluenesulphonic acid (PTSA) in presence of toluene. The phosphoramidite reagent may be prepared as follows: a solution of epiandrosterone (0.5g), 1,2-dithiane-4,5-diol (0.28 g), and p-toluenesulfonic acid (15 mg) in toluene (30 mL) was refluxed for 7 h under conditions for removal of water (Dean Stark apparatus); then the toluene was removed under reduced pressure and the reside taken up in ethyl acetate. This solution was washed with water, dried over sodium sulfate, and concentrated to a syrupy reside, which on standing overnight in pentane/ether afforded a steroid-dithioketal compound as a white solid (400 mg); Rf (TLC, silica plate, ether as eluent) 0.5; for comparison, Rf values for epiandrosterone and 1,2-dithiane-4,5-diol obtained under the same conditions are 0.4, and 0:3, respectively. Recrystallization from pentane/ether afforded a white powder, mp 110-112 ˚C; $^1$H NMR, $\delta$ 3.6 (1H, C[3]OH), 3.54-3.39 (2H, m 2OCH of the dithiane ring), 3.2-3.0 (4H, m 2CH$_2$S), 2.1-0.7 (29H, m steroid H); mass spectrum (ES[+]) calcd for $C_{23}H_{36}O_3S_2$ (M+H) 425.2179, found 425.2151. Anal. ($C_{23}H_{37}O_3S_2$) S: calcd, 15.12; found, 15.26. To prepare the steroid-disulfide ketal phosphoramidite derivative, the steroid-dithioketal (100 mg) was dissolved in THF (3 mL) and cooled in a dry ice alcohol bath. N,N-diisopropylethylamine (80 μL) and β- cyanoethyl chlorodiisopropylphosphoramidite (80 μL) were added successively; then the mixture was warmed to room temperature, stirred for 2 h, mixed with ethyl acetate (100 mL), washed with 5% aq. $NaHCO_3$ and with water, dried over sodium sulfate, and concentrated to dryness. The residue was taken up in the minimum amount of dichloromethane, precipitated at -70 ˚C by addition of hexane, and dried under vacuum; yield 100 mg; $^{31}$P NMR 146.02. The epiandrosterone-disulfide linked oligonucleotides were synthesized on Applied Biosystems automated gene synthesizer without final DMT removal. After completion, epiandrosterone-disulfide linked oligonucleotides were deprotected from the support under aqueous ammonia conditions and purified on HPLC using reverse phase column.

Reverse phase HPLC was performed with a Dionex DX500 system equipped with a Hewlett Packard ODS hypersil column (4.6 x 200 mm, 5 mm particle size) using 0.03 M $Et_3NH^+$ $OAc^-$ buffer (TEAA), pH 7, with a 1%/min. gradient of 95% $CH_3CN$/5% TEAA. The flow rate was 1 mL/ min. with UV detection at 260 nm. Preparative HPLC was used to purify the DMT-protected unmodified oligonucleotides. After collection and evaporation of the buffer, the DMT was cleaved from the oligonucleotides by treatment with 80% acetic acid for 30 min at room temperature. The solution was then evaporated to near dryness, water was added, and the cleaved DMT was extracted from the aqueous oligonucleotide solution using ethyl acetate. The amount of oligonucleotide was determined by absorbance at 260 nm, and final purity assessed by reverse phase HPLC.

*(c) Microarray Preparation*

3'-amino and 5'- amino containing DNA was synthesized by following standard protocol for DNA synthesis on DNA synthesizer. The amine modified DNA was attached to the aldehyde microarray slide by printing a 1 mM DNA solution in ArrayIt buffer plus (Catalog no.MSP, Company nameTelechem, citySunnyvale, StateCA). An Affymetrix® GMS 417 arrayer (Affymetrix, city Santa Clara, state CA) with 500 micron printing pins was used to orient the microarray on the slide. The microarray slide was purchased from Telechem (catalog no. SMM, city Sunnyvale, state CA) with an aldehyde functionalized surface. After printing, the slides were placed in a humidified chamber at ambient temperature for 12-18 hrs. The slides were removed and dried under vacuum for 30 min to 2 hrs. The slides were then subjected to two washes in 0.2 % w/v SDS and two washes in water to remove any remaining unbound DNA. The slides were then treated with a solution of 2.5 M sodium borohydride in 1X PBS with 20 % v/v 100% ethanol by soaking for 5 min. The slides were then washed three times with 0.2 % w/v SDS and twice with water and centrifuged dry.

*(d) Attachment Of Oligonucleotides To Gold Nanoparticles*

A colloidal solution of citrate stabilized gold nanoparticles (about 10 nM), prepared as described in part A above, was mixed with sulfur modified-$a_{15\,peg}$-probe oligonucleotide (4 $\mu$M), prepared as described in part B, and allowed to stand for 6 hours at room temperature in 20 ml scintillation vials. 0.1 M sodium hydrogen phosphate buffer, pH 7.0, and of 5.0 M NaCl were each added to the solution in amounts resulting in a solution at 0.01 M sodium hydrogen phosphate and 0.1 M NaCl and allowed to stand for an additional 16 hours. Sodium chloride was added in a gradient over 36 hrs to 0.8 M NaCl and the resulting solution was incubated for an additional 18 hours. The solution was aliquoted into 1 ml eppendorf tubes and centrifuged at 14,000 rpm in an Eppendorf Centrifuge 5414 for 25 minutes to give a very pale pink supernatant containing most of the oligonucleotide (as indicated by the absorbance at 260 nm) along with 7-10% of the colloidal gold (as indicated by the absorbance at 520 nm), and a compact, dark, gelatinous residue at the bottom of the tube. The supernatant was removed, and the residue was resuspended in the desired aqueous buffer. In this Example, the buffer used includes 0.1M NaCl, 10mM sodium citrate, and 0.01 % sodium azide at pH 7.

The following nanoparticle-oligonucleotide detection probes and amine modified DNA capture probes specific for mecA or Tuf DNA were prepared in this manner: Here, the oligonucleotide probe can be modified with an amine and immobilized on the glass slide as a capture probe or modified with an epiandrosterone linker and immobilized on the gold particle as a detection probe. In other words, the oligonucleotides and its reverse complements can be interchangeably used as either capture probes or nanoparticle detection probes.

(a) Detection Probes

Probe Tuf 1: gold-S'-5'-[$a_{15}$PEG-ttctatttccgtactactgac-3']$_n$ (SEQ ID NO: 17)
Probe Tuf 2: gold-S'-5'-[$a_{15}$peg- ttctatttccgtactactgacgtaact -3']$_n$ (SEQ ID NO: 18)
Probe Tuf 3: 5'-[amine-peg$_3$-ccattcttctcaaactatcgt -3'] (SEQ ID NO: 19)
Probe Tuf 4: 5'-[amine-peg$_3$-ccattcttcactaactatcgc-3'] (SEQ ID NO: 20)
Probe Tuf 5: 5'-[amine-peg$_3$-cacactccattcttctcaaact-3'] (SEQ ID NO: 21)
Probe Tuf 6: 5'-[amine-peg$_3$-cacactccattcttcactaact-3'] (SEQ ID NO: 22)
Probe Tuf 7: 5'-[amine-peg$_3$-atatgacttcccaggtgac-3'] (SEQ ID NO: 23)
Probe Tuf 8: 5'-[amine-peg$_3$-gtagatacttacattcca-3'] (SEQ ID NO: 24)
Probe Tuf 9: 5'-[amine-peg$_3$-gttgatgattacattcca-3'] (SEQ ID NO: 25)
Probe Tuf 10: 5'-[amine-peg$_3$-ccattcttcactaactaccgc-3'] (SEQ ID NO: 26)
Probe Tuf 11: 5'-[amine-peg$_3$-catacgccattcttcactaact-3'] (SEQ ID NO: 27)
Probe Tuf 15: 5'-[amine-peg$_3$-ccattcttctctaactatcgt-3'] (SEQ ID NO: 28)
Probe Tuf 16: 5'-[amine-peg$_3$-ccattcttcacaaactatcgt-3'] (SEQ ID NO: 29)
Probe Tuf 17: 5'-[amine-peg$_3$-ccattcttcagtaactatcgc-3'] (SEQ ID NO: 30)
Probe Tuf 18: 5'-[amine-peg$_3$-ccattcttcagtaactaccgc-3'] (SEQ ID NO: 31)
Probe Tuf 19: 5'-[amine-peg$_3$-ccattcttctcaaactaccgc -3'] (SEQ ID NO: 32)
Probe Tuf 20: 5'-[amine-peg$_3$-ccattcttctctaactaccgt-3'] (SEQ ID NO: 33)
Probe Tuf 21: 5'-[amine-peg$_3$-catacgccattcttcagtaact-3'] (SEQ ID NO: 34)
Probe Tuf 22: 5'-[amine-peg$_3$-cacactccattcttcagtaact-3'] (SEQ ID NO: 35)
Probe Tuf 23: 5'-[amine-peg$_3$-catactccattcttcactaact-3'] (SEQ ID NO: 36)
Probe Tuf 24: 5'-[amine-peg$_3$-catacaccattcttctcaaact-3'] (SEQ ID NO: 37)
Probe Tuf 25: 5'-[amine-peg$_3$-catactccattcttctctaact-3'] (SEQ ID NO: 38)

Probe Tuf 26: 5'-[amine-peg$_3$-cacactccattcttcacaaact-3'] (SEQ ID NO: 39)
Probe Tuf 27: 5'-[amine-peg$_3$-cacactccattcttctctaact-3'] (SEQ ID NO: 40)
Probe mecA 1:5'-[amine-peg$_3$-tcgatggtaaaggttggc -3'] (SEQ ID NO: 41)
Probe mecA 2:5'-[amine-peg$_3$-atggcatgagtaacgaagaatata-3'] (SEQ ID NO: 42)
Probe mecA 3:gold-S'-5'-[amine-peg$_3$-aaagaacctctgctcaacaag-3']$_n$ (SEQ ID NO: 43)
Probe mecA 4:gold-S'-5'-[amine-peg$_3$-gcacttgtaagcacaccttcat-3']$_n$ (SEQ ID NO: 44)
Probe mecA 6:5'-[amine-peg$_3$-ttccagattacaacttcacca-3'] (SEQ ID NO: 45)
Probe 16S 12: 5'-[amine-peg$_3$-gttcctccatatctctgcg-3'] (SEQ ID NO: 46)
Probe 16S 13: gold-S'-5'-[amine-peg$_3$-atttcaccgctacacatg-3')$_n$ (SEQ ID NO: 47)

S' indicates a connecting unit prepared via an epiandrosterone disulfide group; n represents a variable number of oligonucleotides were used in preparing the nanoparticle-oligonucleotide conjugates.

**Table 2**

| Name | SEQ ID NO: | Sequence 5'→3' | Staph Species |
|---|---|---|---|
| Tuf 1 | 17 48 | TTCTATTTCCGTACTACTGAC GTCAGTAGTACGGAAATAGAA (reverse complement) | Tuf gene General |
| Tuf 2 | 18 49 | TTCTATTTCCGTACTACTGACGTAACT AGTTACGTCAGTAGTACGGAAATAGAA (reverse complement) | Tuf gene General |
| Tuf 3 | 19 50 | CCATTCTTCTCAAACTATCGT ACGATAGTTTGAGAAGAATGG (reverse complement) | *S. aureus* |
| Tuf 4 | 20 51 | CCATTCTTCACTAACTATCGC GCGATAGTTAGTGAAGAATGG (reverse complement) | *S. epidermidis* |
| Tuf 5 | 21 52 | CACACTCCATTCTTCTCAAACT AGTTTGAGAAGAATGGAGTGTG (reverse complement) | *S. aureus* |
| Tuf 6 | 22 53 | CACACTCCATTCTTCACTAACT AGTTAGTGAAGAATGGAGTGTG (reverse complement) | *S. epidermidis* |
| Tuf 7 | 23 54 | ATATGACTTCCCAGGTGAC GTCACCTGGGAAGTCATAT (reverse complement) | Tuf gene general |
| Tuf 8 | 24 55 | GTAGATACTTACATTCCA TGGAATGTAAGTATCTAC (reverse complement) | *S. aureus* |
| Tuf 9 | 25 56 | GTTGATGATTACATTCCA TGGAATGTAATCATCAAC (reverse complement) | *S. epidermidis* |
| Tuf 10 | 26 57 | CCATTCTTCACTAACTACCGC GCGGTAGTTAGTGAAGAATGG (reverse complement) | *S. saprophyticus S. simulans* |

(continued)

| Name | SEQ ID NO: | Sequence 5'→3' | Staph Species |
|---|---|---|---|
| Tuf 11 | 27 58 | CATACGCCATTCTTCACTAACT AGTTAGTGAAGAATGGCGTATG (reverse complement) | *S. saprophyticus* |
| Tuf 15 | 28 59 | CCATTCTTCTCTAACTATCGT ACGATAGTTAGAGAAGAATGG (reverse complement) | *S. hominis* |
| Tuf 16 | 29 60 | CCATTCTTCACAAACTATCGT ACGATAGTTTGTGAAGAATGG (reverse complement) | *S. haemoylticus* |
| Tuf 17 | 30 61 | CCATTCTTCAGTAACTATCGC GCGATAGTTACTGAAGAATGG (reverse complement) | *S. cohnii* |
| Tuf 18 | 31 62 | CCATTCTTCAGTAACTACCGC GCGGTAGTTACTGAAGAATGG (reverse complement) | *S. warneri* *S. capitis* |
| Tuf 19 | 32 63 | CCATTCTTCTCAAACTACCGC GCGGTAGTTTGAGAAGAATGG (reverse complement) | *S. lugdunenis* |
| Tuf 20 | 33 64 | CCATTCTTCTCTAACTACCGT ACGGTAGTTAGAGAAGAATGG (reverse complement) | *S. auricularis* |
| Tuf 21 | 34 65 | CATACGCCATTCTTCAGTAACT AGTTACTGAAGAATGGCGTATG (reverse complement) | *S. cohnii* |
| Tuf 22 | 35 66 | CACACTCCATTCTTCAGTAACT AGTTACTGAAGAATGGAGTGTG (reverse complement) | *S. warneri* *S. capitis* |
| Tuf 23 | 36 67 | CATACTCCATTCTTCACTAACT AGTTAGTGAAGAATGGAGTATG (reverse complement) | *S. simulans* |
| Tuf 24 | 37 68 | CATACACCATTCTTCTCAAACT AGTTTGAGAAGAATGGTGTATG (reverse complement)` | *S. lugdunensis* |
| Tuf 25 | 38 69 | CATACTCCATTCTTCTCTAACT AGTTAGAGAAGAATGGAGTATG (reverse complement) | *S. hominis* |
| Tuf 26 | 39 70 | CACACTCCATTCTTCACAAACT AGTTTGTGAAGAATGGAGTGTG (reverse complement) | *S. haemolyticus* |
| Tuf 27 | 40 71 | CACACTCCATTCTTCTCTAACT AGTTAGAGAAGAATGGAGTGTG (reverse complement) | *S. auricularis* |
| mecA 1 | 41 72 | TCGATGGTAAAGGTTGGC GCCAACCTTTACCATCGA (reverse complement) | *mecA* gene |

(continued)

| Name | SEQ ID NO: | Sequence 5'→3' | Staph Species |
|---|---|---|---|
| mecA 2 | 42 73 | ATGGCATGAGTAACGAAGAATATA TATTGTATTCGTTACTCATGCCAT (reverse complement) | *mecA* gene |
| mecA 3 | 43 74 | AAAGAACCTCTGCTCAACAAG CTTGTTGAGCAGAGGTTCTTT (reverse complement) | *mecA* gene |
| mecA 4 | 44 75 | GCACTTGTAAGCACACCTTCAT ATGAAGGTGTGCTTACAAGTGC (reverse complement) | *mecA* gene |
| mecA 6 | 45 76 | TTCCAGATTACAACTTCACCA TGGTGAAGTTGTAATCTGGAA (reverse complement) | |
| 16S 12 | 46 77 | GTTCCTCCATATCTCTGCG CGCAGAGATATGGAGGAAC (reverse complement) | 16S rRNA |
| 16S 13 | 47 78 | ATTTCACCGCTACACATG CATGTGTAGCGGTGAAAT (reverse complement) | 16S rRNA |

**Example 4**

**Detection of mecA gene sequences from bacterial genomic DNA with gold nanoparticle probes**

In this Example, a method for detecting mecA gene sequences using gold nanoparticle-based detection in an array format is described. Microarray plates having mecA 2 and mecA 6 oligonucleotides as capture probes were used along with gold nanoparticles labeled with mecA 4 oligonucleotides as a detection probe. The microarray plates, capture probes, and detection probes were prepared as described in Example 3.

Gold nanoparticles (13 nm diameter) having oligonucleotide probes attached to them prepared as described in Example 3 were used to indicate the presence of DNA from the mecA gene hybridized to a transparent substrate in a three-component sandwich assay format. Nanoparticles having probe oligonucleotides attached to them and genomic DNA targets isolated from methicillin resistant (MecA+) or methicillin sensitive (MecA-) *S. aureus* bacterial cells were then cohybridized to these substrates. Therefore, the presence of nanoparticles at the surface indicated the detection of the mecA gene sequence, Figure 16. At the target amounts tested (250 ng (7.5 E7copies) - 1 ug (3.0 E8)), the attached nanoparticles could not be visualized with the naked eye. In order to facilitate the visualization of nanoparticles hybridized to the substrate surface, a signal amplification method in which silver ions are catalytically reduced by hydroquinone to form silver metal on the slide surface was employed. Although this method has been used for enlargement of protein- and antibody-conjugated gold nanoparticles in histochemical microscopy studies (Hacker, in Colloidal Gold: Principles, Methods, and Applications, M. A. Hayat, Ed. (Academic Press, San Diego, 1989), vol. 1, chap. 10; Zehbe et al., Am. J. Pathol. 150, 1553 (1997)) its use in quantitative DNA hybridization assays is novel (Tomlinson et al., Anal. Biochem., 171:217 (1988)). Not only did this method allow very low surface coverages of nanoparticle probes to be visualized by a simple flatbed scanner or the naked eye, it also permitted quantification of target hybridization based on the light scattered from the silver amplified gold probes on the stained area. Significantly, the signal intensities obtained from the samples containing methicillin resistant *S. aureus* genomic DNA were much larger than the signal intensities obtained from methicillin sensitive *S. aureus genomic DNA* at each genomic DNA amount tested. This demonstrated that this detection methodology can be used for specific detection of the mecA gene in the presence of complex bacterial genomic DNA background, Figure 16. This result is an extraordinary feature of the nanoparticle-oligonucleotide conjugates which enables ultra-sensitive and -selective detection of nucleic acids. It also should be noted that this procedure requires no enzymatic target or signal amplification procedures, providing a novel method of gene detection from bacterial genomic DNA samples.

*(a) Target DNA preparation*

Purified total genomic DNA isolated from *Staphylococcus* bacterial cells was purchased from ATCC. The total genomic DNA was fragmented by sonication to shear DNA molecules as described in Example 5 (see below) prior to hybridization on the array.

*(b) MecA gene detection assay*

*(ii) Assay procedure*

Reaction mixtures of bacterial genomic DNA ranging in amount from 250 ng - 1 ug and 1nM nanoparticle probes were made in 1x hybridization buffer (5X SSC, 0.05 % Tween 20). The reaction mixture was heated to 95 ˚C for 5 minutes. Subsequently, 10-25 ul of the reaction mixture was added to the microarray surface and hybridized at 40 ˚C and 90 % relative humidity for 2 hours. The microarray surface was washed for 30 sec in 5X SSC, 0.05 % Tween 20 at room temperature, then washed for another 30 sec with 0.5 M $NaNO_3$ also at room temperature. The microarray was dried and exposed with silver development using commercial grade silver enhancer solutions (Silver Enhancer Kit, Catalog No. SE-100, Sigma, St. Louis) for 4 minutes. The silver stained microarray plate was then washed, dried and imaged using an Arrayworx® scanner (Model No. AWE, Applied Precision, Inc., Issaquah, WA).

**Example 5**

**Staphylococcal speciation using bacterial genomic DNA and gold nanoparticle-labeled Tuf probes**

In this Example, Staphylococcal speciation was performed via discrimination of Tuf gene sequences corresponding to the species of *S. aureus* and *S. epidermidis.* Tuf 372 bp PCR amplicons amplified from total genomic DNA isolated from *S. aureus* and *S. epidermidis* bacterial cells served as a positive control to demonstrate sequence specificity of the array. In separate hybridization reactions, total genomic DNA isolated from *S. aureus* and *S. epidermidis* bacterial cells was fragmented and hybridized to the micro array plates. Microarray plates included either Tuf 3 and Tuf 4 or Tuf 5 and Tuf 6 capture probes bound thereto. Gold nanoparticles labeled with Tuf 2 oligonucleotides were used as detection probes. The microarray plates, capture and detection probes were prepared as described in Example 3. The Tuf 372 bp amplicon was prepared by conventional PCR amplification procedures.

*(a) Target DNA preparation*

The genomic DNA was prepared as follows: genomic DNA isolated from cultured *Staphylococcus* bacterial cells was purchased from ATCC (American Type Culture Collection). This dry DNA, in >10 ug portions was rehydrated in DNase free water at a volume of 200 ul. This was then sonicated using a Misonix, Ultrasonic cell disruptor XL Farmingdale, NY with 12, ~0.5 sec pulses at 2 Watts. The total DNA concentration was determined using a commercially available Picogreen kit from Molecular Probes and read on a Tecan spectrafluor plus fluorescence plate reader. The size of the DNA fragments were measured to average 1.5 Kb by performing a smear analysis on an Agilent 2100 Bioanalyzer. The positive control tuf gene 372 base-pair PCR amplicon was prepared from *S. aureus* or *S. epidermidis* genomic DNA using conventional PCR amplification techniques.

*(b) Tuf gene detection assay procedure*

In separate hybridization wells, fragmented total genomic DNA isolated from Staphylococcus epidermidis or Staphylococcus aureus bacterial cells (8.0 E07 copies, ~ 250 ng) and 1nM nanoparticle probes were mixed in 1x hybridization buffer (5X SSC, 0.05 % Tween 20). As a positive control, PCR-amplifed Tuf gene fragments of the same genomic DNA samples were mixed with probes and buffer in separate hybridization wells on the glass slide. The reaction mixture was heated to 95 ˚C for 5 minutes. Subsequently, 50 ul of the reaction mixture was added to the microarray surface and hybridized at 45 ˚C and 90 % relative humidity for 1.5 hours. The microarray surface was washed for 30 sec in 0.5 M $NaNO_3$ at room temperature. The microarray was dried and exposed with silver development using commercial grade silver enhancer solutions (Silver Enhancer Kit, Catalog No. SE-100, Sigma, St. Louis, MO) for 4 minutes. The silver stained microarray plate was then washed, dried, and the light scattered from silver amplified nanoparticle probes on the array was imaged and quantified using an Arrayworx® scanner (Model No. AWE, Applied Precision, Issaquah, WA).

The results are shown in Figures 17(a) and (b) for Tuf3 and Tuf4 capture probes, and in Figures 17(c) and (d) for Tuf5 and Tuf6 capture probes. Using the Tuf3 and Tuf4 capture probe set, specific signals are observed on the array corresponding to the Staphlococcal species *S. aureus* and *S. epidermidis* when genomic DNA is hybridized to the array. This effectively demonstrates that complexity reduction and amplification of tuf gene target by PCR is not required for differentiation of these closely related sequences in the presence of total genomic DNA. Using the Tuf5 and Tuf6 capture probe set, signals corresponding to the appropriate species are also observed, but there is some cross reactivity with the mismatched capture sequence which leads to a lower discrimination ratio. This demonstrates that sequence design is crucial to the accurate identification of species.

**Example 6**

**Staphylococcal speciation and methicillin Resistance assay using PCR amplicons and gold nanoparticles labeled mecA and Tuf oligonucleotides as detection probes**

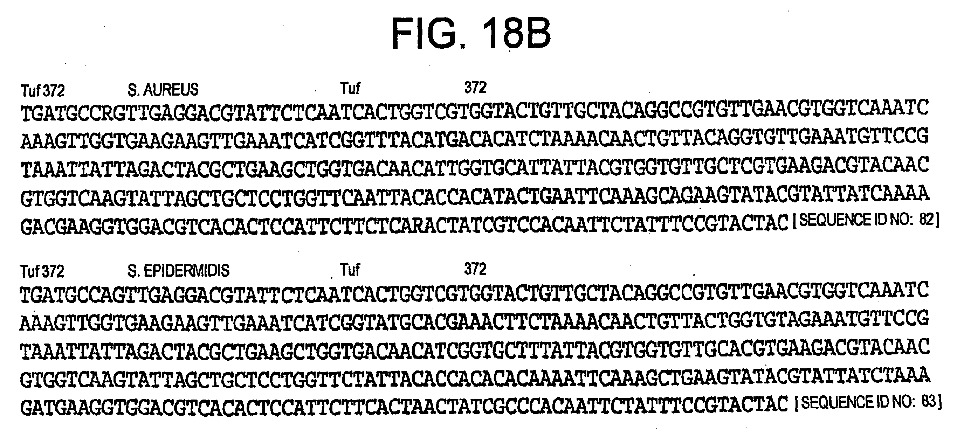

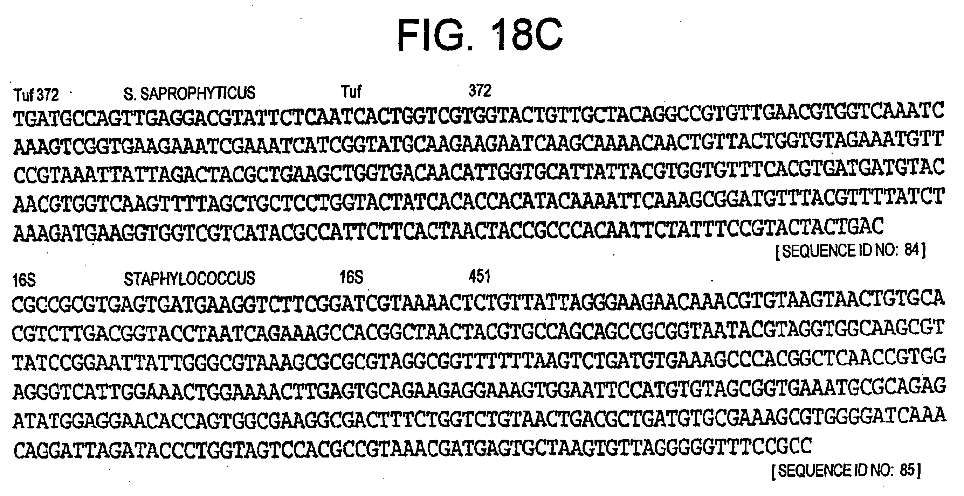

In this Example, an array designed to identify Staphylococcus genus, species, and antibiotic resistance status was fabricated using sequences from the 16S rRNA gene (genus), Tuf gene (species specific captures for *S. aureus*, *S. epidermidis*, and *S. saprophyticus*) and mecA gene (antibiotic resistance status). Note that the *S. epidermidis* and *S. saprophyticus* capture probes differ by only a single nucleotide, while the *S. aureus* and *S. epidermidis* capture probes differ by three nucleotides. Microarray plates included all of the following sequences: 16S 12, mecA 6, Tuf 3, Tuf 4, Tuf 10 capture probes and one negative hybridization capture probe bound thereto. Gold nanoparticle-labeled Tuf 2, mecA 4, and 16S 12 probes were used as detection probes. The microarray plates, capture and detection probes were prepared as described in Example 4. The specificity of the array was tested using PCR-amplified gene sequences from various methicillin resistant and methicillin sensitive Staphylococcal species (*S. aureus, S. epidermidis, and S. saprophyticus).* The specific PCR amplified gene fragments used for testing are shown in Figure 18 (mecA 281, 16S 451, and Tuf 372). The Tuf gene sequences from different Staphylococcus species shown in Figure 18 were acquired from GenBank.

*Target preparation:*

The PCR-amplified gene products were prepared using standard PCR amplification procedures.

*Assay:*

Each reaction consisted of 50 ul of 5x SSC, 0.05 % Tween 20, 0.01 % BSA, 200 pM each nanoparticle probe, 15 % formamide and 750 pM of each target amplicon. The reagents were hybridized for 1 hr at 40 C and 90 % humidity. The microarray surface was washed for 30 sec in 0.5 M $NaNO_3$ at room temperature. The microarray was dried and exposed with silver development using commercial grade silver enhancer solutions (Silver Enhancer Kit, Catalog No. SE-100, Sigma, St. Louis, MO) for 4 minutes. The silver stained microarray plate was then washed, dried and imaged using an Arrayworx® scanner (Model No. AWE, Applied Precision, Issaquah, WA).
The results are shown in Figures 19(a) and (b). The species and methicillin resistance status of five selected Staphylococcus samples (see Table 3 below) were correctly identified using PCR amplicons demonstrating the specificity of the array sequences when standard PCR amplification procedures are employed.

**Table 3**

| ATCC Sample ID # | Description |
| --- | --- |
| 35556 | S. aureus |
| 700699 | S. aureus, Mu50-resistant to methicillin |
| 12228 | S. epidermidis |
| 35984 | S. epidermidis, RP62A-multiply antibiotic-resistant |
| 15305 | S. saprophyticus |

**Example 7**

**Staphylococcal speciation and methicillin resistance assay using genomic DNA and gold nanoparticle-labeled mecA, 16S and Tuf probes**

In this Example, the identification of Staphylococcus genus, species, and antibiotic resistance status was tested using total genomic DNA isolated from *S. aureus* and *S. epidermidis* bacterial cells. The genomic DNA samples tested were characterized by ATCC as described in table 3 above. The microarray plates and detection probes used for testing in example 6 also were used for this example. The microarray plates and capture and detection probes were prepared as described in Example 3. The genomic DNA samples were prepared as described in example 5. Each reaction consisted of 50 ul of 5x SSC, 0.05 % Tween 20, 0.01 % BSA, 200 pM each nanoparticle probe, and 15 % formamide and 3.3 ng/ul of sonicated genomic DNA. The reagents were hybridized for 2 hrs at 40 C and 90 % humidity. The microarray surface was washed for 30 sec in 0.5 M $NaNO_3$ at room temperature. The microarray was dried and exposed with silver development using commercial grade silver enhancer solutions (Silver Enhancer Kit, Catalog No. SE-100, Sigma, St. Louis) for 4 minutes. The silver stained microarray plate was then washed, dried and imaged using an Arrayworx®) scanner (Model No. AWE, Applied Precision, Issaquah, WA).

The results are shown in Figure 20. Significantly, Staphylococcus species and antibiotic resistance status was correctly identified for three genomic DNA samples tested based on net signal intensities that were above 3 standard deviations over background at only the correct capture probe site in each sample. This experiment demonstrates that even single nucleotide mutations can be detected within the tuf gene when Staphylococcus genomic DNA is hybridized to the array and labeled with silver amplified gold nanoparticle probes. Therefore, speciation of biological microorganisms that differ by as little as a single nucleotide within a given gene sequence is achievable by this novel detection methodology without any enzyme-based target amplification (e.g. PCR) or signal amplification (e.g. horseradish peroxidase) procedures.

The assay sensitivity was measured by titrating known amounts of total genomic DNA isolated from methicillin resistant *S.* aureus cells into the assay and measuring the net signal intensity from the mecA gene capture probes, Figure 21. The lowest detectable quantity was 34 ng, which corresponds to roughly 10 million copies of the genome. Further optimization of the described detection procedures should enable much lower quantities of genomic DNA to be detectable.

**Claims**

1. A method for detecting one or more target nucleic acid sequences in a sample, the sample comprising nucleic acid molecules of higher biological complexity relative to amplified nucleic acid molecules and the target nucleic acid sequence differs from one or more nucleic acid sequences by at least one nucleotide, the method comprising the steps of:

   (a) providing an addressable substrate having at least one capture oligonucleotide bound thereto, wherein the at least one capture oligonucleotide has a sequence that is complementary to at least part of a first portion of the target nucleic acid sequence;
   (b) providing one or more detection probes comprising detector oligonucleotides, wherein the detector oligonucleotides have a sequence that is complementary to at least part of a second portion of the target nucleic acid sequence of step (a);
   (c) contacting the sample with the substrate and the one or more detection probes under conditions that are effective for the hybridization of the at least one capture oligonucleotide to the first portion of the target nucleic acid sequence and the hybridization of the one or more detection probes to the second portion of the target nucleic acid sequence and to allow for discrimination between the target and said one or more nucleic acid sequences that differ by at least one nucleotide; and
   (d) detecting whether the at least one capture oligonucleotide and one or more detection probes hybridized with the first and second portions of the target nucleic acid sequence,

   wherein the detector probe is a nanoparticle probe having detector oligonucleotides bound thereto, and
   wherein one or more of the at least one target nucleic acid sequences, at least one detection oligonucleotides, and at least one of the capture oligonucleotides comprises the sequence set forth in SEQ ID NO:19. SEQ ID NO: 20, SEQ ID NO: 50, SEQ ID NO: 51.

2. The method of claim 1, wherein the target nucleic acid sequence comprises a Single Nucleotide Polymorphism.

3. The method of claim 1 or 2, wherein the at least one nucleotide difference is recognized by one or both of the capture oligonucleotide bound to the substrate or the detector oligonucleotides.

4. The method of any preceding claim, wherein the nucleic acid molecules in the sample comprise genomic DNA, genomic RNA, expressed RNA, plasmid DNA, mitochondrial or other cell organelle DNA, free cellular DNA, viral DNA or viral RNA, or a mixture of two or more of the above.

5. The method of any preceding claim, wherein the substrate comprises a plurality of capture oligonucleotides, each of which can recognize a different single nucleotide polymorphism.

6. The method of any preceding claim, wherein the sample comprises more than one nucleic acid target, each of which comprises one or more different single nucleotide polymorphisms.

7. The method of any preceding claim, wherein the one or more detector probes are capable of hybridizing with a different nucleic acid target.

8. A method of any preceding claim, wherein sample is contacted with either or both of

   a. the detector probe, so that a nucleic acid target present in the sample hybridizes with the detector oligonucleotides on the detector probe, and the nucleic acid target bound to the detector probe is then contacted with the substrate so that the nucleic acid target hybridizes with the capture oligonucleotide on the substrate, and
   b. the substrate so that a nucleic acid target present in the sample hybridizes with a capture oligonucleotide, and the nucleic acid target bound to the capture oligonucleotide is then contacted with the detector probe so that the nucleic acid target hybridizes with the detector oligonucleotides on the detector probe;

   and preferably the sample is contacted simultaneously with the detector probe and the substrate.

9. The method of any preceding claim, wherein one or more detector probes comprise a detectable label.

10. The method of claim 9, wherein the detection label allows detection by photonic, electronic, acoustic, opto-acoustic, gravity, electro-chemical, electro-optic, mass-spectrometric, enzymatic, chemical, biochemical, or physical means and optionally is one or more of the following: fluorescent, luminescent, phosphorescent, radioactive, a nanoparticle, a dendrimer, a molecular aggregate, a quantum dot, or a bead.

11. The method of claim 1, wherein the nanoparticles are made of a noble metal, preferably gold or silver.

12. The method of any preceding claim, wherein the detecting comprises one or more detection methods selected from contacting the substrate with silver stain, detecting light scattered by nanoparticle, or observation with an optical scanner or a flatbed scanner.

13. The method of claim 12, wherein the observation is with an optical scanner or a flatbed scanner and the scanner is linked to a computer loaded with software capable of calculating grayscale measurements, and the grayscale measurements are calculated to provide a quantitative measure of the amount of nucleic acid detected.

14. The method of any preceding claim, wherein the oligonucleotides attached to the substrate are located between two electrodes, the nanoparticles are made of material that is a conductor of electricity, and step (d) comprises detecting a change in conductivity.

15. The method of claim 14, wherein the electrodes are made of gold and the nanoparticles are made of gold.

16. The method of any of claims 14-15 wherein the substrate is contacted with silver stain to produce the change conductivity.

17. The method of any of claims 14-16 wherein a plurality of oligonucleotides, each of which can recognize a different target nucleic acid sequence, are attached to the substrate in an array of spots and each spot of oligonucleotides is located between two electrodes, the nanoparticles are made of a material that is a conductor of electricity, and step (d) comprises detecting a change in conductivity.

18. The method of any preceding claim, wherein the target nucleic acid sequence is a portion of a gene of a biological organism, preferably a Staphylococcus bacterium, and most preferably selected from the group consisting of *S. aureus*, *S. haemolyticus, S. epidermidis, S. lugdenensis, S. hominis,* or *S. saprophyticus.*

19. The method of claim 18, wherein the target nucleic acid sequence is the Turf gene.

20. The method of any preceding claim, wherein the method is used to distinguish between two or more species of a common genus; preferably where the species differ by at least one nucleotide and/or by two or more consecutive or non-consecutive nucleotides.

**Patentansprüche**

1. Ein Verfahren zum Nachweis von einer oder mehr Zielnukleinsäuresequenzen in einer Probe, die Probe umfassend Nukleinsäuremoleküle von höherer biologischer Komplexität relativ zu amplifizierten Nukleinsäuremolekülen und die Zielnukleinsäuresequenz unterscheidet sich von einer oder mehr Nukleinsäuresequenzen durch mindestens ein Nukleotid, das Verfahren umfassend die Schritte:

   (a) Bereitstellen eines adressierbaren Substrates mit mindestens einem daran gebundenen Einfangoligonukleotid, wobei das mindestens eine Einfangoligonukleotid eine Sequenz hat, die komplementär ist zu wenigstens einem Teil eines ersten Bereichs der Zielnukleinsäuresequenz;
   (b) Bereitstellen von einer oder mehr Nachweissonden umfassend Nachweisoligonukleotide, wobei die Nachweisoligonukleotide eine Sequenz haben, die komplementär ist zu wenigstens einem Teil eines zweiten Bereichs der Zielnukleinsäuresequenz aus Schritt (a);
   (c) Kontaktieren der Probe mit dem Substrat und den ein oder mehr Nachweissonden unter Bedingungen, die wirksam sind für die Hybridisierung des mindestens einen Einfangoligonukleotids mit dem ersten Bereich der Zielnukleinsäuresequenz und die Hybridisierung der einen oder mehr Nachweissonden mit dem zweiten Bereich der Zielnukleinsäuresequenz und die Unterscheidung erlauben zwischen der Ziel- und den ein oder mehr Nukleinsäuresequenzen, die sich um mindestens ein Nukleotid unterscheiden; und
   (d) Nachweisen, ob das mindestens eine Einfangoligonukleotid und ein oder mehr Nachweissonden mit den ersten und zweiten Bereichen der Zielnukleinsäuresequenz hybridisiert haben, wobei die Nachweissonde eine Nanopartikelsonde mit daran gebundenen Nachweisoligonukleotiden ist und wobei ein oder mehr der mindestens einen Zielnukleinsäuresequenzen, mindestens ein Nachweisoligonukleotid und mindestens eines der Einfangoligonukleotide die in SEQ ID NO:19, SEQ ID NO:20, SEQ ID NO:50, SEQ ID NO:51 dargelegte Sequenz umfasst.

2. Das Verfahren aus Anspruch 1, wobei die Zielnukleinsäuresequenz einen Einzelnukleotidpolymorphismus umfasst.

3. Das Verfahren aus Anspruch 1 oder 2, wobei der mindestens eine Nukleotidunterschied erkannt wird von einem oder beiden aus dem an das Substrat gebundenen Einfangoligonukleotid oder den Nachweisoligonukleotiden.

4. Das Verfahren aus irgendeinem vorhergehenden Anspruch, wobei die Nukleinsäuremoleküle in der Probe genomische DNS, genomische RNS, exprimierte RNS, Plasmid-DNS, mitochondriale oder andere Zellorganellen-DNS, freie zelluläre DNS, virale DNS oder virale RNS oder ein Gemisch von zwei oder mehr der Obigen umfassen.

5. Das Verfahren aus irgendeinem vorhergehenden Anspruch, wobei das Substrat eine Vielzahl von Einfangoligonukleotiden umfasst, von denen jedes einen anderen Einzelnukleotidpolymorphismus erkennen kann.

6. Das Verfahren aus irgendeinem vorhergehenden Anspruch, wobei die Probe mehr als ein Nukleinsäureziel umfasst, von denen jedes ein oder mehr unterschiedliche Einzelnukleotidpolymorphismen umfasst.

7. Das Verfahren aus irgendeinem vorhergehenden Anspruch, wobei die ein oder mehr Nachweissonden in der Lage sind, mit einem verschiedenen Nukleinsäureziel zu hybridisieren.

8. Das Verfahren aus irgendeinem vorhergehenden Anspruch, wobei Probe kontaktiert wird mit einem oder beiden von

   a. der Nachweissonde, so dass das in der Probe vorhandene Nukleinsäureziel mit den Nachweisoligonukleotiden auf der Nachweissonde hybridisiert und das an die Nachweissonde gebundene Nukleinsäureziel dann mit dem Substrat kontaktiert wird, so dass das Nukleinsäureziel mit dem Einfangoligonukleotid auf dem Substrat hybridisiert, und
   b. dem Substrat, so dass ein in der Probe vorhandenes Nukleinsäureziel mit einem Einfangoligonukleotid hybridisiert und das an das Einfangoligonukleotid gebundene Nukleinsäureziel dann mit der Nachweissonde kontaktiert wird, so dass das Nukleinsäureziel mit den Nachweisoligonukleotiden auf der Nachweissonde hybridisiert;

und vorzugsweise die Sonde gleichzeitig kontaktiert wird mit der Nachweissonde und dem Substrat.

9. Das Verfahren aus irgendeinem vorhergehenden Anspruch, wobei eine oder mehr Nachweissonden einen nachweisbaren Marker umfassen.

10. Das Verfahren aus Anspruch 9, wobei der Nachweismarker den Nachweis mittels photonischer, elektronischer, akustischer, optoakustischer, Schwerkrafts-, elektrochemischer, elektrooptischer, massenspektrometrischer, enzymatischer, chemischer, biochemischer oder physikalischer Mittel erlaubt und optional eines oder mehr von Folgenden ist: fluoreszent, lumineszent, phosphoreszent, radioaktiv, ein Nanopartikel, ein Dendrimer, ein molekulares Aggregat, ein Quantum-Dot oder eine Perle.

11. Das Verfahren aus Anspruch 1, wobei die Nanopartikel aus einem Edelmetall, vorzugsweise Gold oder Silber, gefertigt sind.

12. Das Verfahren aus irgendeinem vorhergehenden Anspruch, wobei der Nachweis ein oder mehr Nachweisverfahren umfasst ausgewählt aus Kontaktieren des Substrats mit Silberfarbstoff, Nachweisen von durch Nanopartikel gestreuten Lichts oder Beobachtung mit einem optischen Scanner oder Flachbettscanner.

13. Das Verfahren aus Anspruch 12, wobei die Beobachtung mit einem optischen Scanner oder einem Flachbettscanner stattfindet und der Scanner mit einem Computer verbunden ist, auf den Software geladen ist, die Grauskalenmessungen berechnen kann, und die Grauskalenmessungen berechnet werden, um ein quantitatives Maß der Menge von nachgewiesener Nukleinsäure zu liefern.

14. Das Verfahren aus irgendeinem vorhergehenden Anspruch, wobei die an dem Substrat angebrachten Oligonukleotide sich zwischen zwei Elektroden befinden, die Nanopartikel aus einem Material gefertigt sind, das ein Leiter von Elektrizität ist, und Schritt (d) den Nachweis einer Änderung der Leitfähigkeit umfasst.

15. Das Verfahren aus Anspruch 14, wobei die Elektroden aus Gold gefertigt sind und die Nanopartikel aus Gold gefertigt sind.

16. Das Verfahren aus irgendeinem der Ansprüche 14-15, wobei das Substrat mit Silberfarbstoff kontaktiert wird, um die Änderung der Leitfähigkeit auszulösen.

17. Das Verfahren aus irgendeinem der Ansprüche 14-16, wobei eine Vielzahl von Oligonukleotiden, von denen jedes eine andere Zielnukleinsäuresequenz erkennen kann, in einer Anordnung von Flecken an dem Substrat angebracht sind und sich jeder Fleck von Oligonukleotiden zwischen zwei Elektroden befindet, die Nanopartikel aus einem Material gefertigt sind, das ein Leiter von Elektrizität ist, und Schritt (d) den Nachweis einer Änderung der Leitfähigkeit umfasst.

18. Das Verfahren aus irgendeinem vorhergehenden Anspruch, wobei die Zielnukleinsäuresequenz ein Abschnitt eines Gens eines biologischen Organismus ist, vorzugsweise eines Staphylococcus-Bakteriums, und am meisten bevorzugt ausgewählt aus der Gruppe bestehend aus *S. aureus, S. haemolyticus, S. epidermidis, S. lugdenensis, S. hominis* oder *S. saprophyticus.*

19. Das Verfahren aus Anspruch 18, wobei die Zielnukleinsäuresequenz das Turf-Gen ist.

20. Das Verfahren aus irgendeinem vorhergehenden Anspruch, wobei das Verfahren verwendet wird, um zwischen zwei oder mehr Arten einer gemeinsamen Gattung zu unterscheiden; vorzugsweise wobei die Arten sich in mindestens einem Nukleotid und/oder in zwei oder mehr aufeinanderfolgenden oder nicht aufeinanderfolgenden Nukleotiden unterscheiden.

**Revendications**

1. Procédé de détection d'une ou plusieurs séquences d'acide nucléique cible dans un échantillon, l'échantillon comprenant des molécules d'acide nucléique d'une complexité biologique supérieure par rapport à des molécules d'acide nucléique amplifié et la séquence d'acide nucléique cible diffère d'une ou plusieurs séquences d'acide nucléique par au moins un nucléotide, le procédé comprenant les étapes consistant à :

(a) fournir un substrat abordable ayant au moins un oligonucléotide de capture qui y est lié, ou le au moins un oligonucléotide de capture a une séquence qui est complémentaire d'au moins une partie d'une première partie de la séquence d'acide nucléique cible ;

(b) fournir une ou plusieurs sondes de détection comprenant des oligonucléotides détecteurs, où les oligonucléotides détecteurs ont une séquence qui est complémentaire d'au moins une partie d'une seconde partie de la séquence d'acide nucléique cible de l'étape (a) ;

(c) mettre en contact l'échantillon avec le substrat et l'une ou plusieurs sondes de détection dans des conditions qui sont efficaces pour l'hybridation du au moins un oligonucléotide de capture à la première partie de la séquence d'acide nucléique cible et l'hybridation de la une ou plusieurs sondes de détection à la seconde partie de la séquence d'acide nucléotide cible et pour permettre de faire la discrimination entre la cible et lesdites une ou plusieurs séquences d'acide nucléique qui diffèrent par au moins un nucléotide ; et

(d) détecter si le au moins un oligonucléotide de capture et une ou plusieurs sondes de détection hybridés aux première et seconde parties de la séquence d'acide nucléique cible,

dans lequel la sonde détecteur est une sonde nanoparticulaire ayant des oligonucléotides détecteurs qui y sont liés, et dans lequel un ou plusieurs de la au moins une des séquences d'acide nucléique cible, au moins un des oligonucléotides de détection et au moins un des oligonucléotides de capture comprend la séquence illustrée par SEQ ID NO : 19, SEQ ID NO : 20, SEQ ID NO : 50, SEQ ID NO : 51.

2. Procédé selon la revendication 1, dans lequel la séquence d'acide nucléique cible comprend un polymorphisme d'un seul nucléotide.

3. Procédé selon la revendication 1 ou 2, dans lequel la au moins une différence de nucléotide est reconnue par l'un ou les deux de l'oligonucléotide de capture lié au substrat ou des oligonucléotides détecteurs.

4. Procédé selon l'une quelconque des revendications précédentes, dans lequel les molécules d'acide nucléique dans l'échantillon comprennent de L'ADN génomique, de l'ARN génomique, de l'ARN exprimé, de l'ADN plasmidique, de l'ADN mitochondrial ou d'un autre organite cellulaire, de l'ADN cellulaire libre, de l'ADN viral de l'ARN viral, ou un mélange de deux ou plus de ceux cités ci-dessus.

5. Procédé selon l'une quelconque des revendications précédentes, dans lequel le substrat comprend une pluralité d'oligonucléotides de capture, dont chacun peut reconnaître un polymorphisme d'un seul nucléotide différent.

6. Procédé selon l'une quelconque des revendications précédentes, dans lequel l'échantillon comprend plus d'un acide nucléique cible, chacun comprenant un ou plusieurs polymorphismes d'un seul nucléotide différents.

7. Procédé selon l'une quelconque des revendications précédentes, dans lequel les une ou plusieurs sondes détecteurs sont capables de s'hybrider à un acide nucléique cible différent.

8. Procédé selon l'une quelconque des revendications précédentes dans lequel l'échantillon est mis en contact avec l'un ou l'autre ou les deux

   a. de la sonde détecteur, de telle manière qu'un acide nucléique cible présent dans l'échantillon s'hybride aux oligonucléotides détecteurs sur la sonde détecteur, et l'acide nucléique cible lié à la sonde détecteur est ensuite mis en contact avec le substrat de telle manière que l'acide nucléique cible s'hybride à l'oligonucléotide de capture sur le substrat, et

   b. du substrat, de telle manière qu'un acide nucléique cible présents dans l'échantillon s'hybride à un oligonucléotide de capture, et l'acide nucléique cible lié à l'oligonucléotide de capture est ensuite mis en contact avec la sonde détecteur de telle manière que l'acide nucléique cible s'hybride aux oligonucléotides détecteurs sur la sonde détecteur ;

et de préférence, l'échantillon est mis en contact simultanément avec la sonde détecteur et le substrat.

9. Procédé selon l'une quelconque des revendications précédentes, dans lequel une ou plusieurs sondes détecteurs comprennent un marqueur détectable.

10. Procédé selon la revendication 9, dans lequel le marqueur de détection permet une détection par un moyen photonique, électronique, acoustique, opto-acoustique, de gravité, électrochimique, électro-optique, spectrométrique de masse, enzymatique, chimique, biochimique ou physique et est éventuellement un ou plusieurs des suivants : fluorescent, luminescent, phosphorescent, radioactif, une nanoparticule, un dendrimère, un agrégat moléculaire, un quantum dot ou une bille.

11. Procédé selon la revendication 1, dans lequel les nanoparticules sont faites d'un métal noble, de préférence d'or ou d'argent.

12. Procédé selon l'une quelconque des revendications précédentes, dans lequel la détection comprend un ou plusieurs procédés de détection choisis parmi la mise en contact du substrat avec un colorant à l'argent, la détection de la lumière dispersée par une nanoparticule ou l'observation avec un scanner optique ou un scanner à plat.

13. Procédé selon la revendication 12, dans lequel l'observation est réalisée avec un scanner optique ou un scanner à plat et le scanner est relié à un ordinateur chargé avec un logiciel capable de calculer des mesures avec différentes nuances de gris et les mesures avec différentes nuances de gris sont calculées pour fournir une mesure quantitative de la quantité d'acide nucléique détectée.

14. Procédé selon l'une quelconque des revendications précédentes, dans lequel les oligonucléotides fixés au substrat sont localisés entre deux électrodes, les nanoparticules sont faites d'un matériau qui est un conducteur de l'électricité et l'étape (d) comprend la détection d'un changement de la conductivité.

15. Procédé selon la revendication 14, dans lequel les électrodes sont faites d'or et les nanoparticules sont faits d'or.

16. Procédé selon l'une quelconque des revendications 14 ou 15, dans lequel le substrat est mis en contact avec un colorant à l'argent pour produire le changement de conductivité.

17. Procédé selon l'une quelconque des revendications 14 à 16, dans lequel une pluralité d'oligonucléotides, chacun pouvant reconnaître une séquence d'acide nucléique cible différente, sont fixés au substrat dans une collection de spots et chaque spot des oligonucléotides est localisé entre deux électrodes, les nanoparticules sont faites d'un matériau qui est un conducteur de l'électricité et l'étape (d) comprend la détection d'un changement de la conductivité.

18. Procédé selon l'une quelconque des revendications précédentes, dans lequel la séquence d'acide nucléique cible est une partie d'un gène d'un organisme biologique, de préférence une bactérie staphylocoque, et de manière préférée entre toutes choisie dans le groupe comprenant *S. aureus, S. haemolyticus, S. epidermidis, S. lugdenensis, S. hominis* ou *S. saprophyticus.*

19. Procédé selon la revendication 18, dans lequel la séquence d'acide nucléique cible est le gène Turf.

20. Procédé selon l'une quelconque des revendications précédentes, où le procédé est utilisé pour faire la distinction entre deux espèces où plus d'un genre commun ; de préférence ou les espèces diffèrent par au moins un nucléotide et/ou par deux nucléotides consécutifs ou non consécutifs ou plus.

FIG. 1

ADD TARGET
ANALYTES AND
NANOPARTICLE
PROBES TOGETHER

mut CAPTURE    wt CAPTURE

NANOPARTICLE
PROBES

wt
GENOMIC
DNA

SPECIFIC BINDING OF NANOPARTICLE
PROBES TO wt CAPTURE PROBES VIA wt
TARGET ANALYTE IN A SANDWICH FORMAT

FIG. 3

C  G

T      G

WILD TYPE
CAPTURE

MUTANT
CAPTURE

# FIG. 2

mut CAPTURE      wt CAPTURE

HYBRIDIZE wt DNA TO
CAPTURE PROBES
UNDER HIGH
STRINGENCY

CAPTURE OF wt TARGET
ANALYTES BY wt
CAPTURE PROBES ONLY

ADD HIGH
SENSITIVITY
DETECTION
PROBES TO
DETECT

HIGH
SENSITIVITY
NANOPARTICLE
PROBES

EP 1 578 952 B1

FIG. 4A 10ug OF NORMAL HUMAN GENOMIC DNA

Mut

WT

FIG. 4B 10ug OF SALMON SPERM DNA

Mut

WT

FIG. 4C

# FIG. 5

# FIG. 6A

Wt GENOMIC DNA (SIGMA)

Mut FII & Wt F V GENOMIC DNA (PATIENT #16000)

FII - Mut
FII - Wt
FV - Mut
FV - Wt

# FIG. 6B

NET SIGNAL

RATIO=2.1

RATIO=12.5

- Mut
- Wt

F II

F V

# FIG. 6C

NET SIGNAL

RATIO=41.1

RATIO=31.8

- Mut
- Wt

F II

F V

FIG. 7A

FV CAPTURE PROBES

MUTANT

WILD TYPE

32% FM     29% FM     26% FM     23% FM

MUTANT

WILD TYPE

FIG. 7B

SIGNAL
INTENSITY
(RFU)

EP 1 578 952 B1

# FIG. 8

| 20% FM | 30% FM | 40% FM |
|---|---|---|
| MUTANT WILD TYPE | MUTANT WILD TYPE | MUTANT WILD TYPE |
| NO SNP DISCRIMINATION STRINGENCY TOO LOW | GOOD SNP DISCRIMINATION STRINGENCY CORRECT | INSUFFICIENT OR NO SIGNAL STRINGENCY TOO HIGH |

FV CAPTURE PROBE

Mut - 26mer

Wt - 24mer

Mut - 21mer

Wt - 20mer

EP 1 578 952 B1

# FIG. 9A

30% FM       FV CAPTURE PROBES

MUTANT 26mer
WILD TYPE 24mer
MUTANT 21mer
WILD TYPE 20mer

Mutant    Wild Type

# FIG. 9B

RATIO OF SIGNAL INTENSITIES

# FIG. 9C

HYBRIDIZATION WITH MUTANT
GENOMIC DNA

# FIG. 9D

HYBRIDIZATION WITH
WILD TYPE GENOMIC DNA

# FIG. 10A

FV CAPTURE
PROBES

| 0.5 ug | 1 ug | 2.5 ug | |
| --- | --- | --- | --- |

Mut 26mer
Wt 24mer
Mut 21mer
Wt 20mer

IMAGES OF FAC. V ARRAY HYBRIDIZED
WITH MUTANT GENOMIC DNA.

# FIG. 10B

SIGNAL INTENSITIES FOR MUTANT (26mer)
AND WILD TYPE (24mer) CAPTURE PROBES

# FIG. 11A

|  | | | | | | FV CAPTURE PROBES |
|---|---|---|---|---|---|---|
| WELL 2 | WELL 4 | WELL 6 | WELL 8 | WELL 10 | | Mut 26mer |
| | | | | | | WILD TYPE 24mer |
| | | | | | | Mut 21mer |
| | | | | | | WILD TYPE 20mer |
| | | | | | | Mut 26mer |
| | | | | | | WILD TYPE 24mer |
| | | | | | | Mut 21mer |
| | | | | | | WILD TYPE 20mer |
| WELL 1 | WELL 3 | WELL 5 | WELL 7 | WELL 9 | | |

# FIG. 11B

EP 1 578 952 B1

FIG. 12A

GENES

MUTANT          WILD TYPE

Fac V

MTHFR

Fac II

FIG. 12B

WILD TYPE/MUTANT RATIO

43

FIG. 13A

GENES

FacV

MTHFR

Fac II

MUTANT        WILD TYPE

FIG. 13B

WILD TYPE/MUTANT RATIO

## FIG. 14A

GENES | WILD TYPE | MUTANT

Fac V

Fac II

MTHFR

## FIG. 14B

IMAGED WITH VERIGENE ID, 100ms.

NET SIGNAL

- FV-Wt: 20505
- FV-Mut: 465
- F II-Wt: 22942
- F II-Mut: 29055
- M-Wt: 272
- M-Mut: 10154

## FIG. 14C

MATCH/ MISMATCH RATIO

- Fac V: 44.1
- Fac II: 0.8
- MTHFR: 37.4

GENES

# FIG. 15A

PATIENT #14899
mut FV & wt F II

CAPTURE
PROBES

FV - Wt →
FV - Mut →
F II - Wt →
F II - Mut →

# FIG. 15B

◆ Wt FV
■ Mut FV

AVERAGED SIGNALS FROM 4 SPOTS/ARRAY

NET SIGNAL X 1000

DISCRIMINATION FACTOR = 2.7

ARRAY #

# FIG. 15C

◆ Wt-FII
■ Mut-FII

NET SIGNAL X 1000

DISCRIMINATION FACTOR = 2.3

ARRAY #

# FIG. 15D

PATIENT #16000
wt FV & mut F II

← FV - Wt
← FV - Mut    CAPTURE
← F II - Wt    PROBES
← F II - Mut

# FIG. 15E

◆ Wt FV
■ Mut FV

AVERAGED SIGNALS FROM 4 SPOTS/ARRAY

NET SIGNAL X 1000

DISCRIMINATION FACTOR = 7.2

ARRAY #

# FIG. 15F

◆ Wt-FII
■ Mut-FII

NET SIGNAL X 1000

DISCRIMINATION FACTOR = 4.0

ARRAY #

MSRA 281 bp
2.7 E7 COPIES
PCR

MRSA
7.5 E7 COPIES

MRSA
1.5 E8 COPIES

MRSA
2.3 E8 COPIES

MRSA
3.0 e8 COPIES

CAPTURE SEQUENCE
mecA 2
mecA 6

MSRA 281 bp
2.7 E8 COPIES
PCR

MSSA
7.5 E7 COPIES

MSSA
1.5 E8 COPIES

MSSA
2.3 E8 COPIES

MSSA
3.0 e8 COPIES

mecA 2
mecA 6

FIG. 16A

FIG. 16B

EP 1 578 952 B1

# FIG. 17A

SAMPLE

| TARGET COPY | S. AUREUS | S. EPIDERMIDIS | CAPTURE SEQUENCES |
|---|---|---|---|
| 3.0 E + 7 Tuf 372 | | | ← Tuf 3 = S. AUREUS<br>← Tuf 4 = S. EPIDERMIDIS |
| NO | | | ← Tuf 3 = S. AUREUS<br>← Tuf 4 = S. EPIDERMIDIS |
| 8.0 E + 7 GENOMIC | | | ← Tuf 3 = S. AUREUS<br>← Tuf 4 = S. EPIDERMIDIS |

# FIG. 17B

Legend:
- ⊠ Tuf 4/S. AUREUS TARGET
- □ Tuf 3/S. AUREUS TARGET
- ▨ Tuf 4/S. EPIDERMIDIS TARGET
- ⊟ Tuf 3/S. EPIDERMIDIS TARGET

NET SIGNAL (y-axis: 0 to 700)

TARGET COPY NUMBER (x-axis: 3 E7 PCR, 7 E7 g. DNA)

# FIG. 17C

# FIG. 17D

TARGET COPY NUMBER

# FIG. 18A

| NAME | TEMPLATE SPECIES | GENE | LENGTH | SEQUENCE 5'⟶3' (CODING STRAND) |
|---|---|---|---|---|
| mecA 281 | S. AUREUS | mecA | 281 | |

ATCCACCCTCAAACAGGTGAATTATTAGCATTGTAAGCACACCTTCATATGACGTCTATCCATTTATGTATGGCATG
AGTAACGAAGAATATAATAAATTAACCGAAGATAAAAAAGAACCTCTGCTCAAGTCAATCCAGATTACAACTTCACC
AGGTTCAACTCAAAAAATATTAACAGCAATGATTGGGTTAAATAACAAAACATTAGACGATAAAACAAGTTATAAAA
TCGATGGTAAAGGTTGGCAAAAAGATAAATCTTGGGGTGGTTACAACGT [SEQUENCE ID NO: 79]

| COA | S. AUREUS | COAGULASE | 480 | |
|---|---|---|---|---|

CGAGACCAAGATTCAACAAGCCAAGTGAAACAAATGCATACAACGTAACGACAAATCAAGATGGCACAGTATCATAC
GGAGCTCGCCCAACACAAAACAAGCCAAGTGAAAAAACGCATATAACGTAACAACACATGCAAATGGTCAAGTATCA
TACGGTGCTCGCCCAACACAAACAAGCCAAGCAAAACAAATGCATACAACGTAACAACACATGCAAATGGTCAAGTA
TCATATGGCGCTCGCCCGACACAAAAAAAGCCAAGCAAAACAAATGCATATAACGTAACAACACATGCAAATGGTCA
AGTATCATACGGAGCTCGCCCGACATACAAGAAGCCAAGCGAAACAAATGCATACAACGTAACAACACATGCAAATG
GTCAAGTATCATATGGCGCTCGCCCGACACAAAAAAAGCCAAGCGAAACAAACGCATATAACGTAACAACACATGCA
GATGGTACTGCGACAT [SEQUENCE ID NO: 80]

| Tuf 142 | S. AUREUS | Tuf | 142 | |
|---|---|---|---|---|

GTGGTCAAGTATTAGCTGCTCCTGGTTCAATTACACCACATACTGAATTCAAAGCAGAAGTATACGTATTATCAAAA
GACGAAGGTGGACGTCACACTCCATTCTTCTCARACTATCGTCCACAATTCTATTTCCGTACTAC [SEQUENCE ID NO: 81]

EP 1 578 952 B1

# FIG. 18B

Tuf 372        S. AUREUS                Tuf          372

TGATGCCRGTTGAGGACGTATTCTCAATCACTGGTCGTGGTACTGTTGCTACAGGCCGTGTTGAACGTGGTCAAATC
AAAGTTGGTGAAGAAGTTGAAATCATCGGTTTACATGACACATCTAAAACAACTGTTACAGGTGTTGAAATGTTCCG
TAAATTATTAGACTACGCTGAAGCTGGTGACAACATTGGTGCATTATTACGTGGTGTTGCTCGTGAAGACGTACAAC
GTGGTCAAGTATTAGCTGCTCCTGGTTCAATTACACCACATACTGAATTCAAAGCAGAAGTATACGTATTATCAAAA
GACGAAGGTGGACGTCACACTCCATTCTTCTCARACTATCGTCCACAATTCTATTTCCGTACTAC [SEQUENCE ID NO: 82]

Tuf 372        S. EPIDERMIDIS           Tuf       372

TGATGCCAGTTGAGGACGTATTCTCAATCACTGGTCGTGGTACTGTTGCTACAGGCCGTGTTGAACGTGGTCAAATC
AAAGTTGGTGAAGAAGTTGAAATCATCGGTATGCACGAAACTTCTAAAACAACTGTTACTGGTGTAGAAATGTTCCG
TAAATTATTAGACTACGCTGAAGCTGGTGACAACATCGGTGCTTTATTACGTGGTGTTGCACGTGAAGACGTACAAC
GTGGTCAAGTATTAGCTGCTCCTGGTTCTATTACACCACACACAAAATTCAAAGCTGAAGTATACGTATTATCTAAA
GATGAAGGTGGACGTCACACTCCATTCTTCACTAACTATCGCCCACAATTCTATTTCCGTACTAC [SEQUENCE ID NO: 83]

EP 1 578 952 B1

# FIG. 18C

EP 1 578 952 B1

Tuf 372    S. SAPROPHYTICUS      Tuf      372

TGATGCCAGTTGAGGACGTATTCTCAATCACTGGTCGTGGTACTGTTGCTACAGGCCGTGTTGAACGTGGTCAAATC
AAAGTCGGTGAAGAAATCGAAATCATCGGTATGCAAGAAGAATCAAGCAAAACAACTGTTACTGGTGTAGAAATGTT
CCGTAAATTATTAGACTACGCTGAAGCTGGTGACAACATTGGTGCATTATTACGTGGTGTTTCACGTGATGATGTAC
AACGTGGTCAAGTTTTAGCTGCTCCTGGTACTATCACACCACATACAAAATTCAAAGCGGATGTTTACGTTTTATCT
AAAGATGAAGGTGGTCGTCATACGCCATTCTTCACTAACTACCGCCCACAATTCTATTTCCGTACTACTGAC

[SEQUENCE ID NO: 84]

16S      STAPHYLOCOCCUS      16S      451

CGCCGCGTGAGTGATGAAGGTCTTCGGATCGTAAAACTCTGTTATTAGGGAAGAACAAACGTGTAAGTAACTGTGCA
CGTCTTGACGGTACCTAATCAGAAAGCCACGGCTAACTACGTGCCAGCAGCCGCGGTAATACGTAGGTGGCAAGCGT
TATCCGGAATTATTGGGCGTAAAGCGCGCGTAGGCGGTTTTTTAAGTCTGATGTGAAAGCCCACGGCTCAACCGTGG
AGGGTCATTGGAAACTGGAAAACTTGAGTGCAGAAGAGGAAAGTGGAATTCCATGTGTAGCGGTGAAATGCGCAGAG
ATATGGAGGAACACCAGTGGCGAAGGCGACTTTCTGGTCTGTAACTGACGCTGATGTGCGAAAGCGTGGGGATCAAA
CAGGATTAGATACCCTGGTAGTCCACGCCGTAAACGATGAGTGCTAAGTGTTAGGGGGTTTCCGCC

[SEQUENCE ID NO: 85]

# FIG. 19A

CAPTURE SEQUENCES

16S ⟶
mecA ⟶
S. AUREUS ⟶
S. EPIDERMIDIS ⟶
S. SPROPHYTICUS ⟶
NEG. CONTROL ⟶

ATCC SAMPLE ID          35556

# FIG. 19B

EP 1 578 952 B1

FIG. 19C

CAPTURE SEQUENCES

16S ⟶
mecA ⟶
S. AUREUS ⟶
S. EPIDERMIDIS ⟶
S. SPROPHYTICUS ⟶
NEG. CONTROL ⟶

ATCC SAMPLE ID          35984

FIG. 19D

55

# FIG. 19E

CAPTURE SEQUENCES

16S ⟶

mecA ⟶

S. AUREUS ⟶

S. EPIDERMIDIS ⟶

S. SPROPHYTICUS ⟶

NEG. CONTROL ⟶

ATCC SAMPLE ID          12228

# FIG. 19F

FIG. 19G

CAPTURE SEQUENCES

16S ⟶
mecA ⟶
S. AUREUS ⟶
S. EPIDERMIDIS ⟶
S. SPROPHYTICUS ⟶
NEG. CONTROL ⟶

ATCC SAMPLE ID          700699

FIG. 19H

# FIG. 19I

CAPTURE SEQUENCES

16S ⟶
mecA ⟶
S. AUREUS ⟶
S. EPIDERMIDIS ⟶
S. SPROPHYTICUS ⟶
NEG. CONTROL ⟶

ATCC SAMPLE ID          15305

# FIG. 19J

☐ ATCC 15305

ATCC SAMPLE ID

50000
40000
30000
20000
10000
0

16S
mecA
S.AUREUS
S.EPIDERMIDIS
S.SPROPHYTICUS
NEG. CONTROL

15305

EP 1 578 952 B1

FIG. 20A

ATCC 35984

FIG. 20B

59

# FIG. 20C

ATCC 700699

# FIG. 20D

# FIG. 20E

ATCC 12228

# FIG. 20F

□ 5X10$^7$ COPIES OF ATCC12228

16S  mecA  S.A.  S.E.  S.S.  NEG CONTROL

FIG. 21

$y = 8E\text{-}06x + 23.021$
$R^2 = 0.9913$

NET SIGNAL INTENSITY

COPY NUMBER

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5679524 A, Nikiforov
- WO 9859066 A, McIntosh
- WO 9512607 A, Goelet
- US 5151510 A, Stec
- US 6361944 B
- US 6417340 B
- US 6506564 B
- US 02116382 W
- US 43134103 A
- US 0314100 W
- US 5506564 A
- US 0146418 W
- US 9712783 W
- US 0017507 A
- US 0101190 W
- US 0017507 W

### Non-patent literature cited in the description

- **WANG et al.** *Science,* 1998, vol. 280, 1077-1082
- **TYAGI et al.** *Nature Biotechnol.,* 1998, vol. 16, 49-53
- **CHEN et al.** *Genome Res.,* 1998, vol. 8, 549-556
- **PASTINEN et al.** *Clin. Chem.,* 1996, vol. 42, 1391-1397
- **CHEN et al.** *Proc. Natl. Acad. Sci.,* 1997, vol. 94, 10756-10761
- **SHUBER et al.** *Hum. Mol. Gen.,* 1997, vol. 6, 337-347
- **LIU et al.** *Genome Res.,* 1997, vol. 7, 389-398
- **LIVAK et al.** *Nature Genet.,* vol. 9, 341-342
- **DAY ; HUMPHRIES.** *Anal. Biochem.,* 1994, vol. 222, 389-395
- **KENNEDY et al.** *Nature Biotechnol.,* 2003, vol. 21, 1233-1237
- **LANDER E.** *Nature Genetics,* 1999, vol. 21, 3-4
- **EDWARDS.** *J. Clin. Micro.,* vol. 39, 3047-3051
- **OLIVE ; BEAN.** *J. Clin. Micro.,* 1999, vol. 37, 1661-1669
- **HAMELS.** *Biotechniques,* 2001, vol. 31, 1364-1372
- **MARTINEAU F et al.** the development of a PCR assay for identification of staphylococci at genus and species levels. *JOURNAL OF CLINICAL MICROBIOLOGY,* July 2001, vol. 39 (7), 2541-2547
- **CAO Y C et al.** nanoparticles with Raman spectroscopic fingerprints for DNA and RNA detection. *SCIENCE,* 30 August 2002, vol. 297 (5586), 1536-1540
- **LAPLANCHE et al.** *Nucl. Acids Res.,* 1986, vol. 14, 9081
- **STEC et al.** *J. Am. Chem. Soc.,* 1984, vol. 106, 6077
- **STEIN et al.** *Nucl. Acids Res.,* 1988, vol. 16, 3209
- **ZON et al.** *Anti-Cancer Drug Design,* 1991, vol. 6, 539
- **ZON et al.** OLIGONUCLEOTIDES AND ANALOGUES: A PRACTICAL APPROACH. Oxford University Press, 1991, 87-108
- **UHLMANN ; PEYMAN.** *Chemical Reviews,* 1990, vol. 90, 543
- **LEWIN.** GENE EXPRESSION. John Wiley & Sons, 1980, vol. 2
- **BRITTEN ; KOHNE.** *Science,* 1968, vol. 161, 529-540
- Nanoscale Materials in Chemistry. Wiley Interscience, 2001
- **STORHOFF.** *J. Am. Chem. Soc,* 1998, vol. 120, 1959
- **BASSELL et al.** *J. Cell Biol,* 1994, vol. 126, 863-876
- **BRAUN-HOWLAND et al.** *Biotechniques,* 1992, vol. 13, 928-931
- **BRAUN et al.** *Nature,* 1998, vol. 391, 775
- Eukaryotic Chromosomes. **LEWIN.** GENE EXPRESSION. John Wiley & Sons, 1980, vol. 2
- **WENZEL et al.** *Am. J. Med.,* 1992, vol. 91 (3B), 221-7
- **BLUMBERG et al.** *J. Inf. Disease,* 1991, vol. 63, 1279-85
- **MURAKAMI ; TOMASZ.** *J. Bacteriol.,* 1989, vol. 171, 874-79
- **MATTHEWS ; TOMASZ.** *Antimicrobial Agents and Chemotherapy,* 1990, vol. 34, 1777-9
- **FRENS.** *Nature Phys. Sci.,* 1973, vol. 241, 20

- **GRABAR.** *Anal. Chem.,* 1995, vol. 67, 735
- Oligonucleotides and Analogues: A Practical Approach. IRL Press, 1991
- **LETSINGER et al.** *Bioconjugate Chem.,* 2000, vol. 11, 289-291
- **T. MANIATIS ; E.F. FRITSCH ; J. SAMBROOK.** Molecular Cloning, A Laboratory Manual. Cold Spring Harbor Laboratory, 1982, 324
- Oligonulcleotides and Analogues: A Practical Approach. IRL Press, 1991
- **HACKER.** Colloidal Gold: Principles, Methods, and Applications. Academic Press, 1989, vol. 1
- **ZEHBE et al.** *Am. J. Pathol.,* 1997, vol. 150, 1553
- **TOMLINSON et al.** *Anal. Biochem.,* 1988, vol. 171, 217